# EUROPÄISCHE PATENTSCHRIFT

(11) **EP 2 185 550 B1**
(45) Veröffentlichungstag und Bekanntmachung des Hinweises auf die Patenterteilung: **02.11.2011**
(21) Anmeldenummer: 08774012.2
(22) Anmeldetag: 17.07.2008
(51) Int. Cl.: C07D 413/12, C07D 413/14, C07D 419/14, A61K 31/40, A61P 9/06

(54) **SUBSTITUIERTE ARYLOXAZOLE UND IHRE VERWENDUNG**
SUBSTITUTED ARYLOXAZOLES AND THE USE THEREOF
ARYLOXAZOLES SUBSTITUÉS ET LEUR UTILISATION

(30) Priorität: 27.07.2007 DE 102007035367
(43) Veröffentlichungstag der Anmeldung: 19.05.2010
(73) Patentinhaber: Bayer Pharma Aktiengesellschaft, 13353 Berlin (DE)
(72) Erfinder: NELL, Peter, 42115 Wuppertal (DE); HÜBSCH, Walter, 42113 Wuppertal (DE); ALBRECHT-KÜPPER, Barbara, 42289 Wülfrath (DE); KELDENICH, Jörg, 42113 Wuppertal (DE); VAKALOPOULOS, Alexandros, 40721 Hilden (DE); SÜSSMEIER, Frank, 42277 Wuppertal (DE); ZIMMERMANN, Katja, 40470 Düsseldorf (DE); LANG, Dieter, 42553 Velbert (DE); MEIBOM, Daniel, 51373 Leverkusen (DE)
(86) Internationale Anmeldenummer: PCT/EP2008/005833
(87) Internationale Veröffentlichungsnummer: WO 2009/015776

(56) Entgegenhaltungen:
- WO-A-01/25210
- WO-A-02/070484
- WO-A-02/070485

## Beschreibung

Die vorliegende Anmeldung betrifft neue substituierte Aryloxazol-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, vorzugsweise zur Behandlung und/oder Prävention von kardiovaskulären und Stoffwechsel-Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosin-rezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmern geeignet sein.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herzfrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

In Adipozyten bewirkt die Aktivierung von A1- und A2b-Rezeptoren eine Inhibition der Lipolyse. Die selektive bzw. kombinierte Wirkung von A1- und A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren [siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis", J. Biol. Chem. 267 (1992), Seiten 10764-10770, deren Offenbarung hiermit im vollen Umfang durch Bezugnahme eingeschlossen ist].

Die Wirkung der Substanzen an solchen Zelllinien lässt sich erfassen durch biochemische Messung des intrazellulären Botenstoffes cAMP [siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9, deren Offenbarung hiermit im vollen Umfang durch Bezugnahme eingeschlossen ist].

Bei den aus der Literatur bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Adenosin-Liganden dieses Strukturtyps haben jedoch in der Regel den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet.

In WO 01/25210, WO 02/070484 und WO 02/070485 werden substituierte 2-Thio- bzw. 2-Oxy-3,5-dicyano-4-phenyl-6-aminopyridine als Adenosinrezeptor-Liganden für die Behandlung von Erkrankungen offenbart. In WO 03/053441 werden spezifisch substituierte 2-Thio-3,5-dicyano-4-phenyl-6-aminopyridine als selektive Liganden des Adenosin A1-Rezeptors beschrieben, und in WO 2006/027142 werden substituierte Phenylaminothiazol-Derivate als duale Adenosin A1/A2b-Agonisten für die Behandlung der Hypertonie und anderer kardiovaskulärer Erkrankungen beansprucht. Allerdings zeigte es sich, dass diese Verbindungen zum Teil bezüglich ihrer physikochemischen Eigenschaften, wie beispielsweise ihrer Löslichkeit und/oder Formulierbarkeit, oder hinsichtlich ihrer *in vivo*-Eigenschaften, wie beispielsweise ihrem pharmakokinetischen Verhalten, ihrer Dosis-Wirkungsbeziehung und/oder ihrem Metabolisierungsweg, Nachteile aufweisen.

Weiterhin werden in WO 01/62233 verschiedene Pyridin- und Pyrimidin-Derivate sowie deren Verwendung als Adenosinrezeptor-Modulatoren offenbart. Substituierte 3,5-Dicyanopyridine als Calcium-abhängige Kaliumkanalöffner zur Behandlung urologischer Erkrankungen werden in EP 1302 463-A1 beansprucht. In WO 2004/054505 wird die Verwendung von Aminocyanopyridin-Derivaten als MK 2-Inhibitoren zur Behandlung TNFα-mediierter Erkrankungen beansprucht. Die Verwendung von 4-Aryl- oder 4-Heteroaryl-substituierten Aminocyanopyridinen als Androgenrezeptor-Modulatoren wird in US 2005/0182105 beschrieben.

Aufgabe der vorliegenden Erfindung war die Bereitstellung neuer Verbindungen, die als selektive Agonisten des Adenosin A1-Rezeptors oder als selektive duale Agonisten des Adenosin A1- und A2b-Rezeptors wirken, als solche zur Behandlung und/oder Prävention insbesondere von kardiovaskulären Erkrankungen wie Hypertonie, Angina pectoris, Myokardinfarkt, Herzinsuffizienz und Vorhofflimmern, des Metabolischen Syndroms, von Diabetes und Dyslipidämien sowie zur Organprotektion bei Transplantationen und operativen Eingriffen geeignet sind und darüber hinaus ein verbessertes therapeutisches Profil gegenüber den aus dem Stand der Technik bekannten Verbindungen aufweisen.

Gegenstand der vorliegenden Erfindung sind Verbindungen der Formel (I) 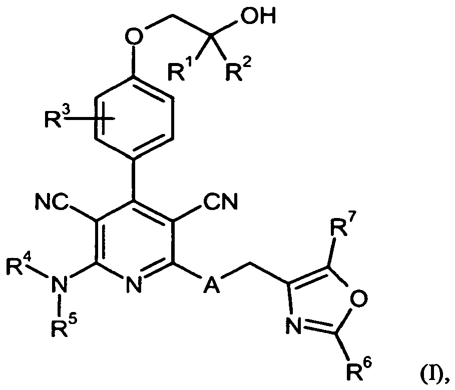 in welcher
- A: für O oder S steht,
- R¹: für Wasserstoff oder (C₁-C₄)-Alkyl steht,
- R²: für Wasserstoff oder (C₁-C₄)-Alkyl, das mit Hydroxy, (C₁-C₄)-Alkoxy oder bis zu dreifach mit Fluor substituiert sein kann, steht
oder
- R¹ und R²: miteinander verknüpft sind und gemeinsam mit dem Kohlenstoffatom, an das sie gebunden sind, einen Cyclopropan- oder Cyclobutan-Ring bilden,
- R³: für Wasserstoff, Halogen oder (C₁-C₄)-Alkyl steht,
- R⁴ und R⁵: gleich oder verschieden sind und unabhängig voneinander für Wasserstoff oder (C₁-C₆)-Alkyl, das ein- oder zweifach, gleich oder verschieden, mit Hydroxy, (C₁-C₄)-Alkoxy,

Amino, Mono-(C₁-C₄)-alkylamino, Di-(C₁-C₄)-alkylamino, Carboxyl, (C₁-C₄)-Alkoxycarbonyl und/oder einem 4- bis 7-gliedrigen Heterocyclus substituiert sein kann, stehen,
wobei der genannte Heterocyclus ein oder zwei Ring-Heteroatome aus der Reihe N, O und/ oder S enthält und seinerseits ein- oder zweifach, gleich oder verschieden, mit (C₁-C₄)-Alkyl, Hydroxy, Oxo und/oder (C₁-C₄)-Alkoxy substituiert sein kann,
oder
- R⁴ und R⁵: zusammen mit dem Stickstoffatom, an das sie gebunden sind, einen 4- bis 7-gliedrigen Heterocyclus bilden, der ein weiteres Ring-Heteroatom aus der Reihe N, O oder S enthalten und ein- oder zweifach, gleich oder verschieden, mit (C₁-C₄)-Alkyl, Hydroxy, Oxo und/oder (C₁-C₄)-Alkoxy substituiert sein kann,
und entweder (i)
- R⁶: für (C₆-C₁₀)-Aryl oder 5- bis 10-gliedriges Heteroaryl mit bis zu drei Ring-Heteroatomen aus der Reihe N, O und/oder S steht, welche jeweils ein- bis dreifach, gleich oder verschieden, mit Halogen, Nitro, Cyano, (C₁-C₄)-Alkyl, Trifluormethyl, Hydroxy, (C₁-C₄)-Alkoxy, Difluormethoxy, Trifluormethoxy, Mono-(C₁-C₄)-alkylaminocarbonyl, (C₁-C₄)-Alkoxycarbonyl und/oder Carboxyl substituiert sein können,
und
- R⁷: für Wasserstoff, Fluor, Chlor, (C₁-C₄)-Alkyl, Trifluormethyl, (C₁-C₄)-Alkoxycarbonyl, Carboxyl oder Phenyl steht, wobei (C₁-C₄)-Alkyl mit Hydroxy oder (C₁-C₄)-Alkoxy und Phenyl mit Halogen, Cyano, (C₁-C₄)-Alkyl oder Trifluormethyl substituiert sein können,
oder (*ii*)
- R⁶: für Wasserstoff oder (C₁-C₄)-Alkyl steht
und
- R⁷: für Phenyl oder 5- oder 6-gliedriges Heteroaryl mit bis zu zwei Ring-Heteroatomen aus der Reihe N, O und/oder S steht, welche jeweils ein- oder zweifach, gleich oder verschieden, mit Halogen, Cyano, (C₁-C₄)-Alkyl und/oder Trifluormethyl substituiert sein können,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, 1-Ethylpropyl, *n*-Pentyl und *n*-Hexyl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy und *tert*.-Butoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, *n*-Propoxycarbonyl, Isopropoxycarbonyl und *tert*.-Butoxycarbonyl.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino und *tert*.-Butylamino.
Mono-(C₁-C₄)-alkylaminocarbonyl steht im Rahmen der Erfindung für eine Amino-Gruppe, die über eine Carbonylgruppe verknüpft ist und die einen geradkettigen oder verzweigten Alkylsubstituenten mit 1 bis 4 Kohlenstoffatomen aufweist. Beispielhaft und vorzugsweise seien genannt: Methylaminocarbonyl, Ethylaminocarbonyl, *n*-Propylaminocarbonyl, Isopropylaminocarbonyl, *n-*Butylaminocarbonyl und *tert*.-Butylaminocarbonyl.
Di-C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N*,*N*-Dimethylamino, *N,N*-Diethylamino, *N*-Ethyl-*N*-methylamino, *N*-Methyl-*N*-*n*-propylamino, *N*-Isopropyl-*N*-*n*-propylamino, *N*,*N*-Diisopropylamino, *N*-*n*-Butyl-*N*-methylamino und *N*-*tert*.-Butyl-*N*-methylamino.
(C₆-C₁₀-Aryl steht im Rahmen der Erfindung für einen aromatischen Carbocyclus mit 6 oder 10 Ring-Kohlenstoffatomen. Bevorzugte Arylreste sind Phenyl und Naphthyl.

Ein 4- bis 7-gliedriger Heterocyclus steht im Rahmen der Erfindung für einen gesättigten Heterocyclus mit insgesamt 4 bis 7 Ringatomen, der ein oder zwei Ring-Heteroatome aus der Reihe N, O und/oder S enthält und über ein Ring-Kohlenstoffatom oder gegebenenfalls über ein Ring-Stickstoffatom verknüpft ist. Bevorzugt ist ein 4- bis 6-gliedriger Heterocyclus mit ein oder zwei Ring-Heteroatomen aus der Reihe N und/oder O. Beispielhaft seien genannt: Azetidinyl, Oxetanyl, Pyrrolidinyl, Pyrazolidinyl, Tetrahydrofuranyl, Piperidinyl, Piperazinyl, Tetrahydropyranyl, Morpholinyl, Thiomorpholinyl, Hexahydroazepinyl und Hexahydro-1,4-diazepinyl. Bevorzugt sind Azetidinyl, Pyrrolidinyl, Tetrahydrofuranyl, Piperidinyl, Piperazinyl, Tetrahydropyranyl und Morpholinyl.

Ein Azetidino-, Pyrrolidino-, Piperidino- oder Morpholino-Rest steht im Rahmen der Erfindung für einen über das jeweilige Ring-Stickstoffatom verknüpften Azetidin-, Pyrrolidin-, Piperidin- bzw. Morpholin-Ring.

5- bis 10-gliedriges Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedrige Heteroaryl-Reste mit bis zu zwei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Pyridyl, Pyrimidinyl, Pyridazinyl, Pyrazinyl.

Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt im Rahmen der vorliegenden Erfindung sind Verbindungen der Formel (I), in welcher
- A: für O oder S steht,
- R¹: für Wasserstoff oder Methyl steht,
- R²: für Wasserstoff, Methyl, Hydroxymethyl, Methoxymethyl oder Trifluormethyl steht,
- R³: für Wasserstoff, Fluor oder Methyl steht,
- R⁴: für Wasserstoff oder (C₁-C₄)-Alkyl, das ein- oder zweifach, gleich oder verschieden, mit Hydroxy, (C₁-C₄)-Alkoxy, Amino, Mono-(C₁-C₄)-alkylamino, Di-(C₁-C₄)-alkylamino, Carboxyl und/oder einem 4- bis 6-gliedrigen Heterocyclus substituiert sein kann, steht, wobei der genannte Heterocyclus ein oder zwei Ring-Heteroatome aus der Reihe N und/ oder O enthält und seinerseits ein- oder zweifach, gleich oder verschieden, mit Methyl, Hydroxy und/oder Methoxy substituiert sein kann,
- R⁵: für Wasserstoff oder Methyl steht
oder
- R⁴ und R⁵: zusammen mit dem Stickstoffatom, an das sie gebunden sind, einen 4- bis 6-gliedrigen Heterocyclus bilden, der ein weiteres Ring-Heteroatom aus der Reihe N oder O enthalten und ein- oder zweifach, gleich oder verschieden, mit Methyl, Hydroxy und/oder Methoxy substituiert sein kann,
und entweder (*i*)
- R⁶: für Phenyl, Pyridyl oder Thienyl steht, welche jeweils ein- bis dreifach, gleich oder verschieden, mit Fluor, Chlor, Cyano, (C₁-C₄)-Alkyl, Trifluormethyl, (C₁-C₄)-Alkoxy, Trifluormethoxy, Mono-(C₁-C₄)-alkylaminocarbonyl, (C₁-C₄)-Alkoxycarbonyl und/oder Carboxyl substituiert sein können,
und
- R⁷: für Wasserstoff, (C₁-C₄)-Alkyl, Trifluormethyl, (C₁-C₄)-Alkoxycarbonyl, Carboxyl oder Phenyl, das mit Fluor oder Chlor substituiert sein kann, steht,
oder (ii)
- R⁶: für Wasserstoff steht
und
- R⁷: für Phenyl, das ein- oder zweifach, gleich oder verschieden, mit Fluor, Chlor, Cyano, Methyl und/oder Trifluormethyl substituiert sein kann, steht,
sowie ihre Salze, Solvate und Solvate der Salze.

Besonders bevorzugt im Rahmen der vorliegenden Erfindung sind Verbindungen der Formel (I), in welcher
- A: für O oder S steht,
- R¹: für Wasserstoff oder Methyl steht,
- R²: für Wasserstoff, Methyl, Hydroxymethyl oder Trifluormethyl steht,
- R³: für Wasserstoff oder Fluor steht,
- R⁴: für Wasserstoff oder (C₁-C₄)-Alkyl, das ein- oder zweifach, gleich oder verschieden, mit Hydroxy, Amino, Methylamino, Ethylamino, Dimethylamino und/oder Diethylamino substituiert sein kann, steht,
- R⁵: für Wasserstoff oder Methyl steht
oder
- R⁴ und R⁵: zusammen mit dem Stickstoffatom, an das sie gebunden sind, einen Azetidino-, Pyrrolidino- oder Piperidino-Ring, welcher jeweils mit Hydroxy substituiert sein kann, oder einen Morpholino-Ring bilden,
- R⁶: für Phenyl oder Thienyl steht, welche jeweils ein- oder zweifach, gleich oder verschieden, mit Fluor, Chlor, Methyl, Trifluormethyl, Methoxy und/oder Carboxyl substituiert sein können,
und
- R⁷: für Wasserstoff, Methyl, Trifluormethyl, Methoxycarbonyl oder Carboxyl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Insbesondere bevorzugt im Rahmen der vorliegenden Erfindung sind die nachfolgend genannten Verbindungen
2-Amino-6-({[2-(3-chlor-4-fluorphenyl)-1,3-oxazol-4-yl]methyl}sulfanyl)-4-[4-(2-hydroxyethoxy)-phenyl]pyridin-3,5-dicarbonitril;
2-Amino-6-({[2-(3,4-difluorphenyl)-1,3-oxazol-4-yl]methyl}sulfanyl)-4-[4-(2-hydroxyethoxy)-phenyl]pyridin-3,5-dicarbonitril;
2-Amino-6-({[2-(4-fluor-3-methylphenyl)-1,3-oxazol-4-yl]methyl}sulfanyl)-4-[4-(2-hydroxyethoxy)phenyl]pyridin-3,5-dicarbonitril;
2-Amino-6-({[2-(4-chlorphenyl)-1,3-oxazol-4-yl]methyl}sulfanyl)-4-(4-{[(2*S*)-2,3-dihydroxypropyl]oxy}phenyl)pyridin-3,5-dicarbonitril;
2-Amino-6-({[2-(4-chlorphenyl)-1,3-oxazol-4-yl]methyl}sulfanyl)-4-(4-{[(2*R*)-2,3-dihydroxypropyl]oxy}phenyl)pyridin-3,5-dicarbonitril;
2-Amino-4-(4-{[(2*S*)-2,3-dihydroxypropyl]oxy}phenyl)-6-({[2-(4-fluorphenyl)-5-methyl-1,3-oxazol-4-yl]methyl}sulfanyl)pyridin-3,5-dicarbonitril;
2-Amino-6-({[2-(4-chlorphenyl)-5-methyl-1,3-oxazol-4-yl]methyl}sulfanyl)-4-(4-{[(2*S*)-2,3-dihydroxypropyl]oxy}phenyl)pyridin-3,5-dicarbonitril;
2-Amino-4-(4-{[(2*S*)-2,3-dihydroxypropyl]oxy}phenyl)-6-({[2-(2-fluorphenyl)-5-methyl-1,3-oxazol-4-yl]methyl}sulfanyl)pyridin-3,5-dicarbonitril;
2-Amino-4-(4-{[(2*R*)-2,3-dihydroxypropyl]oxy}phenyl)-6-({[2-(4-methoxyphenyl)-5-methyl-1,3-oxazol-4-yl]methyl}sulfanyl)pyridin-3,5-dicarbonitril;
2-Amino-6-({[2-(4-chlor-3-methylphenyl)-1,3-oxazol-4-yl]methyl}sulfanyl)-4-(4-{[(2*R*)-2,3-dihydroxypropyl]oxy}phenyl)pyridin-3,5-dicarbonitril;
4-{4-[({6-Amino-3,5-dicyano-4-[4-(2-hydroxy-2-methylpropoxy)phenyl]pyridin-2-yl}thio)-methyl]-5-methyl-1,3-oxazol-2-yl}benzoesäure;
2-Amino-4-(4-{[(2*R*)-2,3-dihydroxypropyl]oxy}phenyl)-6-({[2-(4-fluorphenyl)-1,3-oxazol-4-yl]-methyl} sulfanyl)pyridin-3,5-dicarbonitril;
2-Amino-6-{[2-(4-chlorphenyl)-1,3-oxazol-4-yl]methoxy}-4-[4-(2-hydroxyethoxy)phenyl]pyridin-3,5-dicarbonitril;
2-Amino-6-{[2-(3,4-difluorphenyl)-1,3-oxazol-4-yl]methoxy}-4-[4-(2-hydroxyethoxy)phenyl]-pyridin-3,5-dicarbonitril;
2-({[2-(4-Chlorphenyl)-1,3-oxazol-4-yl]methyl}sulfanyl)-4-[4-(2-hydroxyethoxy)phenyl]-6-(pyrrolidin-1-yl)pyridin-3,5-dicarbonitril;
2-({[2-(4-Chlorphenyl)-1,3-oxazol-4-yl]methyl}sulfanyl)-4-[4-(2-hydroxyethoxy)phenyl]-6-[(2-hydroxyethyl)amino]pyridin-3,5-dicarbonitril;
2-({2-(4-Chlorphenyl)-1,3-oxazol-4-yl]methyl}sulfanyl)-6-{[(2*R*)-2,3-dihydroxypropyl]amino}-4-[4-(2-hydroxyethoxy)phenyl]pyridin-3,5-dicarbonitril;
2-({[2-(4-Chlorphenyl)-1,3-oxazol-4-yl]methyl}sulfanyl)-4-[4-(2-hydroxyethoxy)phenyl]-6-[(3*R*)-3-hydroxypyrrolidin-1-yl]pyridin-3,5-dicarbonitril;
2-{[2-(4-Chlorphenyl)-1,3-oxazol-4-yl]methoxy}-4-[4-(2-hydroxyethoxy)phenyl]-6-[(2-hydroxyethyl)amino]pyridin-3,5-dicarbonitril
und
2-{[2-(4-Chlorphenyl)-1,3-oxazol-4-yl]methoxy}-6-(3-hydroxyazetidin-1-yl)-4-[4-(2-hydroxyethoxy)phenyl]pyridin-3,5-dicarbonitril
sowie ihre Salze, Solvate und Solvate der Salze.

Ganz besonders bevorzugt im Rahmen der vorliegenden Erfindung sind die nachfolgend genannten Verbindungen
2-Amino-6-({[2-(3,4-difluorphenyl)-1,3-oxazol-4-yl]methyl}sulfanyl)-4-[4-(2-hydroxyethoxy)-phenyl]pyridin-3,5-dicarbonitril;
2-Amino-6-({[2-(4-chlorphenyl)-1,3-oxazol-4-yl]methyl}sulfanyl)-4-(4-{[(2*S*)-2,3-dihydroxypropyl]oxy}phenyl)pyridin-3,5-dicarbonitril;
2-Amino-6-({[2-(4-chlorphenyl)-1,3-oxazol-4-yl]methyl}sulfanyl)-4-(4-{[(2*R*)-2,3-dihydroxypropyl]oxy}phenyl)pyridin-3,5-dicarbonitril;
2-Amino-6-({[2-(4-chlorphenyl)-5-methyl-1,3-oxazol-4-yl]methyl}sulfanyl)-4-(4-{[(2*S*)-2,3-dihydroxypropyl]oxy}phenyl)pyridin-3,5-dicarbonitril;
2-Amino-6-{[2-(4-chlorphenyl)-1,3-oxazol-4-yl]methoxy}-4-[4-(2-hydroxyethoxy)phenyl]pyridin-3,5-dicarbonitril;
2-({[2-(4-Chlorphenyl)-1,3-oxazol-4-yl]methyl}sulfanyl)-4-[4-(2-hydroxyethoxy)phenyl]-6-[(2-hydroxyethyl)amino]pyridin-3,5-dicarbonitril;
2-({[2-(4-Chlorphenyl)-1,3-oxazol-4-yl]methyl}sulfanyl)-4-[4-(2-hydroxyethoxy)phenyl]-6-[(3*R*)-3-hydroxypyrrolidin-1-yl]pyridin-3,5-dicarbonitril
und
2-{[2-(4-Chlorphenyl)-1,3-oxazol-4-yl]methoxy}-6-(3-hydroxyazetidin-1-yl)-4-[4-(2-hydroxyethoxy)phenyl]pyridin-3,5-dicarbonitril
sowie ihre Salze, Solvate und Solvate der Salze.

Weiterer Gegenstand der vorliegenden Erfindung ist ein Verfahren zur Herstellung der erfindungsgemäßen Verbindungen der Formel (I), dadurch gekennzeichnet, dass man eine Verbindung der Formel (II) 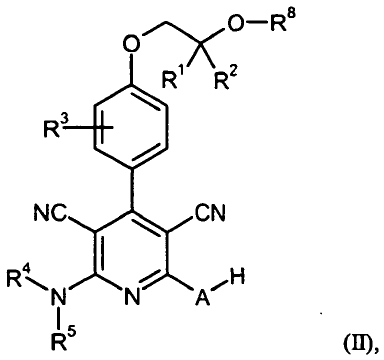 in welcher A, R¹, R², R³, R⁴ und R⁵ jeweils die zuvor angegebenen Bedeutungen haben
und
R⁸ für Wasserstoff oder eine temporäre Hydroxy-Schutzgruppe steht,
in einem inerten Lösungsmittel in Gegenwart einer Base mit einer Verbindung der Formel (III) 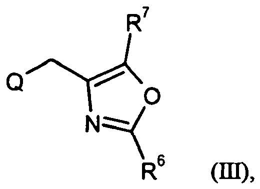 in welcher R⁶ und R⁷ die oben angegebenen Bedeutungen haben und
Q für eine geeignete Abgangsgruppe, vorzugsweise für Halogen, insbesondere Chlor, Brom oder Iod, oder für Mesylat, Tosylat oder Triflat steht,
umsetzt
oder alternativ im Fall, dass A für O steht, eine Verbindung der Formel (IV) 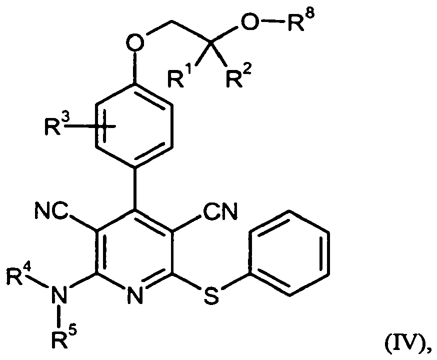 in welcher R¹, R², R³, R⁴, R⁵ und R⁸ jeweils die oben angegebenen Bedeutungen haben,
in einem inerten Lösungsmittel in Gegenwart einer Base mit einer Verbindung der Formel (V) 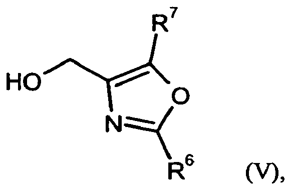 in welcher R⁶ und R⁷ die oben angegebenen Bedeutungen haben,
umsetzt,
anschließend gegebenenfalls vorhandene Schutzgruppen abspaltet und die resultierenden Verbindungen der Formel (I) gegebenenfalls mit den entsprechenden (*i*) Lösungsmitteln und/oder (*ii*) Basen oder Säuren in ihre Solvate, Salze und/oder Solvate der Salze überführt.

In den Verbindungen der Formeln (II) und (IV) bzw. in den Resten R², R⁴ und/oder R⁵ gegebenenfalls vorhandene funktionelle Gruppen - wie insbesondere Amino-, Hydroxy- und Carboxylgruppen - können bei diesem Verfahren, falls zweckmäßig oder erforderlich, auch in temporär geschützter Form vorliegen. Die Einführung und Entfernung solcher Schutzgruppen erfolgt hierbei nach üblichen, dem Fachmann bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer Schutzgruppen kann die Entfernung gegebenenfalls simultan in einer Eintopf-Reaktion oder in separaten Reaktionsschritten vorgenommen werden.

Als Amino-Schutzgruppe wird bevorzugt *tert*.-Butoxycarbonyl (Boc) oder Benzyloxycarbonyl (Z) verwendet. Zum Schutz von Carboxylgruppen eignen sich insbesondere die entsprechenden Methyl-, Ethyl- oder *tert*.-Butylester. Für eine Hydroxy-Funktion wird als Schutzgruppe vorzugsweise Benzyl oder eine Silylgruppe wie Trimethylsilyl, *tert*.-Butyldimethylsilyl oder Dimethylphenylsilyl eingesetzt. Bei Vorliegen einer 1,2- oder 1,3-Diol-Gruppierung wird bevorzugt ein von symmetrischen Ketonen wie Aceton oder Cyclohexanon abgeleitetes Ketal (1,3-Dioxolan bzw. 1,3-Dioxan) als gemeinsame Schutzgruppe verwendet.

Das zuvor beschriebene Verfahren kann durch die folgenden Reaktionsschemata 1 und 2 beispielhaft erläutert werden:

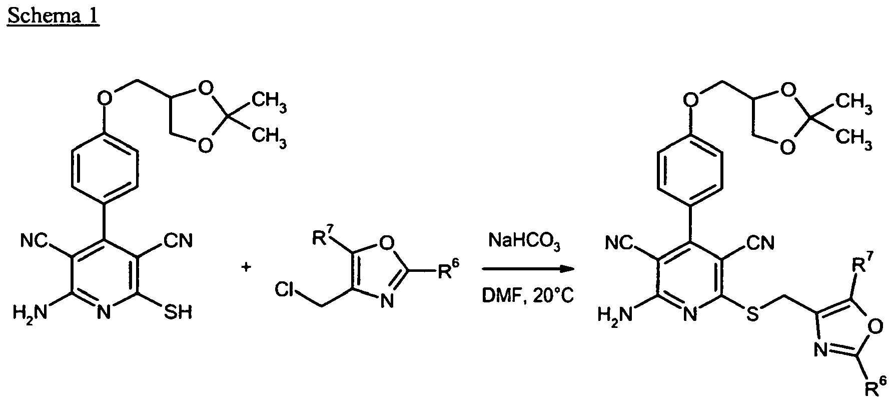

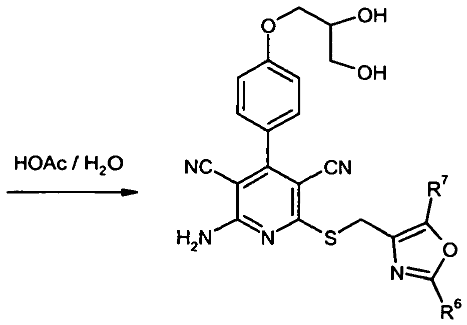

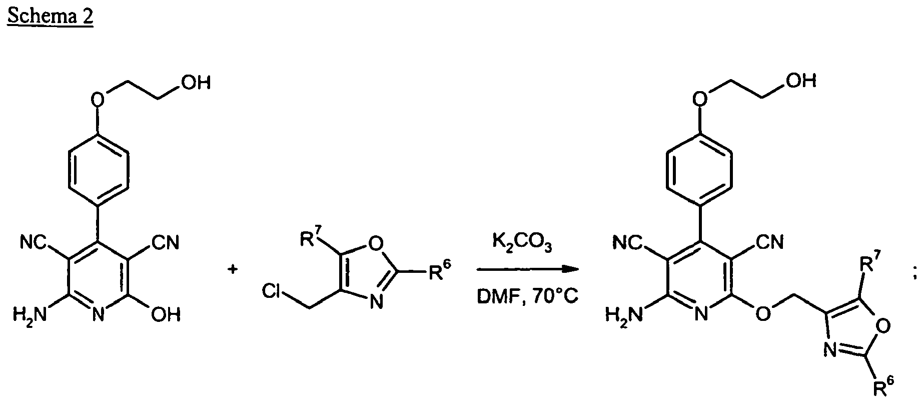

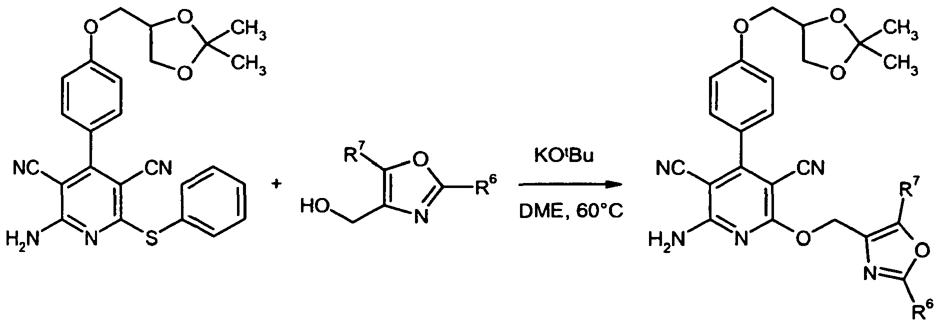

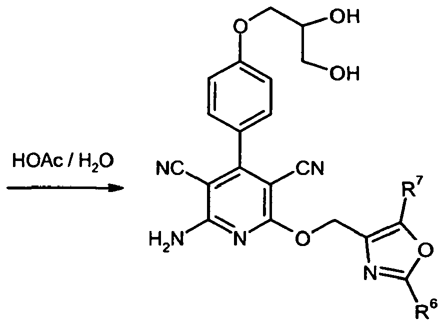

Als Lösungsmittel für die Reaktion (II) + (III) eignen sich alle organischen Lösungsmittel, die unter den Reaktionsbedingungen inert sind. Hierzu gehören Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), *N*-Methylpyrrolidinon (NMP), Acetonitril oder Pyridin. Ebenso ist es möglich, Gemische der zuvor genannten Lösungsmittel einzusetzen. Bevorzugt wird Dimethylformamid verwendet.

Als Basen für diese Umsetzung eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und -hydrogencarbonate wie Kaliumcarbonat und Natriumhydrogencarbonat.

Die Base kann hierbei in einer Menge von 1 bis 10 Mol, bevorzugt von 1 bis 5 Mol, insbesondere von 1 bis 3 Mol, bezogen auf 1 Mol der Verbindung der Formel (II), eingesetzt werden.

Die Reaktion (II) + (III) erfolgt im Allgemeinen in einem Temperaturbereich von -78°C bis +140°C, bevorzugt im Bereich von -20°C bis +100°C, insbesondere bei 0°C bis +60°C (für A = S) bzw. +20°C bis +100°C (für A = O). Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als inerte Lösungsmittel für die Reaktion (IV) + (V) eignen sich insbesondere acyclische und cyclische Ether wie Diethylether, Methyl-tert.-butylether, 1,2-Dimethoxyethan, Tetrahydrofuran und Dioxan, Kohlenwasserstoffe wie Benzol, Toluol, Xylol, Hexan und Cyclohexan, oder dipolare Lösungsmittel wie Dimethylformamid (DMF), Dimethylsulfoxid (DMSO), *N*-Methylpyrrolidinon (NMP) und Pyridin. Ebenso ist es möglich, Gemische dieser Lösungsmittel einzusetzen. Bevorzugt wird 1,2-Dimethoxyethan verwendet.

Als Basen für diese Umsetzung sind insbesondere Alkalialkoholate wie Natrium- oder Kaliummethanolat, Natrium- oder Kaliumethanolat oder Natrium- oder Kalium-tert.-butylat, Amide wie Natriumamid, Lithium-, Natrium- oder Kalium-bis-(trimethylsilyl)amid oder Lithiumdiisopropylamid, oder metallorganische Verbindungen wie Butyllithium oder Phenyllithium geeignet. Bevorzugt wird Kalium-tert.-butylat verwendet.

Die Base wird hierbei in der Regel in einer Menge von 1 bis 1.25 Mol, bevorzugt in äquimolarer Menge, bezogen auf 1 Mol der Verbindung der Formel (V), eingesetzt.

Die Reaktion (IV) + (V) erfolgt im Allgemeinen in einem Temperaturbereich von -20°C bis +120°C, bevorzugt bei +20°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Verbindungen der Formel (II), worin A für S und R⁴ und R⁵ für Wasserstoff stehen, können in Analogie zu literaturbekannten Methoden beispielsweise dadurch hergestellt werden, dass man Aldehyde der Formel (VI) 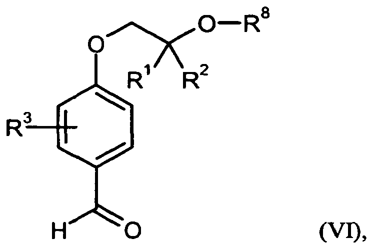 in welcher R¹, R², R³ und R⁸ jeweils die oben angegebenen Bedeutungen haben,
in Gegenwart einer Base mit zwei Äquivalenten Cyanothioacetamid umsetzt [siehe Schema 3; vgl. z.B. Dyachenko et al., Russ. J. Chem. 33 (7), 1014-1017 (1997), 34 (4), 557-563 (1998); Dyachenko et al., Chemistry of Heterocyclic Compounds 34 (2), 188-194 (1998); Qintela et al., Eur. J. Med. Chem. 33, 887-897 (1998); Kandeel et al., Z. Naturforsch. 42b, 107-111 (1987); Reddy et al., J. Med. Chem. 49, 607-615 (2006); Evdokimov et al., Org. Lett. 8, 899-902 (2006)].

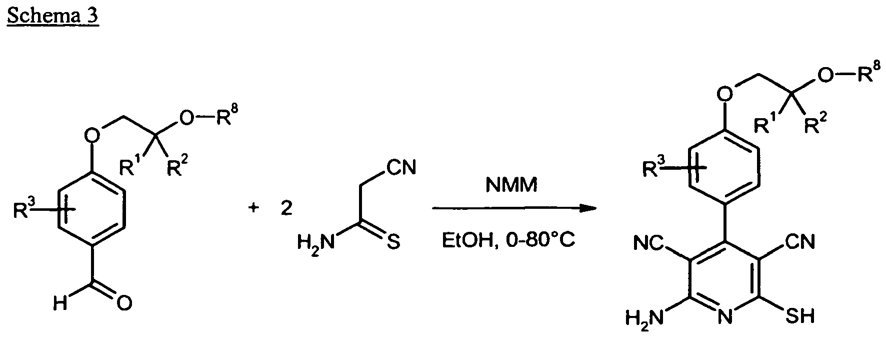

Verbindungen der Formel (II), worin A für S steht, können auch-ausgehend von Verbindungen der Formel (IV) durch Reaktion mit einem Alkalisulfid erhalten werden. Diese Herstellungsmethode wird durch das folgende Formelschema erläutert:

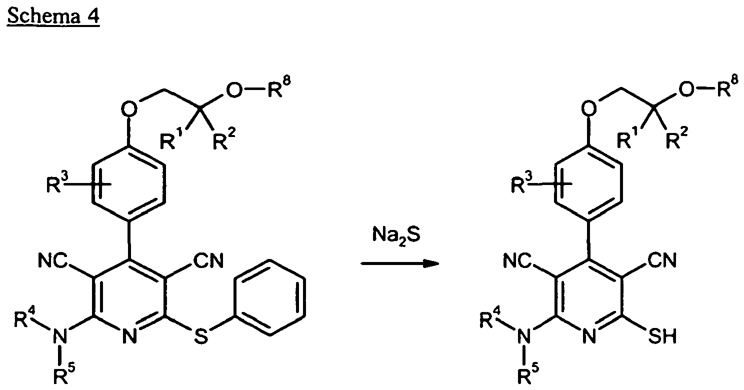

Als Alkalisulfid wird vorzugsweise Natriumsulfid in einer Menge von 1 bis 10 Mol, bevorzugt von 1 bis 8 Mol, insbesondere von 1 bis 5 Mol, bezogen auf 1 Mol der Verbindung der Formel (IV), eingesetzt.

Als Lösungsmittel für diesen Verfahrensschritt eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder dipolare Lösungsmittel wie Acetonitril, Pyridin, Dimethylformamid, Dimethylsulfoxid oder *N*-Methylpyrrolidinon. Wasser ist als Lösungsmittel ebenfalls geeignet. Ebenso ist es möglich, Gemische der zuvor genannten Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +120°C, insbesondere bei +40°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Verbindungen der Formel (IV), worin mindestens einer der beiden Reste R⁴ und R⁵ nicht für Wasserstoff steht, können hergestellt werden, indem man Verbindungen der Formel (IVa) 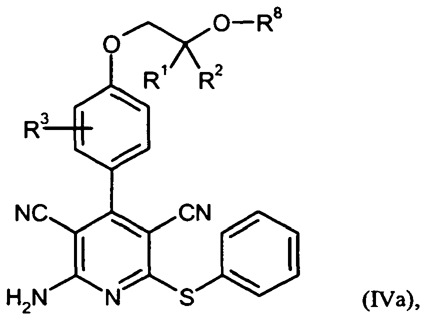 in welcher R¹, R², R³ und R⁸ jeweils die oben angegebenen Bedeutungen haben,
zunächst mit Kupfer(II)chlorid und Isoamylnitrit in einem geeigneten Lösungsmittel in Verbindungen der Formel (VII) 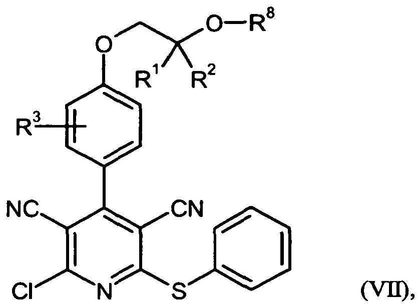 in welcher R¹, R², R³ und R⁸ jeweils die oben angegebenen Bedeutungen haben,
überführt und anschließend mit einer Verbindung der Formel (VIII) 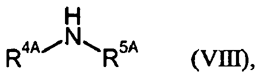 in welcher
R^{4A} die oben angegebene Bedeutung von R⁴ hat,
R^{5A} die oben angegebene Bedeutung von R⁵ hat,
jedoch mindestens einer der beiden Reste nicht für Wasserstoff steht,
zu Verbindungen der Formel (IVb) 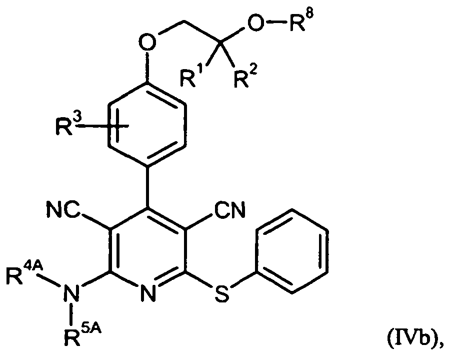 in welcher R¹, R², R³, R^{4A} , R^{5A} und R⁸ jeweils die oben angegebenen Bedeutungen haben,
umsetzt; gegebenenfalls können diese dann mit Hilfe eines Alkalisulfids, wie oben beschrieben, in entsprechende Verbindungen der Formel (II), worin A für S steht und mindestens einer der beiden Reste R⁴ und R⁵ nicht für Wasserstoff steht, überführt werden. Dieses Verfahren kann durch das folgende Reaktionsschema veranschaulicht werden: 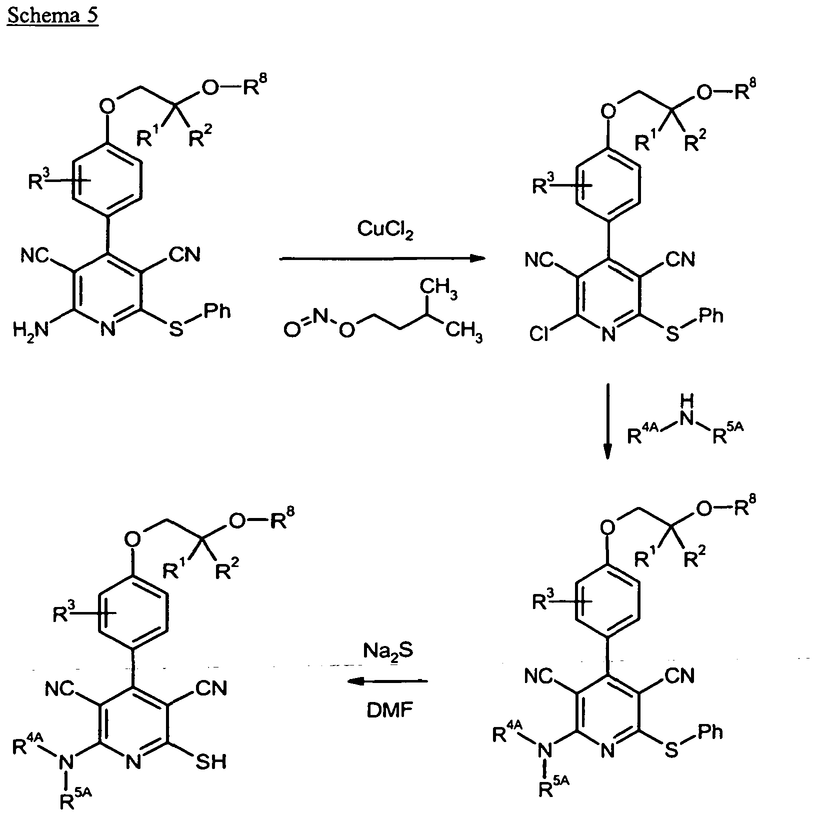 [Ph = Phenyl].

Der Verfahrensschritt (IVa) → (VII) erfolgt im Allgemeinen mit einem Molverhältnis von 2 bis 12 Mol Kupfer(II)chlorid und 2 bis 12 Mol Isoamylnitrit bezogen auf 1 Mol der Verbindung der Formel (IVa).

Als Lösungsmittel für diesen Verfahrensschritt eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische dieser Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von +20°C bis +100°C, insbesondere bei +20°C bis +60°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Der Verfahrensschritt (VII) + (VIII) → (IVb) erfolgt im Allgemeinen mit einem Molverhältnis von 1 bis 8 Mol der Verbindung der Formel (VIII) bezogen auf 1 Mol der Verbindung der Formel (VIII).

Als Lösungsmittel für diesen Verfahrensschritt eignen sich alle organischen Lösungsmittel, die unter den Reaktionsbedingungen inert sind. Hierzu gehören Alkohole wie Methanol, Ethanol, n-Propanol, Isopropanol, n-Butanol und tert.-Butanol, Ketone wie Aceton und Methylethylketon, acyclische und cyclische Ether wie Diethylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril, Pyridin oder Dimethylsulfoxid. Wasser ist als Lösungsmittel ebenfalls geeignet. Ebenso ist es möglich, Gemische dieser Lösungsmittel einzusetzen. Bevorzugtes Lösungsmittel ist Dimethylformamid.

Die Reaktion erfolgt im Allgemeinen in einem Temperaturbereich von 0°C bis +180°C, bevorzugt im Bereich von +20°C bis +120°C, insbesondere bei +20°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Verbindungen der Formel (IVa) können ausgehend von Verbindungen der Formel (VI) in Analogie zu literaturbeschriebenen Verfahren hergestellt werden [vgl. z.B. Kambe et al., Synthesis, 531-533 (1981); Elnagdi et al., Z. Naturforsch. 47b, 572-578 (1991); Reddy et al., J. Med. Chem. 49, 607-615 (2006); Evdokimov et al., Org. Lett. 8, 899-902 (2006)].

Die Verbindungen der Formel (VIII) sind entweder kommerziell erhältlich, dem Fachmann bekannt oder nach üblichen Methoden herstellbar.

Gegebenenfalls können auch Verbindungen der Formel (I), worin R⁴ und R⁵ beide für Wasserstoff stehen, analog zur Reaktionssequenz (IVa) → (VII) → (IVb) in die entsprechenden Verbindungen, worin mindestens einer der beiden Reste R⁴ und R⁵ nicht für Wasserstoff steht, überführt werden, wobei gegebenenfalls ein temporärer Schutz anderer funktioneller Gruppen zweckmäßig ist. Diese Verfahrensvariante wird im folgenden Reaktionsschema veranschaulicht:

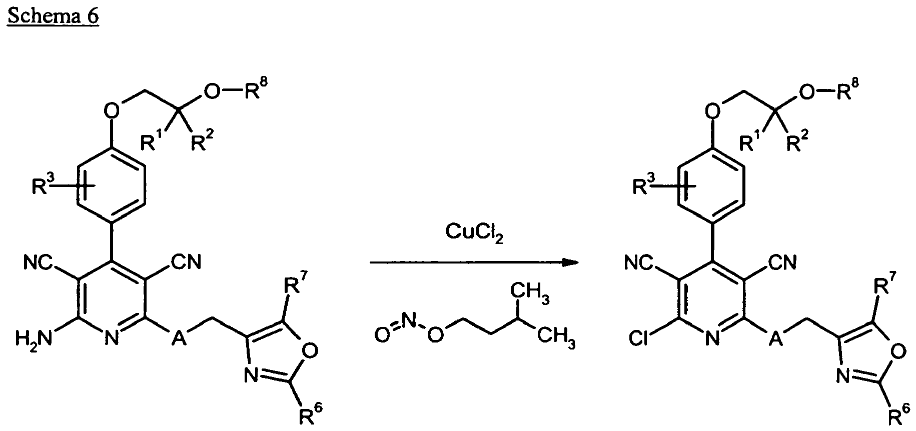

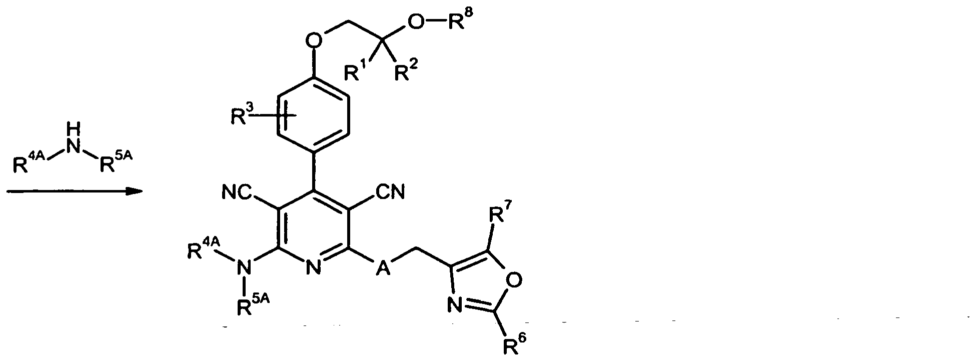

Für diesen Verfahrensweg finden die zuvor für die Sequenz (IVa) → (VII) → (IVb) beschriebenen Reaktionsparameter wie Lösungsmittel, Reaktionstemperaturen und Molverhältnisse in analoger Weise Anwendung.

Verbindungen der Formel (II), worin A für O steht, können ausgehend von Verbindungen der Formel (IV) durch Erhitzen mit einem Alkalihydroxid erhalten werden. Diese Herstellungsmethode wird durch das folgende Reaktionsschema erläutert:

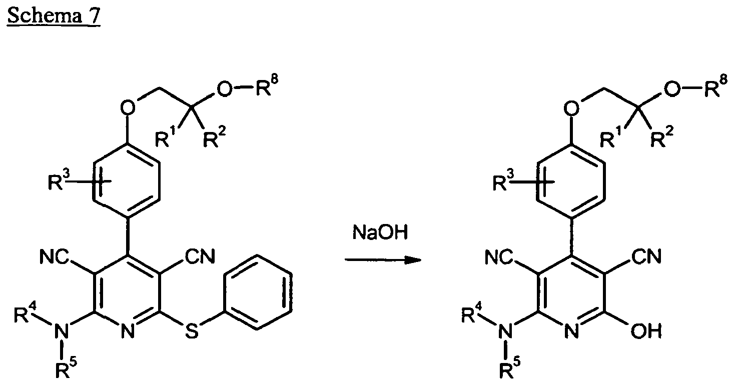

Als Alkalihydroxid wird vorzugsweise überschüssiges Natrium- oder Kaliumhydroxid eingesetzt. Als Lösungsmittel sind insbesondere Alkohole wie Methanol, Ethanol, n-Propanol, Isopropanol, n-Butanol und tert.-Butanol sowie deren Mischungen mit Wasser geeignet. Die Umsetzung erfolgt im Allgemeinen in einem Temperaturbereich von +20°C bis +120°C, bevorzugt bei +50°C bis +100°C.

Die Verbindungen der Formel (III) sind kommerziell erhältlich, literaturbekannt oder nach literaturbekannten Methoden herstellbar. So können beispielsweise durch Reaktion von Amiden mit einem 1,3-Dihalogenaceton 2-substituierte Oxazol-Derivate erhalten werden (siehe Schema 8): 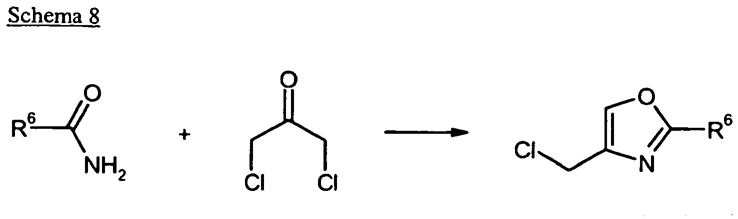 2,5-Disubstituierte Oxazol-Derivate gemäß Formel (III) können in Analogie zu literaturbekannten Verfahren beispielsweise wie im folgenden Reaktionsschema 9 exemplarisch beschrieben hergestellt werden: 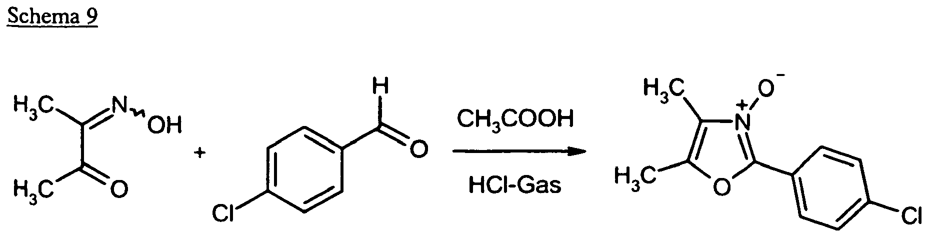 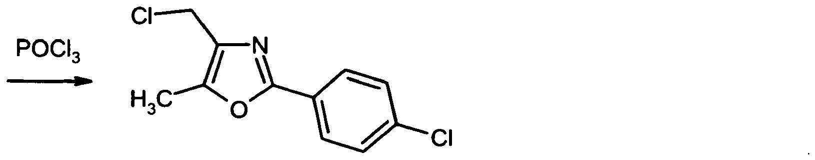 [vgl. z.B. Y. Goto et al., Chem. Pharm. Bull. 1971, 19, 2050-2057].

In 5-Position substituierte Oxazol-Derivate gemäß Formel (III) können beispielsweise durch Reduktion und nachfolgende Halogenierung entsprechender Oxazol-4-carbonsäureester erhalten werden, welche ihrerseits durch Acylierung von α-Isocyanatoacetaten zugänglich sind (siehe Schema 10): 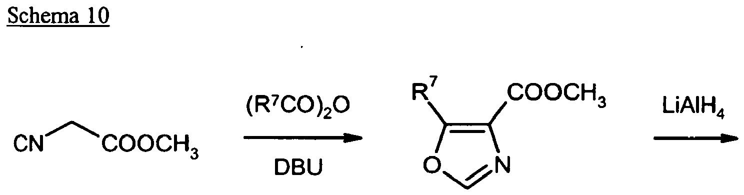 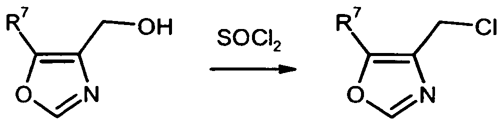 [vgl. z.B. M. Suzuki et al., J. Org. Chem. 1973, 38, 3571-3575].

2-Aryloxazol-Derivate gemäß Formel (III) sind auch über eine Palladium-katalysierte Kupplung von Arylboronsäuren mit 2-Iodoxazol-4-carbonsäureestern erhältlich, wie in Schema 11 beispielhaft dargestellt ist: 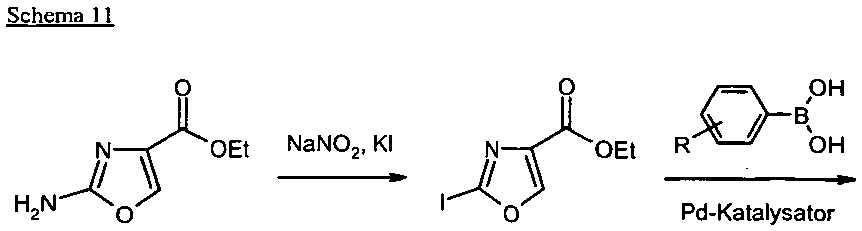 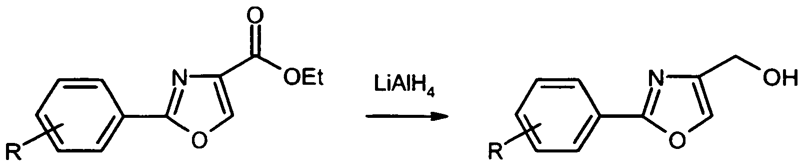 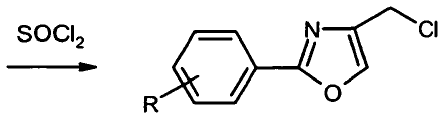 [vgl. z.B. E.A. Krasnokutskaya et al., Synthesis 2007, 1, 81-84; J. Hassan et al., Chem. Rev. 2002, 102, 1359-1469].

Die Verbindungen der Formel (VI) schließlich sind literaturbekannt oder können nach üblichen Verfahren ausgehend von entsprechenden 4-Hydroxybenzaldehyden hergestellt werden (siehe Schema 12):

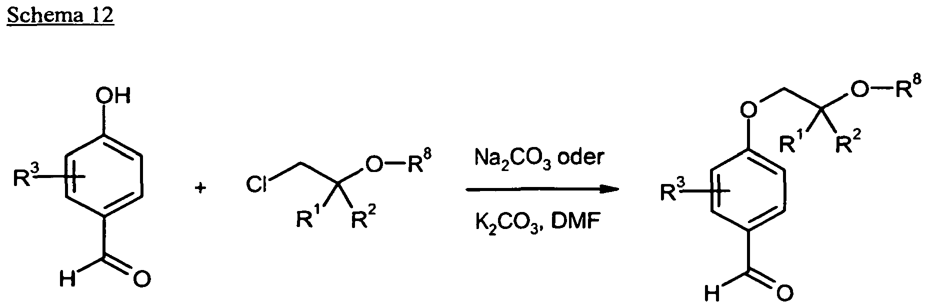

Überraschenderweise zeigen die erfindungsgemäßen Verbindungen ein nicht vorhersehbares, wertvolles pharmakologisches Wirkspektrum und sind daher in besonderem Maße zur Prophylaxe und/ oder Behandlung von Erkrankungen, insbesondere von kardiovaskulären Erkrankungen, geeignet.

Gegenüber den aus dem Stand der Technik bekannten Substanzen weisen die erfindungsgemäßen Verbindungen ein verbessertes Eigenschaftsprofil auf, wie beispielsweise eine erhöhte Löslichkeit in wässrig-organischen, für die Formulierung relevanten Lösungsmittelsystemen, eine längere pharmakokinetische Halbwertszeit nach oraler Gabe und/oder eine erhöhte metabolische Stabilität.

Die pharmakologische Wirksamkeit der erfindungsgemäßen Verbindungen lässt sich über ihre Wirkung als potente, selektive Liganden an Adenosin A1- und/oder A2b-Rezeptoren erklären. Sie agieren hierbei als selektive A1-Agonisten oder als selektive duale A1/A2b-Agonisten.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung an A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Die erfindungsgemäßen Verbindungen fungieren in Abhängigkeit von ihrer jeweiligen Struktur als volle oder als partielle Adenosinrezeptor-Agonisten. Partielle Adenosinrezeptor-Agonisten sind hierbei definiert als Rezeptorliganden, die eine funktionelle Antwort an Adenosinrezeptoren auslösen, welche geringer ist als bei vollen Agonisten (wie beispielsweise Adenosin selbst). Partielle Agonisten weisen infolgedessen eine geringere Wirksamkeit bezüglich der Rezeptoraktivierung auf als volle Agonisten.

Die Verbindungen der Formel (I) sind allein oder in Kombination mit einem oder mehreren anderen Wirkstoffen zur Prophylaxe und/oder Behandlung verschiedener Erkrankungen geeignet, so beispielsweise insbesondere von Hypertonie und anderen Erkrankungen des Herzkreislauf-Systems (kardiovaskulären Erkrankungen), zur Kardioprotektion nach Schädigungen des Herzens sowie von Stoffwechsel-Erkrankungen.

Im Sinne der vorliegenden Erfindung sind unter Erkrankungen des Herzkreislauf-Systems bzw. kardiovaskulären Erkrankungen neben der Hypertonie beispielsweise die folgenden Erkrankungen zu verstehen: periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, akutes Koronarsyndrom mit ST-Erhebung, akutes Koronarsyndrom ohne ST-Erhebung, stabile und instabile Angina pectoris, Herzmuskelschwäche, Prinzmetal-Angina, persistierende ischämische Dysfunktion ("hibernating myocardium"), vorübergehende postischämische Dysfunktion ("stunned myocardium"), Herzinsuffizienz, Tachykardien, atriale Tachykardie, Arrhythmien, Vorhof- und Kammerflimmern, persistierendes Vorhofflimmern, permanentes Vorhofflimmern, Vorhofflimmern mit normaler linksventrikulärer Funktion, Vorhofflimmem mit eingeschränkter linksventrikulärer Funktion, Wolff-Parkinson-White-Syndrom, periphere Durchblutungsstörungen, erhöhte Spiegel von Fibrinogen und von LDL geringer Dichte sowie erhöhte Konzentrationen von Plasminogenaktivator-Inhibitor 1 (PAI-1), insbesondere Hypertonie, koronare Herzerkrankung, akutes Koronarsyndrom, Angina pectoris, Herzinsuffizienz, Myokardinfarkt und Vorhofflimmern.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Weiterhin eignen sich die erfindungsgemäßen Verbindungen insbesondere auch zur Reduktion des von einem Infarkt betroffenen Myokardbereichs sowie zur Prophylaxe von Sekundärinfarkten.

Des weiteren sind die erfindungsgemäßen Verbindungen insbesondere zur Prophylaxe und/oder Behandlung von thromboembolischen Erkrankungen, Reperfusionsschäden nach Ischämie, mikro- und makrovaskulären Schädigungen (Vaskulitis), arteriellen und venösen Thrombosen, Ödemen, Ischämien wie Myokardinfarkt, Hirnschlag und transitorischen ischämischen Attacken, zur Kardioprotektion bei Koronararterien-Bypass-Operationen (CABG), primären perkutan-transluminalen Koronarangioplastien (PTCAs), PTCAs nach Thrombolyse, Rescue-PTCA, Herztransplantationen und Operationen am offenen Herzen, sowie zur Organprotektion bei Transplantationen, Bypass-Operationen, Katheteruntersuchungen und anderen operativen Eingriffen geeignet.

Weitere Indikationsgebiete, für die die erfindungsgemäßen Verbindungen verwendet werden können, sind beispielsweise die Prophylaxe und/oder Behandlung von Erkrankungen des Urogenitalbereiches, wie z.B. Reizblase, erektile Dysfunktion und weibliche sexuelle Dysfunktion, daneben aber auch die Prophylaxe und/oder Behandlung von inflammatorischen Erkrankungen, wie z.B. Asthma und entzündliche Dermatosen, von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzuständen, sowie von Krebserkrankungen und von Übelkeit und Erbrechen in Verbindung mit Krebstherapien.

Ein weiteres Indikationsgebiet sind beispielsweise die Prophylaxe und/oder Behandlung von Erkrankungen der Atemwege wie beispielsweise Asthma, chronisch-obstruktive Atemwegserkrankungen (COPD, chronische Bronchitis), Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prophylaxe und/oder Behandlung von Stoffwechsel-Erkrankungen in Betracht, wie beispielsweise Diabetes, insbesondere Diabetes mellitus, Gestationsdiabetes, Insulin-abhängiger Diabetes und Nicht-Insulin-abhängiger Diabetes, diabetische Folgeerkrankungen wie z.B. Retinopathie, Nephropathie und Neuropathie, metabolische Erkrankungen wie z.B. Metabolisches Syndrom, Hyperglykämie, Hyperinsulinämie, Insulinresistenz, Glukose-Intoleranz und Fettsucht (Adipositas), sowie Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten) sowie Analgetika wie beispielsweise Aspirin.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshemmer, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Diuretika, Aldosteron-Antagonisten, Mineralocorticoid-Rezeptor-Antagonisten, ECE-Inhibitoren sowie der Vasopeptidase-Inhibitoren;
- antithrombotisch wirkenden Mitteln, beispielhaft und vorzugsweise aus der Gruppe der Thrombozytenaggregationshemmer oder der Antikoagulantien;
- Vasopressin-Rezeptor-Antagonisten;
- organischen Nitraten und NO-Donatoren;
- positiv-inotrop wirksamen Verbindungen;
- Verbindungen, die den Abbau von cyclischern Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost und Cicaprost;
- Calcium-Sensitizern, wie beispielhaft und vorzugsweise Levosimendan;
- Kalium-Supplements;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden. Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfindungsgemäßen Verbindungen in Kombination mit einem Agonisten des Niacin-Rezeptors, wie beispielhaft und vorzugsweise Niacin, Acipimox, Acifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylharnstoffe, Biguanide, Meglitinid-Derivate, Glukosidase-Inhibitoren, DPP-IV-Inhibitoren und PPAR-γ-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin oder Vildagliptin, verabreicht.

Bei einer bevorzugten Ausführungsform der Erfindung werden die erfindungsgemäßen Verbindungen in Kombination mit einem PPAR-γ-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Glycerinnitrat, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, oder in Kombination mit Kaliumkanal-Agonisten wie Minoxidil, Diazoxid, Dihydralazin oder Hydralazin verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralocorticoid-Rezeptor-Antagonisten, Vasopressin-Rezeptor-Antagonisten, Thrombozytenaggregationshemmer und Antikoagulantien, sowie deren Verwendung zur Behandlung und/oder Prophylaxe der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfmdungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- Bsp.: Beispiel
- DBU: 1,8-Diazabicyclo[5.4.0]undec-7-en
- DC: Dünnschichtchromatographie
- DCI: direkte chemische Ionisation (bei MS)
- DME: 1,2-Dimethoxyethan
- DMF: *N,N*-Dimethylformamid
- DMSO: Dimethylsulfoxid
- d. Th.: der Theorie (bei Ausbeute)
- EE: Ethylacetat (Essigsäureethylester)
- EI: Elektronenstoß-Ionisation (bei MS)
- ESI: Elektrospray-Ionisation (bei MS)
- Et: Ethyl
- EtOH: Ethanol
- Fp.: Schmelzpunkt
- ges.: gesättigt
- h: Stunde(n)
- HOAc: Essigsäure
- HPLC: Hochdruck-, Hochleistungsflüssigchromatographie
- konz.: konzentriert
- KO'Bu: Kalium-*tert*.-butylat
- LC-MS: Flüssigchromatographie-gekoppelte Massenspektrometrie
- LDA: Lithiumdiisopropylamid
- Lit.: Literatur(stelle)
- Lsg.: Lösung
- min: Minute(n)
- MS: Massenspektrometrie
- NMM: *N*-Methylmorpholin
- NMR: Kernresonanzspektrometrie
- PBS: Phosphat-gepufferte Kochsalz-Lösung
- PEG: Polyethylenglykol
- Ph: Phenyl
- RP-HPLC: reverse phase HPLC
- RT: Raumtemperatur
- Rₜ: Retentionszeit (bei HPLC)
- THF: Tetrahydrofuran
- verd.: verdünnt
- wässr.: wässrig

### HPLC- und LC-MS-Methoden:

### Methode 1 (HPLC):

Instrument: Hewlett Packard Series 1050; Säule: Symmetry TM C18 3.9 x 150 mm; Fluss: 1.5 ml/min; Eluent A: Wasser, Eluent B: Acetonitril; Gradient: → 0.6 min 10% B→ 3.8 min 100% B → 5.0 min 100% B→ 5.5 min 10% B; Stopzeit: 6.0 min; Injektionsvolumen: 10 µl; Diodenarraydetektor-Signal: 214 und 254 nm.

### Methode 2 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl50%-ige Ameisensäure / 1; Gradient: 0.0 min 10% B → 7.0 min 95% B→ 9.0 min 95% B; Ofen: 35°C; Fluss: 0.0 min 1.0 ml/min → 7.0 min 2.0 ml/min→ 9.0 min 2.0 ml/min; UV-Detektion: 210 nm.

### Methode 3 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A→ 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS):

Instrument: Micromass Quattro LCZ mit HPLC Agilent Serie 1100; Säule: Phenomenex Onyx Monolithic C18, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2 min 65% A→4.5 min 5% A → 6 min 5% A; Fluss: 2 ml/min; Ofen: 40°C; UV-Detektion: 208-400 nm.

### Methode 5 (LC-MS):

Gerätetyp MS: Waters ZQ; Gerätetyp HPLC: Waters Alliance 2795; Säule: Merck Chromolith RP-18e, 100 mm x 3 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A→ 2 min 65% A→ 4.5 min 5% A → 6 min 5% A; Fluss: 2 ml/min; Ofen: 40°C; UV-Detektion: 210 nm.

### Methode 6 (LC-MS):

Instrument: Micromass Quattro LCZ mit HPLC Agilent Serie 1100; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 l min 90% A → 3.0 min 5% A → 4.0 min 5% A → 4.1 min 90% A; Fluss: 2 ml/min; Ofen: 50°C; UV-Detektion: 208-400 nm.

### Methode 7 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 8 (LC-MS):

Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 0.2 min 100% A → 2.9 min 30% A → 3.1 min 10% A → 5.5 min 10% A; Fluss: 0.8 ml/min; Ofen: 50°C; UV-Detektion: 210 nm.

### Methode 9 (LC-MS):

Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%-ige Ameisensäure, Eluent B: 11 Acetonitril +0.5 ml 50%-ige Ameisensäure; Gradient: 0.0 min 90% A → 2.5 min 30% A → 3.0 min 5% A →4.5 min 5% A; Fluss: 0.0 min 1 ml/min → 2.5 min/3.0 min/4.5 min 2 ml/min; Ofen: 50°C; UV-Detektion: 208-400 nm.

### Methode 10 (LC-MS):

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 11 (LC-MS):

Gerätetyp MS: Micromass ZQ; Gerätetyp HPLC: HP 1100 Series; UV DAD; Säule: Phenomenex Synergi 2.5µ MAX-RP 100A Mercury 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 3.0 min 5% A → 4.0 min 5% A → 4.1 min 90% A; Fluss: 2 m1/min; Ofen: 50°C; UV-Detektion: 210 nm.

### Methode 12 (LC-MS):

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1; Eluent B: Acetonitril + 500 µl 50%-ige Ameisensäure / 1; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### Methode 13 (LC-MS):

Gerätetyp MS: M-40 DCI (NH₃); Gerätetyp HPLC: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 14 (LC-MS):

Instrument: Micromass QuattroPremier mit Waters UPLC Acquity; Säule: Thermo Hypersil GOLD 1.9µ 50 mm x 1 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 15 (LC-MS):

Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%-ige Ameisensäure, Eluent B: 1 l Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: . 2 ml/min; UV-Detektion: 210 nm.

### Methode 16 (präparative HPLC):

Gerätetyp HPLC: Abimed/Gilson Pump 305/306; Manometric Module 806; UV Knauer Variable Wavelenght Monitor; Säule: Gromsil C18, 10 nm, 250 mm x 30 mm; Eluent A: 1 l Wasser + 0.5 ml 99%-ige Trifluoressigsäure, Eluent B: 1 l Acetonitril; Gradient: 0.0 min 2% B → 10 min 2% B → 50 min 90% B; Fluss: 20 ml/min; Volumen: 628 ml A und 372 ml B.

### Methode 17 (HPLC):

Gerätetyp HPLC: Agilent 1100 mit DAD-Detektion; Säule: Merck Chromolith SpeedROD RP-18e, 50 mm x 4.6 mm; Eluent A: 0.05% H₃PO₄, Eluent B: Acetonitril; Gradient: 0 min 5% B → 2.5 min 95% B → 3.0 min 95% B; Fluss: 5 ml/min; Säulentemperatur: 40°C; UV-Detektion: 210 nm.

### Ausgangsverbindungen und Intermediate:

### Beispiel 1A

2-Amino-4-[4-(2-hydroxyethoxy)phenyl]-6-mercaptopyridin-3,5-dicarbonitril 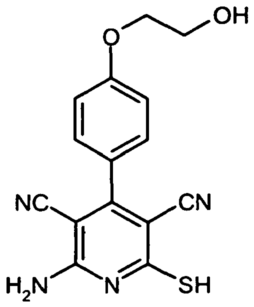 14.90 g (89.66 mmol) 4-(2-Hydroxyethoxy)-benzaldehyd und 17.96 g (179.33 mmol) Cyanothioacetamid werden in 280 ml Ethanol vorgelegt. Anschließend werden 18.14 g (179.33 mmol) 4-Methylmorpholin zugegeben. Das Reaktionsgemisch wird 4 h unter Rückfluss erhitzt und dann weitere 20 h bei RT nachgerührt. Der ausgefallene Niederschlag wird abgesaugt, mit ca. 20 ml Ethanol nachgewaschen und getrocknet.

Ausbeute: 9.90 g (35% d. Th.)

LC-MS (Methode 10): Rₜ = 1.63 min; MS (ESIpos): m/z = 313 [M+H]⁺.

### Beispiel 2A

4-(2-Hydroxy-2-methylpropoxy)benzaldehyd 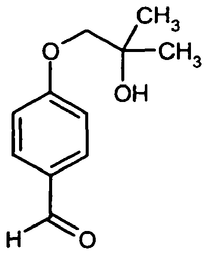 5.00 g (40.94 mmol) 4-Hydroxybenzaldehyd, 4.44 g (40.94 mmol) 1-Chlor-2-methyl-2-propanol und 6.08 g (57.32 mmol) Natriumcarbonat werden in 50 ml trockenem DMF vorgelegt und 24 h unter Rückfluss gerührt. Nach Abkühlen auf RT wird mit 20 ml Ethylacetat und 20 ml ges. wässriger Natriumhydrogencarbonat-Lösung versetzt. Die Phasen werden getrennt, und die organische Phase wird über Magnesiumsulfat getrocknet. Nach Entfernen des Lösungsmittels wird der Rückstand mittels Säulenchromatographie an Kieselgel 60 aufgereinigt (Laufmittel-Gradient: Cyclohexan/Ethylacetat 5:1 → 1:1). Man erhält einen rötlichen Feststoff, welcher ohne weitere Reinigung in der Folgestufe eingesetzt wird.

Ausbeute: 4.40 g (50% d. Th., 90% Reinheit)

LC-MS (Methode 2): Rₜ = 1.37 min; MS (ESIpos): m/z = 195 [M+H]⁺.

### Beispiel 3A

2-Amino-4-[4-(2-hydroxy-2-methylpropoxy)phenyl]-6-mercaptopyridin-3,5-dicarbonitril 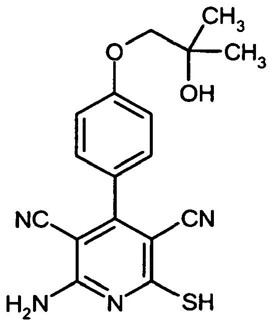 3.38 g (15.49 mmol) der Verbindung aus Beispiel 2A und 3.26 g (32.52 mmol) Cyanothioacetamid werden in 50 ml Ethanol vorgelegt. Anschließend werden 3.13 g (30.98 mmol) 4-Methylmorpholin zugegeben. Es wird 6 h unter Rühren zum Rückfluss erhitzt. Nach Abkühlen auf RT wird 20 h bei dieser Temperatur nachgerührt. Der Ansatz wird dann mit 50 ml ges. wässriger Natriumhydrogencarbonat-Lösung versetzt und viermal mit je 50 ml Ethylacetat extrahiert. Die vereinigten organischen Phasen werden über Magnesiumsulfat getrocknet. Nach Entfernen des Lösungsmittels wird der Rückstand mittels Säulenchromatographie an Kieselgel 60 aufgereinigt (Laufmittel-Gradient: Cyclohexan/Ethylacetat 2:1 → 1:4). Das erhaltene Produkt wird ohne weitere Reinigung in der Folgestufe eingesetzt.

Ausbeute: 0.92 g (16% d. Th., 90% Reinheit)

¹H-NMR (400 MHz, DMSO-d₆): δ = 13.00-12.91 (br. s, 1H), 8.09-7.78 (br. s, 2H), 7.46 (d, 2H), 7.09 (d, 2H), 4.68 (s, 1H), 3.79 (s, 2H), 1.22 (s, 6H).

LC-MS (Methode 2): Rₜ = 1.46 min; MS (ESIpos): m/z = 341 [M+H]⁺.

### Beispiel 4A

(2S)-1-Chlorpropan-2-ol 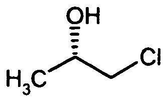 32.50 g (766.66 mmol) Lithiumchlorid werden in 100 ml THF vorgelegt. Anschließend werden langsam 18.75 ml (92.97 mmol) 4.96 M Salzsäure zugegeben. Das Gemisch wird auf -30°C abgekühlt und tropfenweise mit einer Lösung von 5.40 g (92.97 mmol) *S*-(-)-Propylenoxid in 10 ml THF versetzt. Nach beendeter Zugabe wird auf RT erwärmt und 20 h nachgerührt. Der entstandene Niederschlag wird abgesaugt und das Filtrat fraktioniert destilliert (61 mbar, 30-40°C Kopftemperatur). Das so erhaltene Produktgemisch wird ohne weitere Reinigung in der Folgereaktion eingesetzt.

Ausbeute: 4.50 g (16% d. Th., 32% Reinheit)

¹H-NMR (400 MHz, DMSO-d₆): δ = 5.01 (d, 1H), 3.85-3.74 (m, 1H), 3.49 (d, 2H), 1.12 (d, 3H). Das Produkt enthält ca. 10% des Regioisomers (2S)-2-Chlorpropan-1-ol:

¹H-NMR (400 MHz, DMSO-d₆): δ = 5.18-5.12 (m, 1H), 4.11-4.03 (m, 1H), 3.49 (d, 2H), 1.41 (d, 3H).

### Beispiel 5A

4-{[(2S)-2-Hydroxypropyl]oxy})benzaldehyd 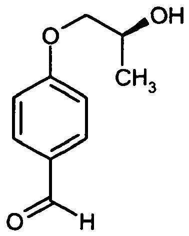 6.30 g (51.62 mmol) 4-Hydroxybenzaldehyd und 4.88 g (51.62 mmol) des Produkts aus Beispiel 4A werden in 100 ml trockenem DMF gelöst. Die Lösung wird mit 16.41 g (154.85 mmol) Natriumcarbonat versetzt und 20 h lang bei 130°C gerührt. Der Ansatz wird nach Abkühlen auf RT mit 100 ml Ethylacetat und 50 ml ges. wässriger Natriumhydrogencarbonat-Lösung versetzt. Es wird dreimal mit je 50 ml Ethylacetat extrahiert. Die vereinigten organischen Phasen werden über Magnesiumsulfat getrocknet. Nach Entfernen des Lösungsmittels wird der Rückstand mittels Säulenchromatographie an Kieselgel 60 aufgereinigt (Laufmittel-Gradient: Cyclohexan/Ethylacetat 5:1→2:1).

Ausbeute: 2.40 g (26% d. Th.)

¹H-NMR (400 MHz, DMSO-d₆): δ = 7.86 (d, 2H), 7.13 (d, 2H), 4.95 (d, 1H), 4.02-3.92 (m, 1H), 3.91 (d, 2H), 1.16 (d, 3H).

LC-MS (Methode 2): Rₜ = 1.19 min; MS (ESIpos): m/z = 181 [M+H]⁺.

Das Produkt enthält ca. 10% des Regioisomers 4-[(1*S*)-2-Hydroxy-1-methylethoxy]benzaldehyd.

### Beispiel 6A

4-{[(2S)-2-{[*tert*.-Butyl(dimethyl)silyl]oxylpropyl]oxy}benzaldehyd 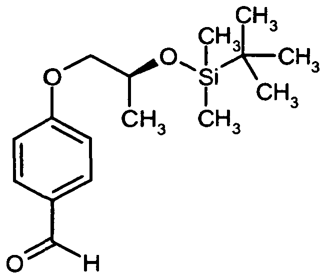 2.40 g (13.32 mmol) der Verbindung aus Beispiel 5A werden in 60 ml trockenem DMF vorgelegt und mit 2.81 g (18.65 mmol) *tert*.-Butyldimethylsilylchlorid sowie 1.72 g (25.30 mmol) Imidazol versetzt. Das Reaktionsgemisch wird 20 h bei RT gerührt. Der Ansatz wird dann mit ca. 30 ml Diethylether und 30 ml ges. wässriger Natriumhydrogencarbonat-Lösung versetzt. Die Phasen werden getrennt und die wässrige Phase zweimal mit je 30 ml Diethylether extrahiert. Die vereinigten organischen Phasen werden über Magnesiumsulfat getrocknet und das Lösungsmittel am Rotationsverdampfer entfernt. Der Rückstand wird mittels Säulenchromatographie an Kieselgel 60 aufgereinigt (Laufmittel-Gradient: Cyclohexan/Ethylacetat 50:1 → 10:1).

Ausbeute: 1.95 g (50% d. Th.)

¹H-NMR (400 MHz, DMSO-d₆): δ = 7.82 (d, 2H), 7.07 (d, 2H), 4.18-4.11 (m, 1H), 3.98 (dd, 1H), 3.87 (dd, 1H), 1.13 (d, 3H), 0.81 (s, 9H), 0.3 (s, 3H), 0.1 (s, 3H).

LC-MS (Methode 9): Rₜ = 3.30 min; MS (ESIpos): m/z = 295 [M+H]⁺.

Das Produkt enthält ca. 10% des Regioisomers 4-[(1*S*)-2-{[*tert*.-Butyl(dimethyl)silyl]oxy}-1-methylethoxy]benzaldehyd.

### Beispiel 7A

2-Amino-4-(4-{[(2*S*)-2-{[*tert*.-butyl(dimethyl)silyl]oxy}propyl]oxy}phenyl)-6-mercaptopyndin-3,5-dicarbonitril 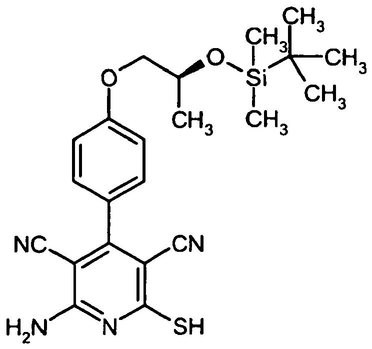 1.95 g (6.62 mmol) der Verbindung aus Beispiel 6A und 1.39 g (13.91 mmol) Cyanothioacetamid werden in 27 ml Ethanol vorgelegt und mit 1.34 g (13.24 mmol) 4-Methylmorpholin versetzt. Es wird 6 h unter Rückfluss erhitzt (Ölbadtemperatur 100°C). Anschließend wird weitere 20 h bei RT nachgerührt. Nach Entfernen des Lösungsmittels am Rotationsverdampfer wird der Rückstand direkt mittels Säulenchromatographie an Kieselgel 60 aufgereinigt (Laufmittel-Gradient: Dichlormethan/Ethanol 50:1 → 5:1).

Ausbeute: 1.30 g (29% d. Th., 65% Reinheit)

¹H-NMR (400 MHz, DMSO-d₆): δ = 7.68-7.48 (br. s, 2H), 7.42 (d, 2H), 7.06 (d, 2H), 4.23-4.15 (m, 1H), 3.96 (dd, 1H), 3.88 (dd, 1H), 1.20 (d, 3H), 0.89 (s, 9H), 0.09 (s, 3H), 0.07 (s, 3H).

LC-MS (Methode 2): Rₜ = 2.79 min; MS (ESIpos): m/z = 441 [M+H]⁺.

Das Produkt enthält ca. 10% des Regioisomers 2-Amino-4-{4-[(1*S*)-2-{[*tert*.-butyl(dimethyl)silyl]-oxy}-1-methylethoxy]phenyl}-6-mercaptopyridin-3,5-dicarbonitril.

### Beispiel 8A

4-{[(4*R*)-2,2-Dimethyl-1,3-dioxolan-4-yl]methoxy}benzaldehyd 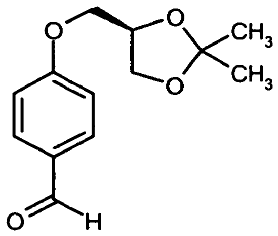 31.2 g (255.4 mmol) 4-Hydroxybenzaldehyd werden in 400 ml trockenem DMF vorgelegt und bei RT mit 105.7 g (766.1 mmol) Kaliumcarbonat sowie 50.0 g (332.0 mmol) (*S*)-(-)-3-Chlor-1,2-propandiol-Acetonid versetzt. Es wird 16 h bei 160°C gerührt. Der Ansatz wird dann mit 4000 ml Wasser versetzt und dreimal mit je 500 ml Ethylacetat extrahiert. Die vereinigten organischen Phasen werden jeweils einmal mit 500 ml Wasser und 500 ml ges. wässriger Natriumchlorid-Lösung gewaschen. Nach Trocknen über Magnesiumsulfat wird das Lösungsmittel am Rotationsverdampfer entfernt und der Rückstand mittels Säulenchromatographie an Kieselgel 60 aufgereinigt (Laufmittel-Gradient: Ethylacetat/Petrolether 1:9 → 2:8).

Ausbeute: 40.4 g (63% d. Th.)

¹H-NMR (400 MHz, DMSO-d₆): δ = 9.90 (s, 1H), 7.85 (d, 2H), 7.03 (d, 2H), 4.50 (q, 1H), 4.22-4.09 (m, 2H), 4.04 (dd, 1H), 3.92 (dd, 1H), 1.48 (s, 3H), 1.41 (s, 3H).

LC-MS (Methode 13): Rₜ = 3.97 min; MS (ESIpos): m/z = 254 [M+NH₄]⁺.

### Beispiel 9A

4-{[(4*S*)-2,2-Dimethyl-1,3-dioxolan-4-yl]methoxy}benzaldehyd

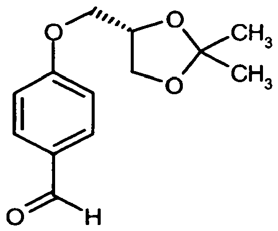

Die Titelverbindung wird aus den entsprechenden Ausgangsverbindungen analog zu Beispiel 8A hergestellt.

Ausbeute: 79% d. Th.

¹H-NMR (400 MHz, DMSO-d₆): δ = 9.89 (s, 1H), 7.85 (d, 2H), 7.03 (d, 2H), 4.50 (q, 1H), 4.22-4.09 (m, 2H), 4.04 (dd, 1H), 3.92 (dd, 1H), 1.48 (s, 3H), 1.41 (s, 3H).

LC-MS (Methode 13): Rₜ = 4.02 min; MS (ESIpos): m/z = 254 [M+NH₄]⁺.

### Beispiel 10A

2-Amino-4-(4-{[(4*R*)-2,2-dimethyl-1,3-dioxolan-4-yl)methoxy}phenyl)-6-mercaptopyridin-3,5-dicarbonitril 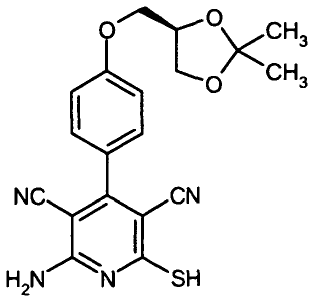 40.4 g (171.0 mmol) der Verbindung aus Beispiel 8A und 34.2 g (342.0 mmol) Cyanothioacetamid werden in 700 ml Ethanol vorgelegt. Das Reaktionsgemisch wird mit 34.5 g (342.0 mmol) 4-Methylmorpholin versetzt und 3 h unter Rühren zum Rückfluss erhitzt. Nach Abkühlen auf RT wird weitere 16 h bei dieser Temperatur nachgerührt. Der ausgefallene Niederschlag wird abgesaugt, mit ca. 100 ml Ethanol gewaschen und im Trockenschrank getrocknet. Das Produkt wird ohne weitere Reinigung in den Folgereaktionen eingesetzt.

Ausbeute: 19.5 g (29% d. Th.)

¹H-NMR (400 MHz, DMSO-d₆): δ = 7.63-7.31 (br. s, 2H), 7.41 (d, 2H), 7.09 (d, 2H), 4.49-4.38 (m, 1H), 4.15-3.99 (m, 2H), 3.78 (dd, 1H), 3.66 (dd, 1H), 2.77-2.68 (br. s, 1H), 1.37 (s, 3H), 1.32 (s, 3H).

LC-MS (Methode 9): Rₜ = 1.95 min; MS (ESIpos): m/z = 424 [M+H+CH₃CN]⁺.

### Beispiel 11A

2-Amino-4-(4-{[(4*S*)-2;2-dimethyl-1,3-dioxolan-4-yl]methoxy}phenyl)-6-mercaptopyridin-3,5-dicarbonitril

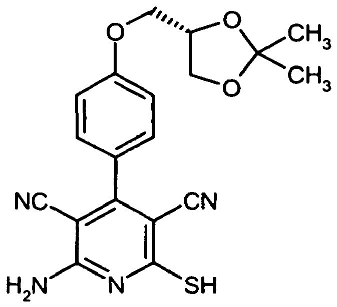

Die Titelverbindung wird ausgehend von der Verbindung aus Beispiel 9A analog zu Beispiel 10A hergestellt.

Ausbeute: 32% d. Th.

¹H-NMR (400 MHz, DMSO-d₆): δ = 7.69-7.37 (br. s, 2H), 7.42 (d, 2H), 7.10 (d, 2H), 4.48-4.39 (m, 1H), 4.15-4.02 (m, 2H), 3.78 (dd, 1H), 3.66 (dd, 1H), 2.77-2.68 (br. s, 1H), 1.37 (s, 3H), 1.31 (s, 3H).

LC-MS (Methode 2): Rₜ = 1.75 min; MS (ESIpos): m/z = 383 [M+H]⁺.

### Beispiel 12A

3-Fluor-4-(2-hydroxyethoxy)benzaldehyd 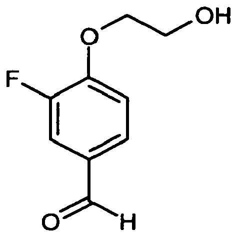 5.00 g (35.69 mmol) 3-Fluor-4-hydroxybenzaldehyd werden in 50 ml trockenem DMF gelöst. Es werden 5.35 g (42.82 mmol) 2-Bromethanol und 19.73 g (142.74 mmol) Kaliumcarbonat zugegeben. Das Reaktionsgemisch wird 10 h bei 150°C gerührt. Der Ansatz wird danach filtriert und das Filtrat am Rotationsverdampfer vom Lösungsmittel befreit. Der Rückstand wird in 30 ml Ethylacetat aufgenommen und mit 20 ml ges. wässriger Natriumhydrogencarbonat-Lösung versetzt. Die Phasen werden getrennt und die organische Phase über Magnesiumsulfat getrocknet. Das Lösungsmittel wird am Rotationsverdampfer entfernt. Das erhaltene Produkt wird ohne weitere Reinigung in der Folgereaktion eingesetzt.

Ausbeute: 4.45 g (67% d. Th.)

LC-MS (Methode 3): Rₜ = 1.39 min; MS (ESIpos): m/z = 185 [M+H]⁺.

### Beispiel 13A

2-Amino-4-[3-fluor-4-(2-hydroxyethoxy)phenyl]-6-sulfanylpyridin-3,5-dicarbonitril 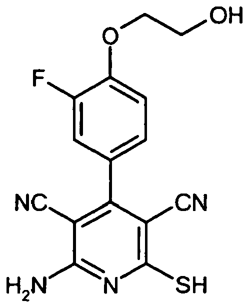 4.45 g (24.16 mmol) des Rohprodukts aus Beispiel 12A und 4.84 g (48.33 mmol) Cyanothioacetamid werden in 54 ml Ethanol vorgelegt und mit 4.89 g (48.33 mmol) 4-Methylmorpholin versetzt. Das Reaktionsgemisch wird 4 h bei +80°C gerührt. Anschließend wird 8 h bei RT nachgerührt. Das Lösungsmittel wird am Rotationsverdampfer entfernt und der Rückstand direkt mittels Säulenchromatographie an Kieselgel 60 aufgereinigt (Laufmittel-Gradient: Dichlormethan/ Ethanol 15:1 → 5:1). Das erhaltene Produkt wird ohne weitere Reinigung in den Folgereaktionen eingesetzt.

Ausbeute: 2.65 g (28% d. Th., Reinheit ca. 90%)

LC-MS (Methode 3): Rₜ = 1.62 min; MS (ESIpos): m/z = 331 [M+H]⁺.

### Beispiel 14A

4-(3,3,3-Trifluor-2-hydroxypropoxy)benzaldehyd

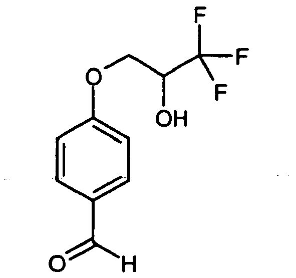

Die Titelverbindung wird ausgehend von 4-Hydroxybenzaldehyd und 3-Brom-1,1,1-trifluorpropan-2-ol analog zu Beispiel 12A hergestellt.

Ausbeute: 84% d. Th.

LC-MS (Methode 6): Rₜ = 1.58 min; MS (ESIpos): m/z = 235 [M+H]⁺.

### Beispiel 15A

2-Amino-6-sulfanyl-4-[4-(3,3,3-trifluor-2-hydroxypropoxy)phenyl]pyridin-3,5-dicarbonitril

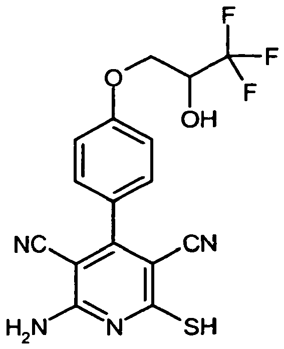

Die Titelverbindung wird ausgehend von der Verbindung aus Beispiel 14A analog zu Beispiel 10A hergestellt. Das erhaltene Produkt wird ohne weitere Reinigung in den Folgereaktionen eingesetzt.

Ausbeute: 26% d. Th. (56% Reinheit)

LC-MS (Methode 3): Rₜ = 1.98 min; MS (ESIpos): m/z = 381 [M+H]⁺.

### Beispiel 16A

4-(Chlormethyl)-2-(4-fluor-3-methylphenyl)-1,3-oxazol 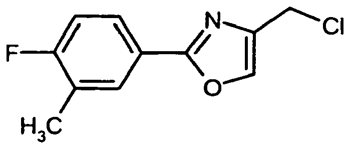 2.00 g (12:80 mmol) 4-Fluor-3-methylbenzamid und 1.79 g (14.08 mmol) 1,3-Dichloraceton werden 2 Tage lang bei 130°C gerührt. Es bildet sich eine Schmelze. Der Ansatz wird danach auf RT abgekühlt, bei dieser Temperatur vorsichtig mit 3.0 ml konz. Schwefelsäure versetzt und 15 min lang gerührt. Die erhaltene Suspension wird auf 20 ml Eiswasser gegossen und über Nacht bei RT gerührt. Der entstandene Niederschlag wird abfiltriert und über Nacht bei 40°C im Vakuumtrockenschrank getrocknet.

Ausbeute: 2.05 g (64% d. Th., 90% Reinheit)

LC-MS (Methode 7): Rₜ = 2.05 min; MS (ESIpos): m/z = 226 [M+H]⁺.

Die in Tabelle 1 aufgeführten Verbindungen werden aus den entsprechenden Ausgangsmaterialien analog zu Beispiel 16A hergestellt:

**Tabelle 1**

| **Beispiel Nr.** | **Struktur (Ausbeute)** | **LC-MS: R, [min] (Methode); MS (ESI): m/z [M+H]⁺** | **¹H-NMR (400 MHz, DMSO-d₆): δ =** |
|---|---|---|---|
| **17A** | 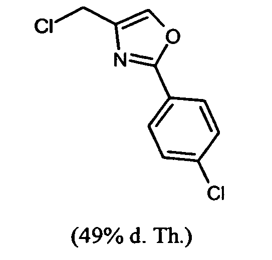 | 3.78 min (8); m/z = 228 [M]⁺ | |
| **18A** | 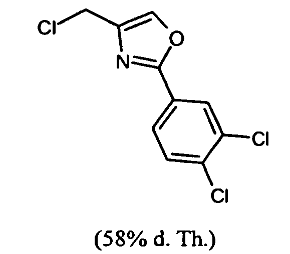 | 2.29 min (7); m/z = 262 | |
| **19A** | 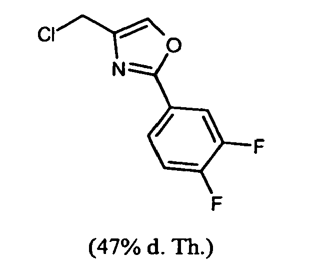 | 2.40 min (9); m/z = 230 | 8.31 (s, 1H), 7.98 (dt, 1H), 7.89-7.82 (m, 1H), 7.64 (q, 1H), 4.76 (s, 2H). |
| **20A** | 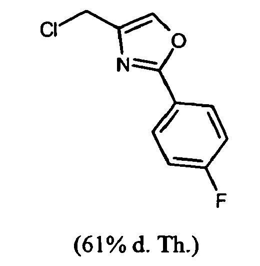 | 2.11 min (12); m/z = 212 | 8.29 (s, 1H), 8.03 (d, 1H), 8.02 (d, 1H), 7.41 (d, 1H), 7.39 (d, 1H), 4.75 (s, 2H). |
| **21A** | 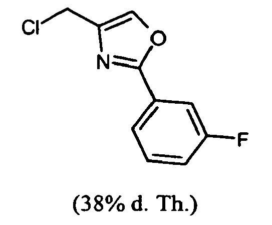 | 2.41 min (9); m/z = 212 | 8.32 (s, 1H), 7.83 (d, 1H), 7.73 (d, 1H), 7.61 (q, 1H), 7.41 (dt, 1H), 4.76 (s, 2H). |
| **22A** | 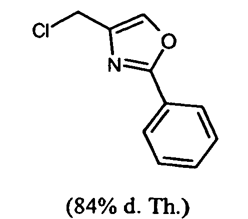 | 1.13 min (14); m/z = 194 | |

### Beispiel 23A

[2-Phenyl-5-(trifluormethyl)-1,3-oxazol-4-yl]methanol 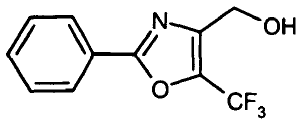 500 mg (1.94 mmol) 2-Phenyl-5-(trifluormethyl)-1,3-oxazol-4-carbonsäure werden in 40 ml trockenem THF gelöst und auf-10°C abgekühlt. Es werden 197 mg (1.94 mmol) 4-Methylmorpholin und 211 mg (1.94 mmol) Chlorameisensäureethylester zugegeben. Die Reaktionslösung wird 1 h bei -10°C gerührt. Dann werden langsam 3.9 ml (3.89 mmol) einer 1 M Lösung von Lithiumaluminiumhydrid in THF zugetropft. Das Reaktionsgemisch wird über Nacht gerührt und dabei langsam auf RT erwärmt. Danach wird erneut auf 0°C gekühlt und vorsichtig mit 0.6 ml Wasser und 1.2 ml 1 N Natronlauge versetzt. Anschließend wird über Nacht bei RT gerührt. Nach Filtration wird das Lösungsmittel am Rotationsverdampfer entfernt. Der Rückstand wird ohne weitere Reinigung in der Folgereaktion eingesetzt.

Ausbeute: 359 mg (58% d. Th., 76% Reinheit)

LC-MS (Methode 8): Rₜ = 3.34 min; MS (ESIpos): m/z = 244 [M+H]⁺.

### Beispiel 24A

4-(Chlormethyl)-2-phenyl-5-(tri fluormethyl)-1,3-oxazol 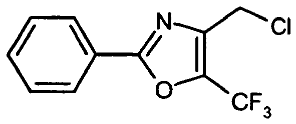 359 mg (1.137 mmol, 76% Reinheit) der Verbindung aus Beispiel 23A werden in 0.63 ml (8.64 mmol) Thionylchlorid vorgelegt. Das Reaktionsgemisch wird 48 h bei RT gerührt. Nach Einengen am Rotationsverdampfer wird der Rückstand in 10 ml Ethylacetat aufgenommen und einmal mit 5 ml ges. wässriger Natriumhydrogencarbonat-Lösung gewaschen. Die organische Phase wird über Magnesiumsulfat getrocknet. Nach Filtration wird das Lösungsmittel am Rotationsverdampfer entfernt. Der Rückstand wird mittels Säulenchromatographie an Kieselgel 60 aufgereinigt (Laufmittel-Gradient: Cyclohexan/Ethylacetat 400:1 → 60:1). Man erhält einen hellbraunen Feststoff.

Ausbeute: 148 mg (50% d. Th.)

LC-MS (Methode 7): Rₜ = 2.34 min; MS (ESIpos): m/z = 262 [M+H)⁺.

### Beispiel 25A

Ethyl 2-iod-1,3-oxazol-4-carboxylat

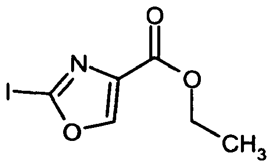

Zu einer Lösung von 8.00 g (38.43 mmol) p-Toluolsulfonsäure-Dihydrat in 48 ml Acetonitril werden 2.00 g (12.81 mmol) Ethyl 2-amino-1,3-oxazol-4-carboxylat gegeben. Die Suspension wird auf 0°C gekühlt und anschließend mit einer Lösung von 1.77 g (25.62 mmol) Natriumnitrit und 5.32 g (32.02 mmol) Kaliumiodid in 7.2 ml Wasser versetzt. Es wird 10 min bei 0°C und nach Erwärmen auf RT über Nacht weiter gerührt. Der Ansatz wird dann mit 200 ml Wasser verdünnt. Durch Zugabe von 1 M wässriger Natriumhydrogencarbonat-Lösung wird auf pH 9 gestellt. Anschließend werden 24 ml 2 M Natriumthiosulfat-Lösung zugegeben. Die wässrige Phase wird dreimal mit je 30 ml Ethylacetat extrahiert. Die vereinigten organischen Phasen werden über Magnesiumsulfat getrocknet. Nach Filtration wird das Lösungsmittel am Rotationsverdampfer entfernt. Der Rückstand wird mittels Säulenchromatographie an Kieselgel 60 aufgereinigt (Laufmittel-Gradient: Cyclohexan/Ethylacetat 300:1 → 2:1).

Ausbeute: 0.97 g (28% d. Th.)

LC-MS (Methode 6): Rₜ = 1.41 min; MS (ESIpos): m/z = 268 [M+H]⁺.

### Beispiel 26A

Ethyl 2-(4-chlor-3-methylphenyl)-1,3-oxazol-4-carboxylat 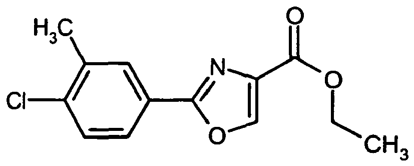 385 mg (1.44 mmol) der Verbindung aus Beispiel 25A und 319 mg (1.87 mmol) 4-Chlor-3-methylphenylboronsäure werden in 12.7 ml trockenem *N*-Methyl-2-pyrrolidon vorgelegt. Anschließend werden 105 mg (0.14 mmol) Bis(diphenylphosphino)ferrocenpalladium(Mehlorid, 0.33 ml Wasser und 940 mg (2.88 mmol) Cäsiumcarbonat zugegeben. Das Reaktionsgemisch wird 4 h bei 50°C gerührt. Der Ansatz wird danach auf RT abgekühlt und mit 20 ml Ethylacetat sowie 10 ml Wasser versetzt. Die wässrige Phase wird zweimal mit je 20 ml Ethylacetat extrahiert. Die vereinigten organischen Phasen werden einmal mit 10 ml ges. wässriger Natriumchlorid-Lösung gewaschen und über Magnesiumsulfat getrocknet. Nach Filtration wird das Lösungsmittel am Rotationsverdampfer entfernt. Der Rückstand wird mittels präparativer HPLC gereinigt (Säule: YMC GEL ODS-AQ S-5 / 15 µm; Laufmittel-Gradient: Acetonitril/Wasser 10:90 → 95:5).

Ausbeute: 162 mg (42% d. Th.)

LC-MS (Methode 7): Rₜ = 2.19 min; MS (ESIpos): m/z = 266 [M+H]⁺.

### Beispiel 27A

[2-(4-Chlor-3-methylphenyl)-1,3-oxazol-4-yl]methanol 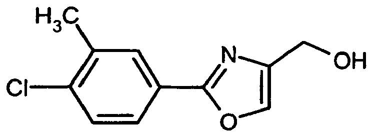 46 mg (1.22 mmol) Lithiumaluminiumhydrid werden in 8.0 ml THF vorgelegt und auf 0°C abgekühlt. Dann wird eine Lösung von 161 mg (0.61 mmol) der Verbindung aus Beispiel 26A in 2.5 ml THF zugetropft. Es wird 2 h bei RT gerührt. Die Reaktionslösung wird dann erneut auf 0°C gekühlt und mit 0.2 ml Wasser sowie 0.4 ml 1 N Natronlauge versetzt. Das Gemisch wird über Nacht bei RT gerührt. Der entstandene Niederschlag wird abfiltriert und das Filtrat am Rotationsverdampfer vom Lösungsmittel befreit. Der Rückstand wird ohne weitere Reinigung in der Folgereaktion eingesetzt.

Ausbeute: 137 mg (70% d. Th., 70% Reinheit)

LC-MS (Methode 3): Rₜ = 2.14 min; MS (ESIpos): m/z = 224 [M+H]⁺.

### Beispiel 28A

4-(Chlormethyl)-2-(4-chlor-3-methylphenyl)-1,3-oxazol 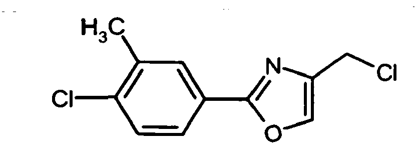 136 mg (0.61 mmol) der Verbindung aus Beispiel 27A werden in 2 ml Dichlormethan suspendiert. Die Suspension wird auf 0°C abgekühlt und langsam mit 49 µl (0.67 mmol) Thionylchlorid versetzt. Die Reaktionslösung wird über Nacht bei RT gerührt. Das Lösungsmittel wird danach am Rotationsverdampfer entfernt. Der Rückstand wird ohne weitere Reinigung in den Folgereaktionen eingesetzt.

Ausbeute: 178 mg (42% d. Th., 35% Reinheit)

LC-MS (Methode 7): Rₜ = 2.56 min; MS (ESIpos): m/z = 242 [M+H]⁺.

### Beispiel 29A

2-(4-Chlorphenyl)-5-ethyl-4-methyl-1,3-oxazol-3-oxid 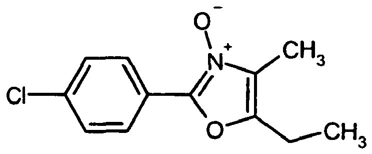 1.00 g (8.69 mmol) 2,3-Pentandion-2-oxim und 1.34 g (9.55 mmol) 4-Chlorbenzaldehyd werden in 2 ml (34.94 mmol) Eisessig vorgelegt. Dann wird 30 min lang Chlorwasserstoff-Gas unter Eiskühlung des Reaktionsgemisches eingeleitet. Anschließend wird das Reaktionsgemisch mit 10 ml Diethylether versetzt. Es fällt ein Niederschlag aus, der abgesaugt und zweimal mit je 2 ml Diethylether gewaschen wird. Der Niederschlag wird in ca. 5 ml Wasser re-suspendiert und die Suspension mit Ammoniak basisch gestellt. Es wird dann viermal mit je 10 ml Dichlormethan extrahiert. Die vereinigten organischen Phasen werden über Magnesiumsulfat getrocknet und das Lösungsmittel am Rotationsverdampfer entfernt. Der Rückstand wird ohne weitere Reinigung in der Folgereaktion eingesetzt.

Ausbeute: 1.6 g (76% d. Th.)

¹H-NMR (400 MHz, DMSO-d₆): δ = 8.42 (d, 2H), 7.63 (d, 2H), 2.76 (q, 2H), 2.10 (s, 3H), 1.24 (t, 3H).

LC-MS (Methode 6): Rₜ = 1.67 min; MS (ESIpos): m/z = 238 [M+H]⁺.

### Beispiel 30A

4-(Chlormethyl)-2-(4-chlorphenyl)-5-ethyl-1,3-oxazol 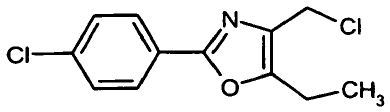 1.00 g (4.21 mmol) der Verbindung aus Beispiel 29A werden in 15 ml Chloroform gelöst und vorsichtig mit 1.4 ml (15.15 mmol) Phosphorylchlorid versetzt. Es wird zum Rückfluss erhitzt und 30 min bei dieser Temperatur gerührt. Der Ansatz wird dann auf 0°C abgekühlt und mit Ammoniak schwach basisch gestellt. Das Reaktionsgemisch wird dreimal mit je 20 ml Ethylacetat extrahiert. Die vereinigten organischen Phasen werden einmal mit 10 ml Wasser gewaschen und anschließend über Magnesiumsulfat getrocknet. Das Lösungsmittel wird am Rotationsverdampfer entfernt und der Rückstand im Vakuumtrockenschrank getrocknet. Das Produkt wird ohne weitere Reinigung in den Folgereaktionen eingesetzt.

Ausbeute: 1.2 g (84% d. Th., 74% Reinheit)

¹H-NMR (400 MHz, DMSO-d₆): δ = 7.96 (d, 2H), 7.60 (d, 2H), 4.77 (s, 2H), 2.85 (q, 2H), 1.23 (t, 3H).

LC-MS (Methode 6): Rₜ = 2.56 min; MS (ESIpos): m/z = 256 [M+H]⁺ .

### Beispiel 31A

2-(4-Chlorphenyl)-4,5-dimethyl-1,3-oxazol-3-oxid 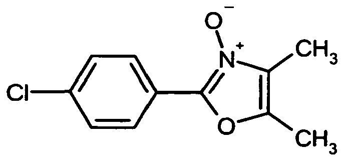 1.00 g (9.89 mmol) Diacetylmonoxim und 1.53 g (10.88 mmol) 4-Chlorbenzaldehyd werden in 2 ml (34.94 mmol) Eisessig vorgelegt. Dann wird 30 min lang Chlorwasserstoff-Gas unter Eiskühlung des Reaktionsgemisches eingeleitet. Anschließend wird das Reaktionsgemisch mit 10 ml Diethylether versetzt. Es fällt ein Niederschlag aus, der abgesaugt und zweimal mit je 2 ml Diethylether gewaschen wird. Der Niederschlag wird in ca. 5 ml Wasser re-suspendiert und die Suspension mit Ammoniak basisch gestellt. Es wird dann viermal mit je 10 ml Dichlormethan extrahiert. Die vereinigten organischen Phasen werden über Magnesiumsulfat getrocknet und das Lösungsmittel am Rotationsverdampfer entfernt. Der Rückstand wird ohne weitere Reinigung in der Folgereaktion eingesetzt.

Ausbeute: 1.85 g (84% d. Th.)

LC-MS (Methode 5): Rₜ = 2.29 min; MS (ESIpos): m/z = 224 [M+H]⁺.

Die in Tabelle 2 aufgeführten Verbindungen werden aus den entsprechenden Ausgangsmaterialien analog zu Beispiel 31 A hergestellt:

**Tabelle 2**

| **Beispiel Nr.** | **Struktur (Ausbeute)** | **LC-MS: Rₜ [min] (Methode); MS (ESI): M/z [M+H]⁺** |
|---|---|---|
| **32A** | 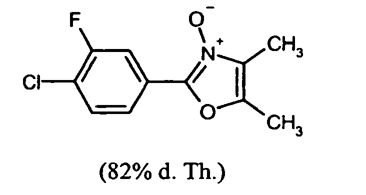 | 1.99 min (3); m/z = 242 |
| **33A** | 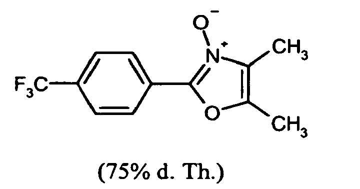 | 1.82 min (15); m/z = 258 |

### Beispiel 34A

4-(Chlormethyl)-2-(4-chlorphenyl)-5-methyl-1,3-oxazol 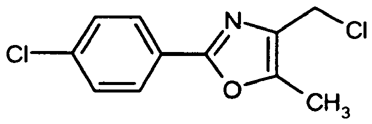 1.00 g (4.47 mmol) der Verbindung aus Beispiel 31A werden in 15 ml Chloroform vorgelegt und vorsichtig mit 1.5 ml (16.10 mmol) Phosphorylchlorid versetzt. Das Reaktionsgemisch wird 30 min unter Rühren zum Rückfluss erhitzt. Der Ansatz wird anschließend auf 0°C abgekühlt und durch Zugabe von Ammoniak schwach basisch gestellt. Das Gemisch wird dreimal mit je 20 ml Ethylacetat extrahiert. Die vereinigten organischen Phasen werden zweimal mit je 5 ml Wasser gewaschen und anschließend über Magnesiumsulfat getrocknet. Das Lösungsmittel wird am Rotationsverdampfer entfernt. Der Rückstand wird ohne weitere Reinigung in den Folgestufen eingesetzt.

Ausbeute: 1.33 g (96% d. Th., 78% Reinheit)

¹H-NMR (400 MHz, DMSC)-d₆): δ = 7.95 (d, 2H), 7.60 (d, 2H), 4.77 (s, 2H), 2.44 (s, 3H).

LC-MS (Methode 3): Rₜ = 2.80 min; MS (ESIpos): m/z = 242 [M+H]⁺.

Die in Tabelle 3 aufgeführten Beispiele werden aus den entsprechenden Ausgangsverbindungen analog zu Beispiel 34A hergestellt:

**Tabelle 3**

| **Beispiel Nr.** | **Struktur (Ausbeute)** | **LC-MS: Rₜ [min] (Methode); MS (ESI): M/z [M+H]⁺** |
|---|---|---|
| **35A** | 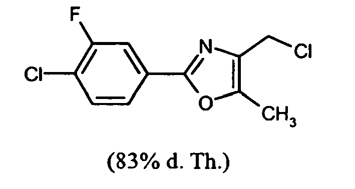 | 2.27 min (7); m/z = 260 |
| **36A** | 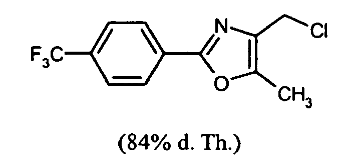 | 1.37 min (14); m/z = 276 |

### Beispiel 37A

Methyl 5-(4-chlorphenyl)-1,3-oxazol-4-carboxylat 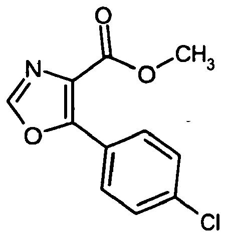 1.40 g (8.00 mmol) 4-Chlorbenzoesäurechlorid, 1.00 g (10.09 mmol) Isocyanatoessigsäuremethylester und 5.9 ml (42.39 mmol) Triethylamin werden in 15 ml trockenem THF gelöst und 48 h bei RT gerührt. Anschließend wird das Lösungsmittel am Rotationsverdampfer entfernt. Der Rückstand wird in 20 ml Ethylacetat aufgenommen und einmal mit 5 ml Wasser gewaschen. Die organische Phase wird über Magnesiumsulfat getrocknet und das Lösungsmittel am Rotationsverdampfer entfernt. Der Rückstand wird in 10 ml Cyclohexan aufgeschlämmt und abfiltriert. Anschließend wird aus ca. 10 ml Methanol umkristallisiert. Man erhält nadelförmige Kristalle, die im Trockenschrank bei 50°C getrocknet werden. Durch Nachfällung erhält man nach Umkristallisation eine weitere Produktfraktion.

Ausbeute: 0.85 g (45% d. Th.)

¹H-NMR (400 MHz, DMSO-d₆): δ = 8.61 (s, 1H), 8.02 (d, 2H), 7.62 (d, 2H), 3.82 (s, 3H).

LC-MS (Methode 14): Rₜ = 1.09 min; MS (ESIpos): m/z = 238 [M+H]⁺.

### Beispiel 38A

[5-(4-Chlorphenyl)-1,3-oxazol-4-yl]methanol 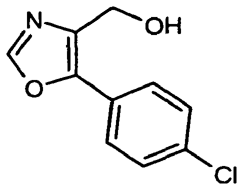 166 mg (4.38 mmol) Lithiumaluminiumhydrid werden in 10 ml trockenem THF vorgelegt und auf 0°C abgekühlt. Es wird eine Lösung von 260 mg (1.09 mmol) der Verbindung aus Beispiel 37A in 10 ml trockenem THF zugetropft. Nach beendeter Zugabe wird die Reaktionslösung langsam auf RT erwärmt und 1 h bei dieser Temperatur nachgerührt. Anschließend wird für 2 h unter Rühren zum Rückfluss erhitzt. Danach wird wieder auf 0°C gekühlt, vorsichtig 0.4 ml Wasser sowie 0.8 ml 1 N Natronlauge zugegeben und 3 h bei RT gerührt. Der entstandene Niederschlag wird abfiltriert und das Filtrat am Rotationsverdampfer vom Lösungsmittel befreit. Der Rückstand wird ohne weitere Reinigung in der Folgereaktion eingesetzt.

Ausbeute: 219 mg (82%-d. Th., 86% Reinheit)

LC-MS (Methode 15): Rₜ = 1.74 min; MS (ESIpos): m/z = 210 [M+H]⁺.

### Beispiel 39A

4-(Chlormethyl)-5-(4-chlorphenyl)-1,3-oxazol 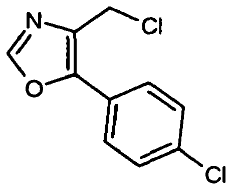 269 mg (0.77 mmol) der Verbindung aus Beispiel 38A werden in 0.43 ml (5.85 mmol) Thionylchlorid vorgelegt. Das Reaktionsgemisch wird 12 h bei RT gerührt und überschüssiges Thionylchlorid anschließend im Vakuum entfernt. Der Rückstand wird in 5 ml Ethylacetat aufgenommen und einmal mit 2 ml ges. wässriger Natriumhydrogencarbonat-Lösung gewaschen. Die organische Phase wird über Magnesiumsulfat getrocknet und das Lösungsmittel am Rotationsverdampfer entfernt. Das erhaltene Produkt wird ohne weitere Reinigung in den Folgereaktionen eingesetzt.

Ausbeute: 145 mg (62% d. Th., 76% Reinheit)

LC-MS (Methode 14): Rₜ = 1.21 min; MS (ESIpos): m/z = 228 [M+H]⁺.

### Beispiel 40A

2-(4-Chlorphenyl)-4-[(methoxymethoxy)methyl]-1,3-oxazol 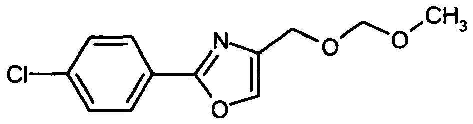 1.32 g (7.54 mmol) [2-(4-Chlorphenyl)-1,3-oxazol-4-yl]methanol (Beispiel 100A) werden in 18.5 ml trockenem THF vorgelegt, auf 0°C abgekühlt und mit 0.33 g (8.29 mmol) Natriumhydrid (60%-ig in Mineralöl) versetzt. Es wird 10 min bei 0°C und anschließend 1 h bei RT nachgerührt. Das Reaktionsgemisch wird wieder auf 0°C gekühlt und tropfenweise mit 0.69 ml (9.04 mmol) Chlordimethylether versetzt. Es wird 10 min bei 0°C und anschließend 2 h bei RT gerührt. Der Reaktionsansatz wird dann mit 5 ml Wasser versetzt und dreimal mit je 25 ml Ethylacetat extrahiert. Die vereinigten organischen Phasen werden über Magnesiumsulfat getrocknet und das Lösungsmittel am Rotationsverdampfer entfernt. Das erhaltene Produkt wird ohne weitere Reinigung in der Folgereaktion eingesetzt.

Ausbeute: 1.70 g (87% d. Th., 85% Reinheit)

LC-MS (Methode 7): Rₜ = 1.58 min; MS (ESIpos): m/z = 220 [M+H]⁺.

### Beispiel 41A

2-(4-Chlorphenyl)-4-[(methoxymethoxy)methyl]-1,3-oxazol-5-carbaldehyd 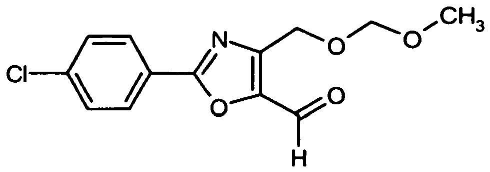 200 mg (0.91 mmol) des Rohprodukts aus Beispiel 40A werden in 3.5 ml trockenem Diethylether vorgelegt und auf -78°C abgekühlt. Es werden langsam 0.63 ml (1.00 mmol) einer 1.6 M Lösung von n-Butyllithium in Hexan zugetropft. Der Reaktionsansatz wird 1 h bei -78°C gerührt. Anschließend werden langsam 0.21 ml (2.74 mmol) *N,N*-Dimethylformamid zugetropft. Man lässt den Ansatz auf RT erwärmen und rührt noch 1 h bei RT nach. Danach wird das Gemisch auf ca. 3 ml Wasser gegossen. Es wird dreimal mit je 10 ml Diethylether extrahiert. Die vereinigten organischen Phasen werden über Magnesiumsulfat getrocknet und das Lösungsmittel am Rotationsverdampfer entfernt. Der Rückstand wird mittels Säulenchromatographie an Kieselgel 60 aufgereinigt (Laufmittel-Gradient: Cyclohexan/Ethylacetat 20:1 → 2:1).

Ausbeute: 161 mg (56% d. Th., 79% Reinheit)

LC-MS (Methode 6): Rₜ = 1.74 min; MS (ESIpos): m/z = 248 [M+H]⁺.

### Beispiel 42A

2-(4-Chlorphenyl)-4-[(methoxymethoxy)methyl]-1,3-oxazol-5-carbonsäure 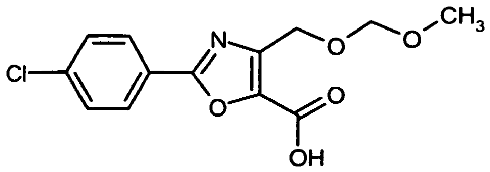 556 mg (2.25 mmol) der Verbindung aus Beispiel 41 A und 14.7 ml Dioxan werden zu einer Suspension von 802 mg (4.72 mmol) Silber(I)nitrat in 2 ml Wasser gegeben. Dann wird langsam eine Lösung von 193 mg (4.84 mmol) Natriumhydroxid in 7.8 ml Wasser zugegeben. Die Mischung wird 3 h bei RT gerührt. Der Ansatz wird anschließend über Celite filtriert, das mit warmen Wasser nachgewaschen wird. Das erhaltene Filtrat wird durch Zugabe von 1 N Salzsäure sauer gestellt und dreimal mit je 20 ml Diethylether extrahiert. Die vereinigten organischen Phasen werden über Magnesiumsulfat getrocknet und das Lösungsmittel am Rotationsverdampfer entfernt. Die Reinigung des Rückstands erfolgt mittels präparativer HPLC (Säule: YMC GEL ODS-AQ S-5 / 15 µm; Laufnüttel-Gradient: Acetonitril/Wasser 10:90 → 95:5).

Ausbeute: 138 mg (21 % d. Th., 92% Reinheit)

LC-MS (Methode 3): Rₜ = 1.93 min; MS (ESIpos): m/z = 286 [M+Na]⁺.

### Beispiel 43A

Methyl 2-(4-chl orphenyl)-4-[(methoxymethoxy)methyl]-1,3-oxazol-5-carboxylat 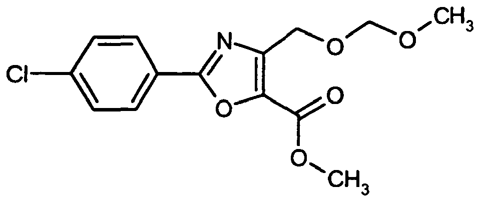 138 mg (0.52 mmol) der Verbindung aus Beispiel 42A werden in 3 ml Toluol und 2.5 ml Methanol gelöst. Anschließend werden 0.4 ml (0.79 mmol) einer 2 M Lösung von Trimethylsilyldiazomethan in Hexan zugetropft. Es wird 10 min bei RT gerührt und dann das Lösungsmittel am Rotationsverdampfer entfernt. Der Rückstand wird ohne weitere Reinigung in der Folgereaktion eingesetzt.

Ausbeute: 144 mg (90% d. Th., 91% Reinheit)

LC-MS (Methode 15): Rₜ = 1.98 min; MS (ESIpos): m/z = 300 [M+Na]⁺.

### Beispiel 44A

Methyl 2-(4-chlorphenyl)-4-(hydroxymethyl)-1,3-oxazol-5-carboxylat 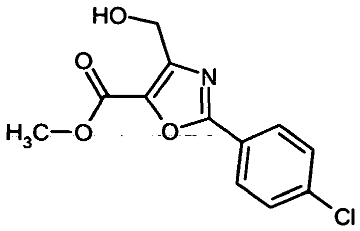 144 mg (0.52 mmol) der Verbindung aus Beispiel 43A werden in 0.5 ml Methanol vorgelegt und mit 0.13 ml 4 N Salzsäure versetzt. Anschließend werden noch 3 Tropfen konz. Salzsäure zugegeben. Das Reaktionsgemisch wird 8 h bei RT gerührt. Der Ansatz wird dann mit ca. 5 ml Wasser verdünnt und dreimal mit je 10 ml Diethylether extrahiert. Die vereinigten organischen Phasen werden über Magnesiumsulfat getrocknet und das Lösungsmittel am Rotationsverdampfer entfernt. Das erhaltene Produkt wird ohne weitere Reinigung in der Folgereaktion eingesetzt.

Ausbeute: 100 mg (81% d. Th., 84% Reinheit)

LC-MS (Methode 7): Rₜ = 1.29 min; MS (ESIpos): m/z = 234 [M+H]⁺.

### Beispiel 45A

Methyl 4-(chlormethyl)-2-(4-chlorphenyl)-1,3-oxazol-5-carboxylat 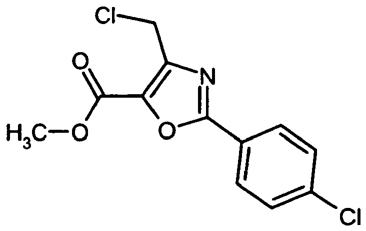 100 mg (0.43 mmol) der Verbindung aus Beispiel 44A werden zusammen mit 0.24 ml (3.24 mmol) Thionylchlorid 8 h bei RT gerührt. Das überschüssige Thionylchlorid wird im Vakuum entfernt und der Rückstand in ca. 5 ml Ethylacetat aufgenommen. Es wird einmal mit 2 ml ges. wässriger Natriumhydrogencarbonat-Lösung gewaschen. Die organische Phase wird über Magnesiumsulfat getrocknet und das Lösungsmittel am Rotationsverdampfer entfernt. Das erhaltene Produkt wird ohne weitere Reinigung in den Folgereaktionen eingesetzt.

Ausbeute: 99 mg (92% d. Th., 98% Reinheit)

LC-MS (Methode 7): Rₜ = 1.96 min; MS (ESIpos): m/z = 252 [M+H]⁺.

### Beispiele 46A

2-Amino-6-({[2-(4-chlorphenyl)-1,3-oxazol-4-yl]methyl}sulfanyl)-4-(4-{[(4R)-2,2-dimethyl-1,3-dioxolan-4-yl]methoxy}phenyl)pyridin-3,5-dicarbonitril 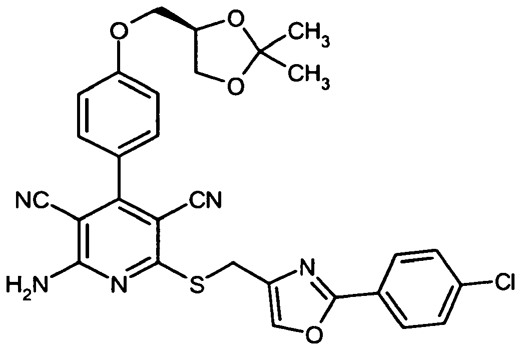 70 mg (0.18 mol) der Verbindung aus Beispiel 10A und 46 mg (0.20 mmol) der Verbindung aus Beispiel 17A werden zusammen mit 46 mg (0.55 mmol) Natriumhydrogencarbonat in 1.9 ml trockenem DMF suspendiert. Das Reaktionsgemisch wird 20 h bei RT gerührt. Der Ansatz wird danach am Rotationsverdampfer vom Lösungsmittel befreit und der Rückstand mittels präparativer HPLC gereinigt (Säule: YMC GEL ODS-AQ S-5 / 15 µm; Laufmittel-Gradient: Acetonitril/Wasser 10:90 → 95:5).

Ausbeute: 79 mg (75% d. Th.)

¹H-NMR (400 MHz, DMSO-d₆): δ = 8.37 (s, 1H), 8.30-8.01 (br. s, 2H), 7.97 (d, 2H), 7.60 (d, 2H), 7.48 (d, 2H), 7.12 (d, 2H), 4.48-4.40 (m, 1H), 4.42 (s, 2H), 4.16-4.03 (m, 3H), 3.78 (dd, 1H), 1.37 (s, 3H), 1.31 (s, 3H).

LC-MS (Methode 3): Rₜ = 2.99 min; MS (ESIpos): m/z = 574 [M+H]⁺.

### Beispiel 47A

2-Amino-6-({[2-(4-chlorphenyl)-1,3-oxazol-4-yl]methyl}sulfanyl)-4-(4-{[(4*S*)-2,2-dimethyl-1,3-dioxolan-4-yl]methoxy}phenyl)pyridin-3,5-dicarbonitril 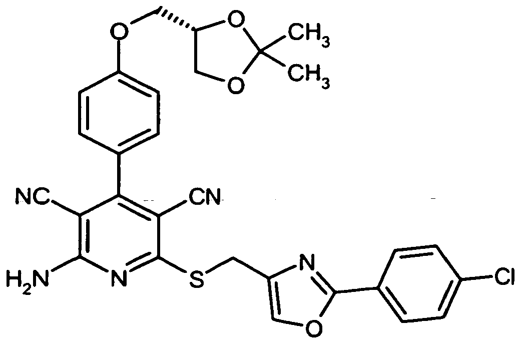 150 mg (0.39 mmol) der Verbindung aus Beispiel 11 A und 98 mg (0.43 mmol) der Verbindung aus Beispiel 17A werden zusammen mit 99 mg (1.18 mmol) Natriumhydrogencarbonat in 2 ml trockenem DMF suspendiert. Das Reaktionsgemisch wird 20 h bei RT gerührt. Der Ansatz wird danach direkt mittels präparativer HPLC aufgereinigt (Säule: YMC GEL ODS-AQ S-5 / 15 µm; Laufmittel-Gradient: Acetonitril/Wasser 10:90 → 95:5).

Ausbeute: 147 mg (65% d. Th.)

¹H-NMR. (400 MHz, DMSO-d₆): δ = 8.37 (s, 1H), 8.29-7.91 (br. s, 2H), 7.97 (d, 2H), 7.61 (d, 2H), 7.47 (d, 2H), 7.12 (d, 2H), 4.48-4.39 (m, 1H), 4.42 (s, 2H), 4.16-4.03 (m, 3H), 3.77 (dd, 1H), 1.37 (s, 3H), 1.31 (s, 3H).

LC-MS (Methode 4): Rₜ = 4.23 min; MS (ESIpos): m/z = 574 [M+H]⁺.

Die in Tabelle 4 aufgeführten Beispiele werden aus den entsprechenden Ausgangsverbindungen analog zu den Beispielen 46A und 47A hergestellt:

**Tabelle 4**

| **Beispiel Nr.** | **Struktur (Ausbeute)** | **LC-MS: Rₜ [min] (Methode); MS (ESn: m/z [M+H]⁺** | ¹H-NMR (DMSO-d₆): δ = |
|---|---|---|---|
| **48A** | 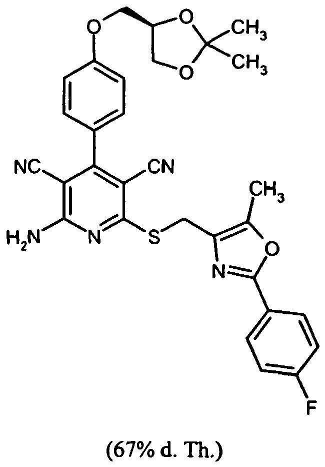 | 2.98 min (3); m/z = 572 | |
| **49A** | 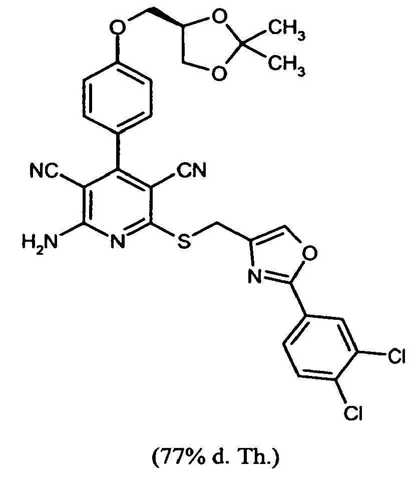 | 2.80 min (6); m/z = 608 | 8.41 (s, 1H), 8.36-7.98 (br. s, 2H), 8.12 (d, 1H), 7.92 (dd, 1H), 7.71 (d, 1H), 7.48 (d, 2H), 7.11 (d, 2H), 4.48-4.39 (m, 1H), 4.42 (s, 2H), 4.15-4.03 (m, 3H), 3.79 (dd, 1H), 1.38 (s, 3H), 1.31 (s, 3H). |
| **50A** | 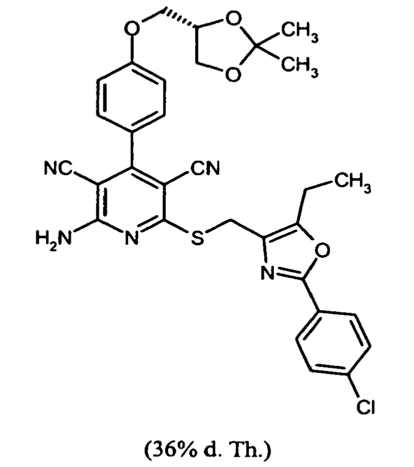 | 2.63 min (7); m/z = 602 | 8.21-7.89 (br. s, 2H), 7.93 (d, 2H), 7.59 (d, 2H), 7.50 (d, 2H), 7.12 (d, 2H), 4.52 (s, 2H), 4.47-4.40 (m, 1H). 4.15-4.03 (m, 3H), 3.78 (dd, 1H), 2.89 (q, 2H), 1.37 (s, 3H), 1.32 (s, 3H), 1.20 (t, 3H). |
| **51A** | 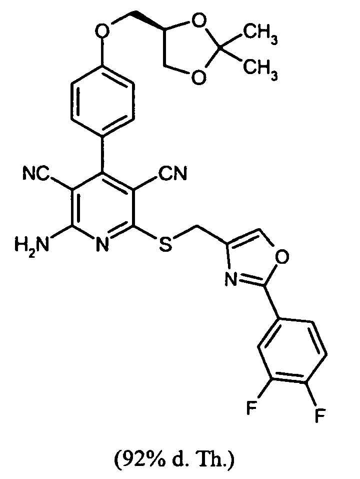 | 2.38 min (7); m/z = 576 | |
| **52A** | 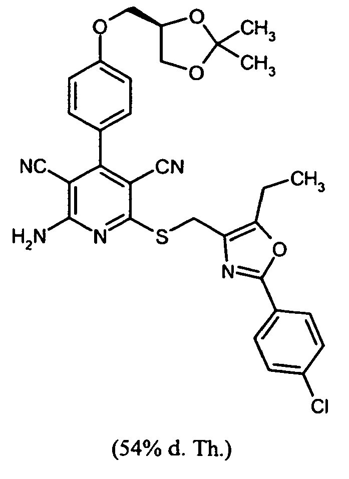 | 3.22 min (3); m/z = 602 | 8.21-7.88 (br. s, 2H), 7.94 (d, 2H), 7.59 (d, 2H), 7.49 (d, 2H), 7.22 (d, 2H), 4.51 (s, 2H), 4.48-4.39 (m, 1H), 4.18-4.04 (m, 3H), 3.79 (t, 1H), 2.88 (q, 2H), 1.38 (s, 3H), 1.32 (s, 3H), 1.21 (t, 3H). |
| **53A** | 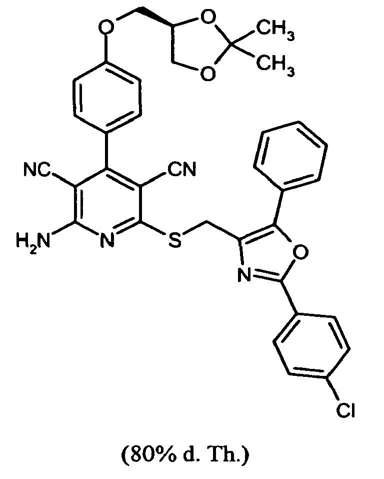 | 3.40 min (3); m/z = 650 | 8.15-8.07 (m, 2H), 7.96-7.88 (m, 2H), 7.84 (d, 2H), 7.68-7.61 (m, 2H), 7.61-7.53 (m, 2H), 7.52-7.44 (m, 3H), 7.12 (d, 2H), 4.81 (s, 2H), 4.48-4.41 (m, 1H), 4.16-4.03 (m, 3H), 3.79 (dd, 1H), 1.38 (s, 3H), 1.32 (s, 3H). |
| **54A** | 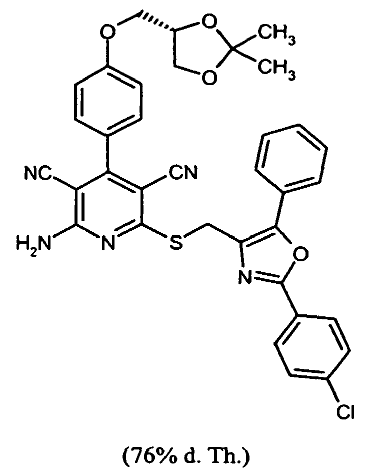 | 3.40 min (3); m/z = 650 | |
| **55A** | 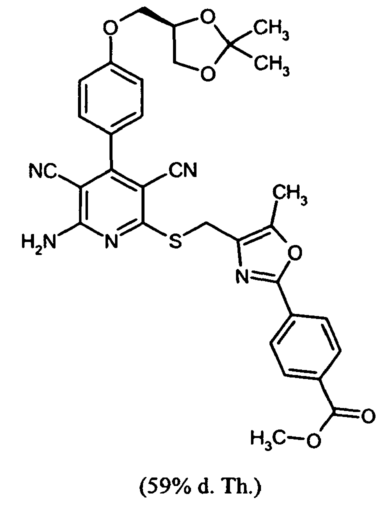 | 3.96 min (5); m/z = 612 | 8.33-8.18 (m, 6H), 7.66 (d, 2H), 7.30 (d, 2H), 4.71 (s, 2H), 4.67-4.58 (m, 1H), 4.36-4.22 (m, 3H), 4.08 (s, 3H), 4.00-3.92 (m, 1H), 2.68 (s, 3H), 1.57 (s, 3H), 1.50 (s, 3H). |
| **56A** | 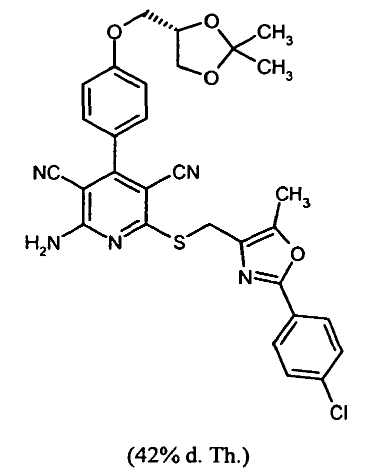 | 2.54 min (7); m/z = 588 | |
| **57A** | 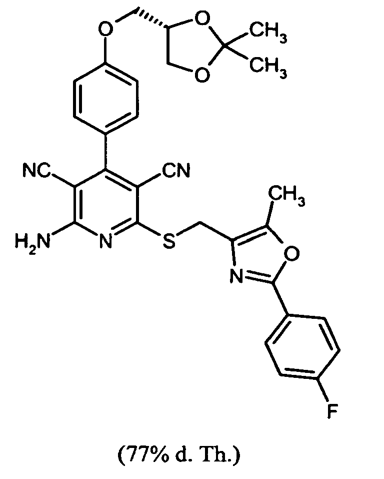 | 2.58 min (6); m/z = 572 | 8.14-7.92 (br. s, 2H), 7.96 (d, 1H), 7.95 (d, 1H), 7.49 (d, 2H), 7.36 (t, 2H), 7.12 (d, 2H), 4.51 (s, 2H), 4.47-4.41 (m, 1H), 4.14-4.03 (m, 1H), 3.79 (dd, 1H), 2.47 (s, 3H), 1.38 (s, 3H), 1.32 (s, 3H). |
| **58A** | 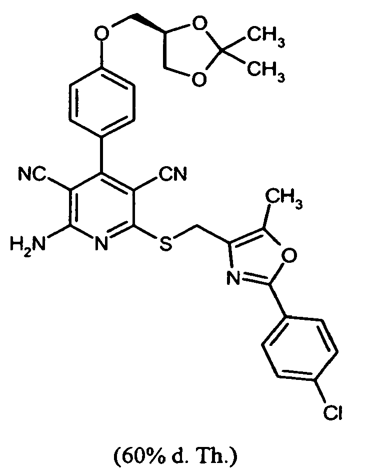 | 2.54 min (7); m/z = 588 | 8.19-7.97 (br. s, 2H), 7.94 (d, 2H), 7.58 (d, 2H), 7.49 (d, 2H), 7.12 (d, 2H), 4.51 (s, 2H), 4.48-4.41 (m, 1H), 4.16-4.03 (m, 3H), 3.79 (dd, 2H), 2.46 (s, 3H), 1.37 (s, 3H), 1.32 (s, 3H). |
| **59A** | 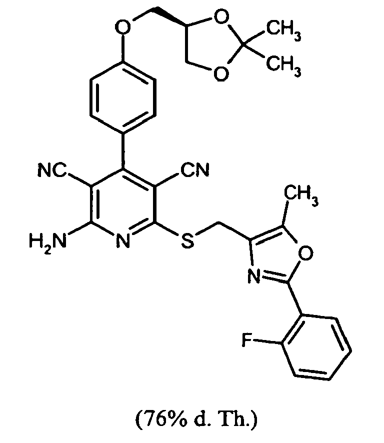 | 2.34 min (7); m/z = 572 | 8.19-7.95 (br. s, 2H), 7.96 (dt, 1H), 7.60-7.52 (m, 1H), 7.49 (d, 2H), 7.42-7.32 (m, 2H), 7.12 (d, 2H), 4.53 (s, 2H), 4.48-4.41 (m, 1H), 4.17-4.04 (m, 3H), 3.78 (dd, 1H), 2.48 (s, 3H), 1.38 (s, 3H), 1.31 (s, 3H). |
| **60A** | 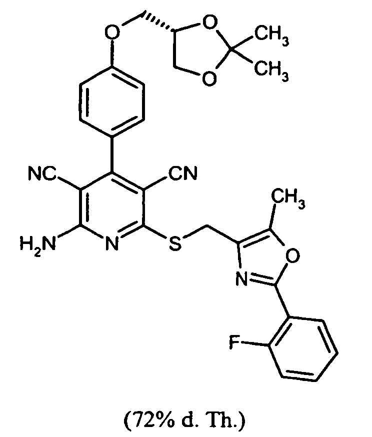 | 2.34 min (7); m/z = 572 | 8.21-7.96 (br. s, 2H), 7.96 (dt, 1H), 7.60-7.53 (m, 1H), 7.49 (d, 2H), 7.42-7.32 (m, 2H), 7.12 (d, 2H), 4.53 (s, 2H), 4.47-4.41 (m, 1H), 4.16-4.04 (m, 3H), 3.79 (dd, 1H), 2.48 (s, 3H), 1.38 (s, 3H), 1.31 (s, 3H). |
| **61A** | 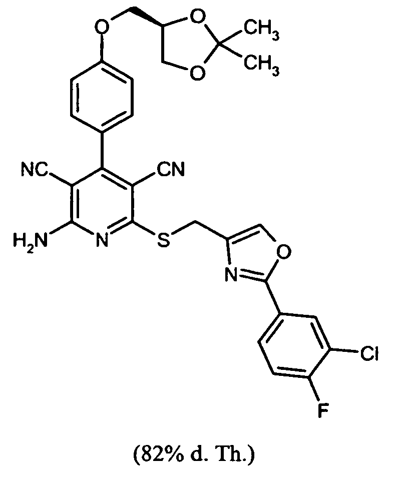 | 2.48 min (7); m/z = 592 | 8.49 (s, 1H), 8.26-7.92 (br. s, 2H), 8.10 (dd, 1H), 8.00-7.94 (m, 1H), 7.60 (pseudo-t, 1H), 7.47 (d, 2H), 7.11 (d, 2H), 4.48-4.39 (m, 1H), 4.42 (s, 2H), 4.15-4.03 (m, 3H), 3.78 (dd, 1H), 1.38 (s, 3H), 1.32 (s, 3H). |
| **62A** | 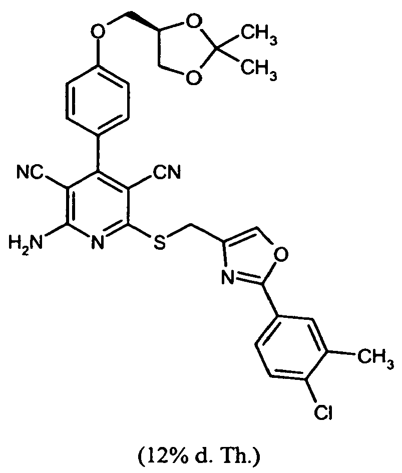 | 3.19 min (3); m/z = 588 | |
| **63A** | 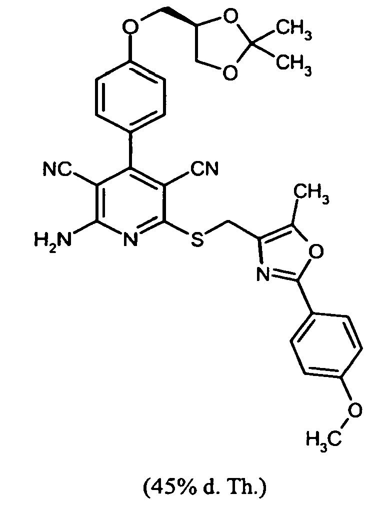 | 2.35 min (7); m/z = 584 | 8.19-7.95 (br. s, 2H), 7.86 (d, 2H), 7.49 (d, 2H), 7.13 (d, 2H), 7.06 (d, 2H), 4.48 (s, 2H), 4.47-4.41 (m, 1H), 4.16-4.03 (m, 3H), 3.81 (s, 3H), 3.81-3.75 (m, 1H), 2.43 (s, 3H), 1.38 (s, 3H), 1.31 (s, 3H). |
| **64A** | 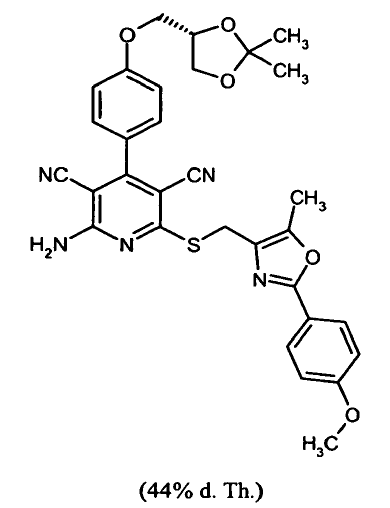 | 2.94 min (3); m/z = 584 | 8.19-7.93 (br. s, 2H), 7.85 (d, 2H), 7.49 (d, 2H), 7.13 (d, 2H), 7.07 (d, 2H), 4.49 (s, 2H), 4.47-4.41 (m, 1H), 4.16-4.03 (m, 3H), 3.81 (s, 3H), 3.81-3.74 (m, 1H), 2.45 (s, 3H), 1.38 (s, 3H), 1.31 (s, 3H). |
| **65A** | 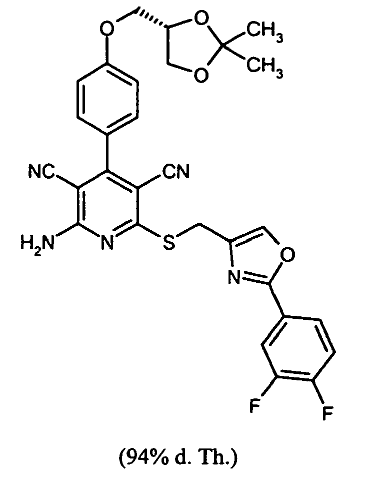 | 2.99 min (3); m/z = 576 | |
| **66A** | 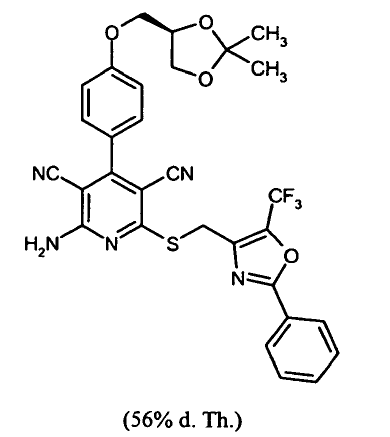 | 3.15 min (3); m/z = 608 | 8.06-7.93 (br. s, 2H), 8.05 (d, 2H), 7.68-7.57 (m, 3H), 7.51 (d, 2H), 7.14 (d, 2H), 4.71 (s, 2H), 4.48-4.41 (m, 1H), 4.18-4.04 (m, 3H), 3.79 (dd, 1H), 1.37 (s, 3H), 1.31 (s, 3H). |
| **67A** | 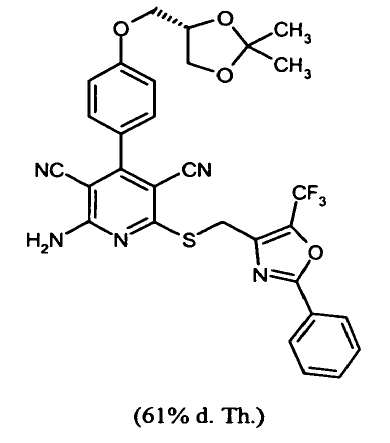 | 3.15 min (3); m/z = 608 | 8.07-7.95 (br. s, 2H), 8.05 (d, 2H), 7.68-7.57 (m, 3H), 7.51 (d, 2H), 7.14 (d, 2H), 4.71 (s, 2H), 4.48-4.41 (m, 1H), 4.17-4.05 (m, 3H), 3.79 (dd, 1H), 1.37 (s, 3H), 1.32 (s, 3H). |
| **68A** | 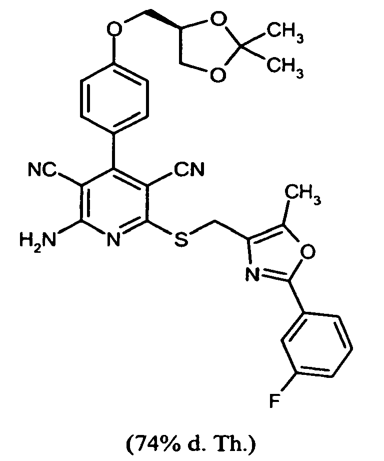 | 3.01 min (3); m/z = 572 | 8.17-7.96 (br. s, 2H), 7.77 (d, 1H), 7.66 (d, 1H), 7.57 (q, 1H), 7.49 (d, 2H), 7.36 (dt, 1H), 7.12 (d, 2H), 4.52 (s, 2H), 4.48-4.41 (m, 1H), 4.15-4.03 (m, 3H), 3.78 (dd, 1H), 2.47 (s, 3H), 1.37 (s, 3H), 1.31 (s, 3H). |
| **69A** | 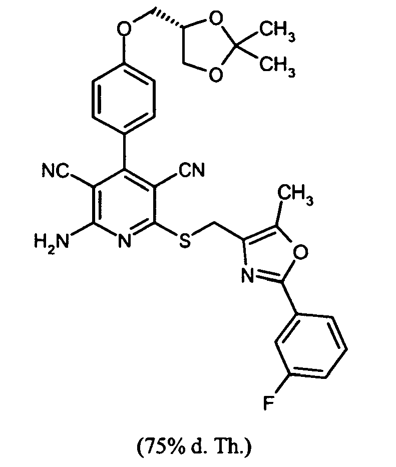 | 3.01 min (3); m/z = 572 | 8.16-7.97 (br. s, 2H), 7.78 (d, 1H), 7.66 (d, 1H), 7.58 (q, 1H), 7.49 (d, 2H), 7.37 (dt, 1H), 7.12 (d, 2H), 4.52 (s, 2H), 4.47-4.41 (m, 1H), 4.15-4.03 (m, 3H), 3.78 (dd, 1H), 2.48 (s, 3H), 1.37 (s, 3H), 1.31 (s, 3H). |
| **70A** | 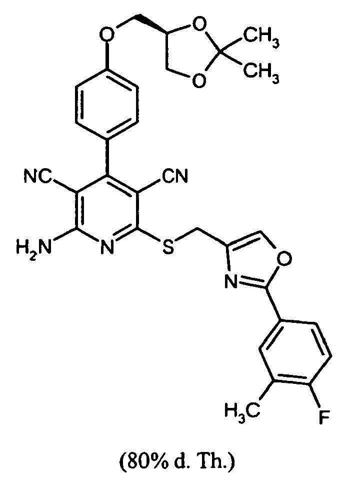 | 2.62 min (6); m/z = 572 | 8.33 (s, 1H), 8.25-7.97 (br. s, 2H), 7.92 (dd, 1H), 7.84-7.79 (m, 1H), 7.48 (d, 2H), 7.30 (t, 1H), 7.11 (d, 2H), 4.48-4.39 (m, in), 4.42 (s, 2H), 4.16-4.04 (m, 3H), 3.78 (dd, 1H), 2.31 (s, 3H), 1.38 (s, 3H), 1.32 (s, 3H). |
| **71A** | 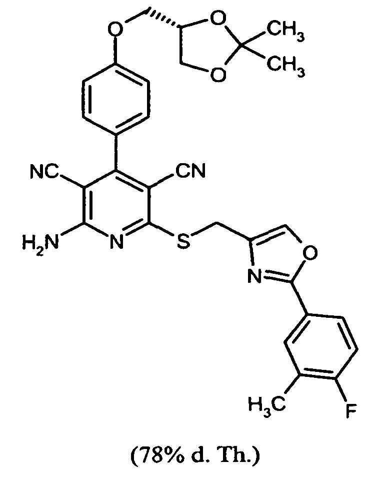 | 2.62 min (6); m/z = 572 | 8.32 (s, 1H), 8.25-7.97 (br. s, 2H), 7.92 (dd, 1H), 7.85-7.80 (m, 1H), 7.48 (d, 2H), 7.30 (t, 1H), 7.12 (d, 2H), 4.48-4.39 (m, 1H), 4.41 (s, 2H), 4.16-4.04 (m, 3H), 3.79 (dd, 1H), 2.31 (s, 3H), 1.38 (s, 3H), 1.31 (s, 3H). |
| **72A** | 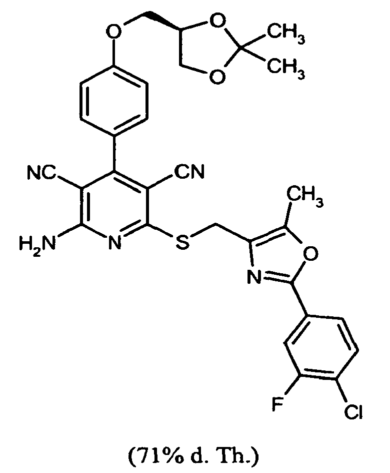 | 3.21 min (3); m/z = 606 | 8.17-7.96 (br. s, 2H), 7.85 (dd, 1H), 7.75 (s, 1H), 7.74 (q, 1H), 7.49 (d, 2H), 7.12 (d, 2H), 4.52 (s, 2H), 4.47-4.40 (m, 1H), 4.15-4.04 (m, 3H), 3.78 (dd, 1H), 2.48 (s, 3H), 1.36 (s, 3H), 1.31 (s, 3H). |
| **73A** | 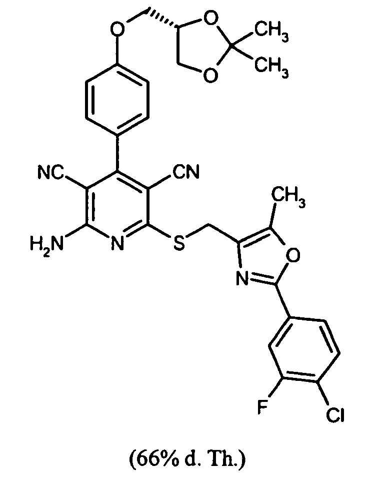 | 3.21 min (3); m/z = 606 | 8.19-7.95 (br. s, 2H), 7.85 (dd, 1H), 7.75 (s, 1H), 7.74 (q, 1H), 7.49 (d, 2H), 7.12 (d, 2H), 4.52 (s, 2H), 4.48-4.41 (m, 1H), 4.15-4.04 (m, 3H), 3.78 (dd, 1H), 2.48 (s, 3H), 1.37 (s, 3H), 1.32 (s, 3H). |
| **74A** | 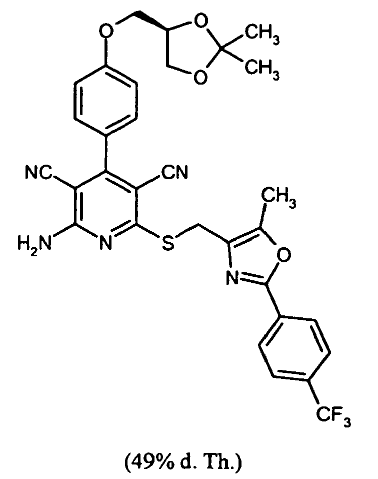 | 3.10 min (3); m/z = 622 | 8.17-7.98 (br. s, 2H), 8.12 (d, 2H), 7.88 (d, 2H), 7.50 (d, 2H), 7.12 (d, 2H), 4.54 (s, 2H), 4.48-4.41 (m, 1H), 4.16-4.05 (m, 3H), 3.79 (dd, 1H), 2.51 (s, 3H), 1.38 (s, 3H), 1.32 (s, 3H). |
| **75A** | 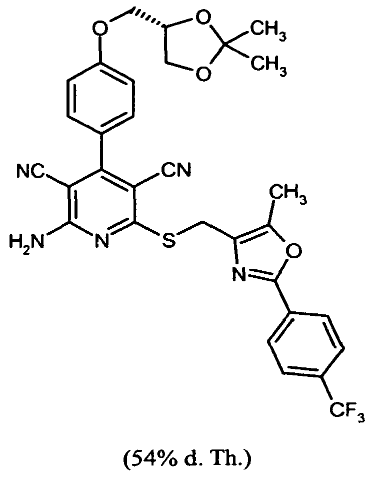 | 2.56 min (7); m/z = 622 | 8.17-7.97 (br. s, 2H), 8.12 (d, 2H), 7.88 (d, 2H), 7.49 (d, 2H), 7.12 (d, 2H), 4.54 (s, 2H), 4.48-4.41 (m, 1H), 4.16-4.04 (m, 3H), 3.79 (dd, 1H), 2.50 (s, 3H), 1.38 (s, 3H), 1.32 (s, 3H). |
| **76A** | 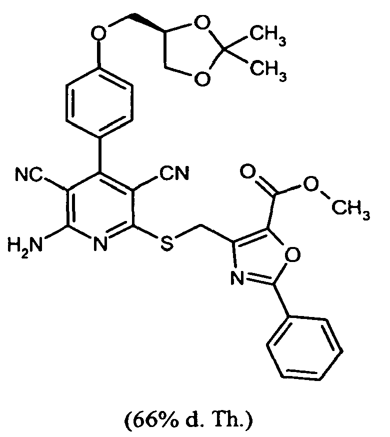 | 1.41 min (14); m/z = 598 | 8.12-7.92 (br. s, 2H), 8.05 (d, 2H), 7.67-7.57 (m, 3H), 7.50 (d, 2H), 7.13 (d, 2H), 4.83 (s, 2H), 4.49-4.41 (m, 1H), 4.17-4.06 (m, 3H), 3.93 (s, 3H), 3.79 (dd, 1H), 1.38 (s, 3H), 1.31 (s, 3H). |
| **77A** | 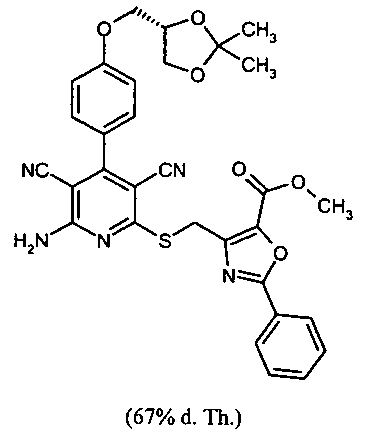 | 2.36 min (7); m/z = 598 | 8.11-7.96 (br. s, 2H), 8.06 (d, 2H), 7.67-7.58 (m, 3H), 7.51 (d, 2H), 7.13 (d, 2H), 4.83 (s, 2H), 4.48-4.41 (m, 1H), 4.15-4.05 (m, 3H), 3.93 (s, 3H), 3.79 (dd, 1H), 1.38 (s, 3H), 1.31 (s, 3H). |
| **78A** | 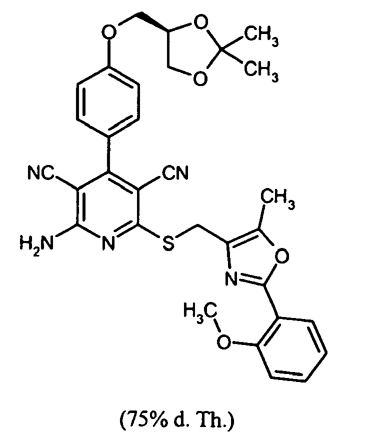 | 1.36 min (14); m/z = 584 | 8.16-7.96 (br. s, 2H), 7.75 (dd, 1H), 7.50 (d, 2H), 7.48 (dd, 1H), 7.19 (d, 1H), 7.12 (d, 2H), 7.05 (t, 1H), 4.52 (s, 2H), 4.48-4.41 (m, 1H), 4.15-4.05 (m, 3H), 3.86 (s, 3H), 3.79 (dd, 1H), 2.43 (s, 3H), 1.37 (s, 3H), 1.30 (s, 3H). |
| **79A** | 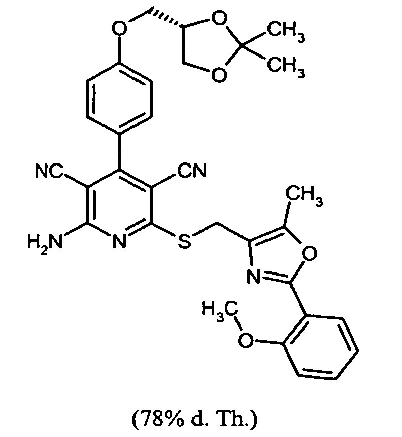 | 2.26 min (7); m/z = 584 | 8.17-7.95 (br. s, 2H), 7.76 (dd, 1H), 7.50 (d, 2H), 7.48 (dd, 1H), 7.19 (d, 1H), 7.12 (d, 2H), 7.04 (t, 1H), 4.52 (s, 2H), 4.48-4.41 (m, 1H), 4.15-4.03 (m, 3H), 3.86 (s, 3H), 3.79 (dd, 1H), 2.44 (s, 3H), 1.37 (s, 3H), 1.31 (s, 3H). |
| **80A** | 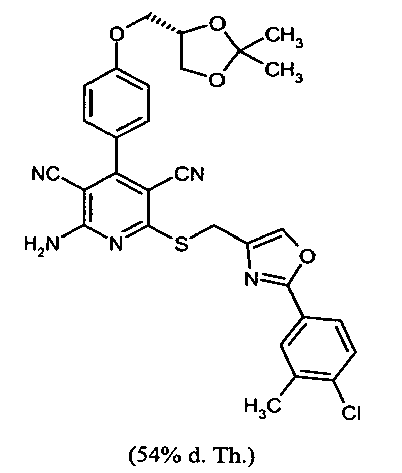 | 3.21 min (3); m/z = 588 | |
| **81A** | 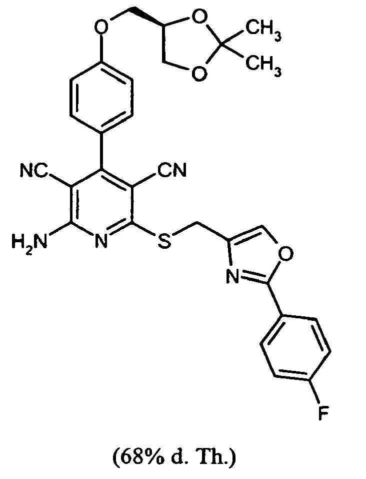 | 2.32 min (7); m/z = 558 | 8.33 (s, 1H), 8.23-8.02 (br. s, 2H), 8.01 (dd, 2H), 7.48 (d, 2H), 7.38 (t, 2H), 7.12 (d, 2H), 4.49-4.40 (m, 1H), 4.52 (s, 2H), 4.15-4.03 (m, 3H), 3.78 (dd, 1H), 1.37 (s, 3H), 1.31 (s, 3H). |
| **82A** | 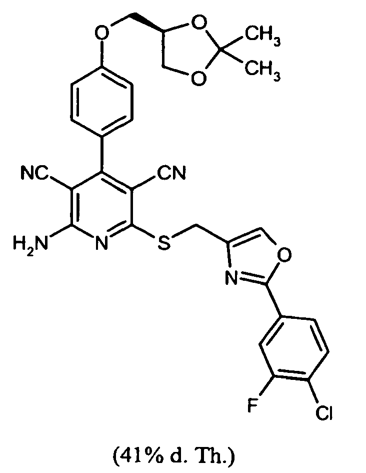 | 1.49 min (14); m/z = 592 | |
| **83A** | 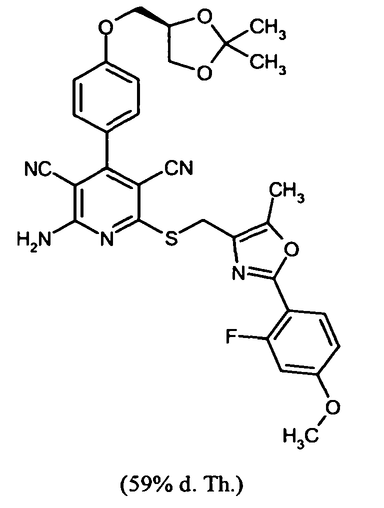 | 1.40 min (14); m/z = 602 | 8.19-7.96 (br. s, 2H), 7.87 (t, 1H), 7.49 (d, 2H, 7.12 (d, 2H), 7.01 (dd, 1H), 6.92 (dd, 1H), 4.50 (s, 2H), 4.48-4.40 (m, 1H), 4.16-4.04 (m, 3H), 3.83 (s, 3H), 3.79 (dd, 1H), 2.45 (s, 3H), 1.38 (s, 3H), 1.31 (s, 3H). |
| **84A** | 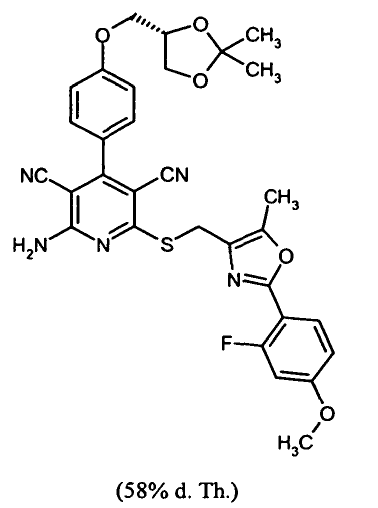 | 1.40 min (14); m/z = 602 | 8.15-7.96 (br. s, 2H), 7.87 (t, 1H), 7.49 (d, 2H), 7.12 (d, 2H), 7.01 (dd, 1H), 6.92 (dd, 1H), 4.51 (s, 2H), 4.49-4.41 (m, 1H), 4.16-4.04 (m, 3H), 3.84 (s, 3H), 3.79 (dd, 1H), 2.45 (s, 3H), 1.37 (s, 3H), 1.31 (s, 3H). |
| **85A** | 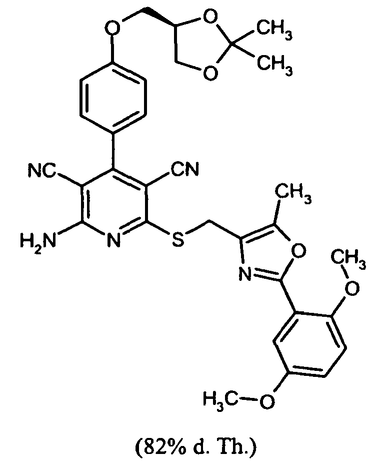 | 1.35 min (14); m/z = 614 | 8.16-7.97 (br. s, 2H), 7.49 (d, 2H), 7.28 (d, 1H), 7.12 (d, 2H), 7.11 (dd, 1H), 7.06 (dd, 1H), 4.51 (s, 2H), 4.48-4.41 (m, 1H), 4.15-4.03 (m, 3H), 3.81-3.73 (m, 1H), 3.79 (s, 3H), 3.75 (s, 3H), 2.43 (s, 3H), 137 (s, 3H), 1.31 (s, 3H). |
| **86A** | 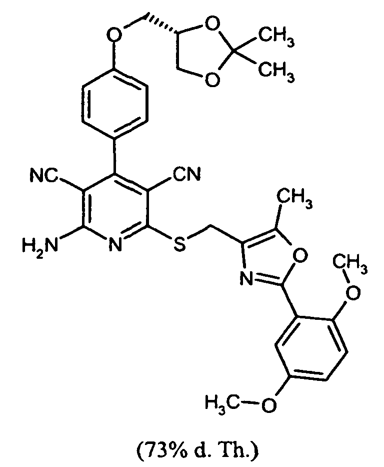 | 1.35 min (14); m/z = 614 | 8.17-7.98 (br. s, 2H), 7.49 (d, 2H), 7.28 (d, 1H), 7.13 (d, 2H), 7.12 (dd, 1H), 7.07 (dd, 1H), 4.51 (s, 2H), 4.48-4.41 (m, 1H), 4.15-4.04 (m, 3H), 3.81-3.73 (m, 1H), 3.79 (s, 3H), 3.75 (s, 3H), 2.43 (s, 3H), 1.37 (s, 3H), 1.31 (s, 3H). |
| **87A** | 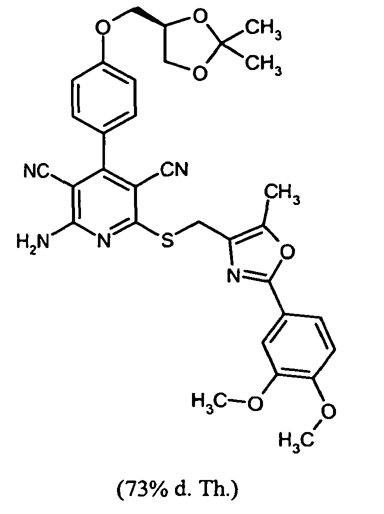 | 2.50 min (15); m/z = 614 | 8.17-7.96 (br. s, 2H), 7.49 (d, 2H), 7.48 (d, 1H), 7.40 (s, 1H), 7.13 (d, 2H), 7.08 (d, 1H), 4.49 (s, 2H), 4.48-4.40 (m, 1H), 4.16-4.04 (m, 3H), 3.84 (s, 3H), 3.83 (s, 3H), 3.82-3.75 (m, 1H), 2.45 (s, 3H), 1.36 (s, 3H), 1.31 (s, 3H). |
| **88A** | 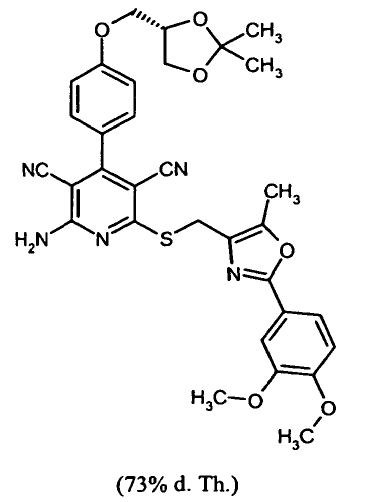 | 2.50 min (15); m/z = 614 | 8.17-7.96 (br. s, 2H), 7.49 (d, 2H), 7.48 (d, 1H), 7.40 (s, 1H), 7.13 (d, 2H), 7.08 (d, 1H), 4.50 (s, 2H), 4.48-4.40 (m, 1H), 4.16-4.04 (m, 3H), 3.84 (s, 3H), 3.83 (s, 3H), 3.79 (dd, 1H), 2.45 (s, 3H), 1.36 (s, 3H), 1.31 (s, 3H). |
| **89A** | 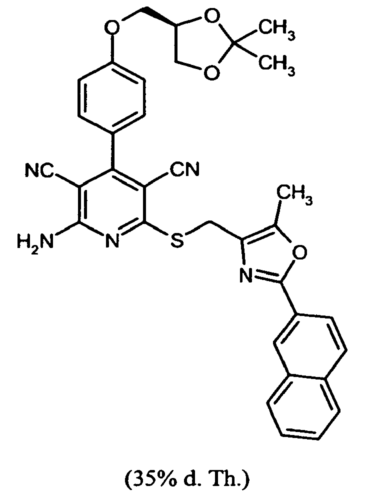 | 2.56 min (7); m/z = 604 | 8.52 (s, 1H), 8.13-7.95 (br. s, 2H), 8.13-8.08 (m, 1H), 8.03 (s, 2H), 8.00-7.95 (m, 1H), 7.63-7.57 (m, 2H), 7.50 (d, 2H), 7.12 (d, 2H), 4.55 (s, 2H), 4.48-4.42 (m, 1H), 4.15-4.05 (m, 3H), 3.79 (dd, 1H), 2.49 (s, 3H), 1.38 (s, 3H), 1.32 (s, 3H). |
| **90A** | 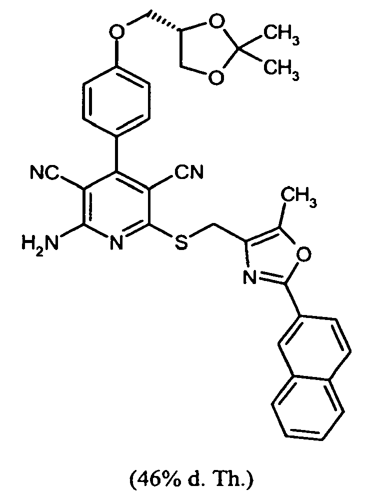 | 2.56 min (7); m/z = 604 | 8.52 (s, 1H), 8.13-7.94 (br. s, 2H), 8.13-8.08 (m, 1H), 8.03 (s, 2H), 8.00-7.94 (m, 1H), 7.62-7.57 (m, 2H), 7.49 (d, 2H), 7.12 (d, 2H), 4.56 (s, 2H), 4.48-4.40 (m, 1H), 4.15-4.05 (m, 3H), 3.79 (dd, 1H), 2.50 (s, 3H), 1.38 (s, 3H), 1.32 (s, 3H). |
| **91A** | 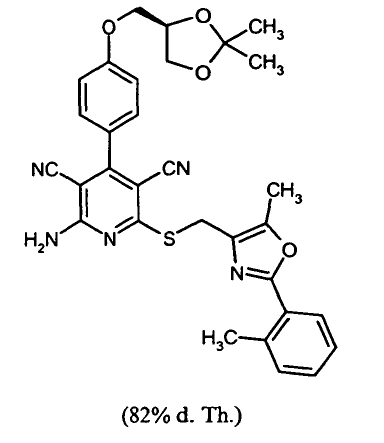 | 2.76 min (15); m/z = 568 | 8.13-7.92 (br. s, 2H), 7.85 (d, 1H), 7.49 (d, 2H), 7.40-7.29 (m, 3H), 7.12 (d, 2H), 4.52 (s, 2H), 4.46-4.41 (m, 1H), 4.15-4.05 (m, 3H), 3.78 (dd, 1H), 2.60 (s, 3H), 2.47 (s, 3H), 1.37 (s, 3H), 1.31 (s, 3H). |
| **92A** | 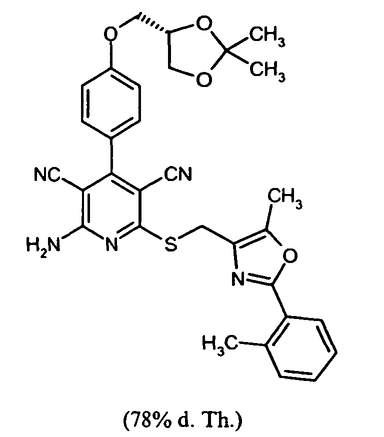 | 2.76 min (15); m/z = 568 | 8.13-7.94 (br. s, 2H), 7.85 (d, 1H), 7.49 (d, 2H), 7.41-7.30 (m, 3H), 7.12 (d, 2H), 4.52 (s, 2H), 4.47-4.41 (m, 1H), 4.15-4.05 (m, 3H), 3.78 (dd, 1H), 2.60 (s, 3H), 2.47 (s, 3H), 1.38 (s, 3H), 1.32 (s, 3H). |
| **93A** | 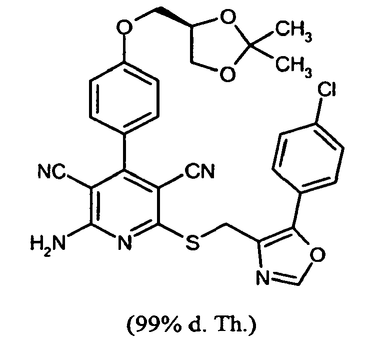 | 1.42 min (14); m/z = 574 | 8.51 (s, 1H), 8.04-7.92 (br. s, 2H), 7.73 (d, 2H), 7.61 (d, 2H), 7.50 (d, 2H), 7.12 (d, 2H), 4.74 (s, 2H), 4.48-4.41 (m, 1H), 4.15-4.04 (m, 3H), 3.79 (dd, 1H), 1.37 (s, 3H), 1.31 (s, 3H). |
| **94A** | 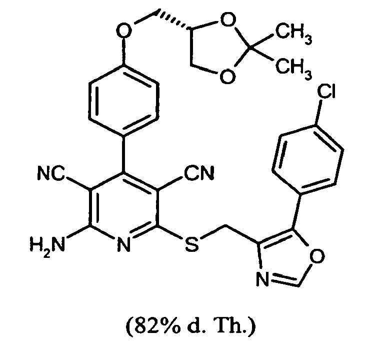 | 1.42 min (14); m/z = 574 | 8.50 (s, 1H), 8.04-7.90 (br. s, 2H), 7.73 (d, 2H), 7.61 (d, 2H), 7.50 (d, 2H), 7.12 (d, 2H), 4.74 (s, 2H), 4.48-4.41 (m, 1H), 4.15-4.05 (m, 3H), 3.79 (dd, 1H), 1.38 (s, 3H), 1.31 (s, 3H). |

### Beispiel 95A

2-Amino-4-(4-{[(2*S*)-2-{[*tert*.-butyl(dimethyl)silyl]oxy}propyl)oxy}phenyl)-6-({[2-(4-fluorphenyl)-5-methyl-1,3-oxazol-4-yl]methyl}thio)pyridin-3,5-dicarbonitril 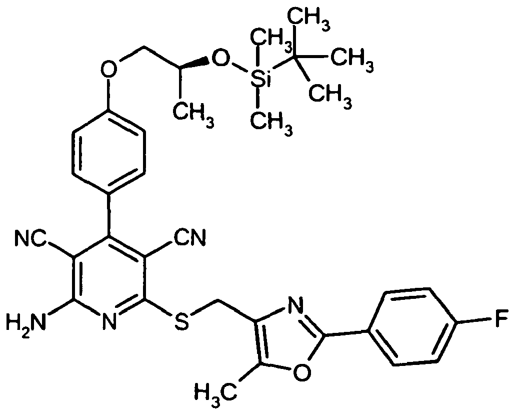 100 mg (0.18 mmol) der Verbindung aus Beispiel 7A, 45 mg (0.20 mmol) 4-(Chlormethyl)-2-(4-fluorphenyl)-5-methyl-1,3-oxazol und 46 mg (0.55 mmol) Natriumhydrogencarbonat werden in 2 ml trockenem DMF 20 h bei RT gerührt. Der Ansatz wird direkt mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 65 mg (57% d. Th.)

LC-MS (Methode 3): Rₜ = 3.53 min; MS (ESIpos): m/z = 630 [M+H]⁺.

Die in Tabelle 5 aufgeführten Beispiele werden aus den entsprechenden Ausgangsverbindungen analog zu Beispiel 95A hergestellt:

**Tabelle 5**

| **Beispiel Nr.** | **Struktur (Ausbeute)** | **LC-MS: Rₜ [min] (Methode); MS (ESI): M/z [M+H]⁺** | **¹H-NMR (DMSO-d₆):** δ **=** |
|---|---|---|---|
| **96A** | 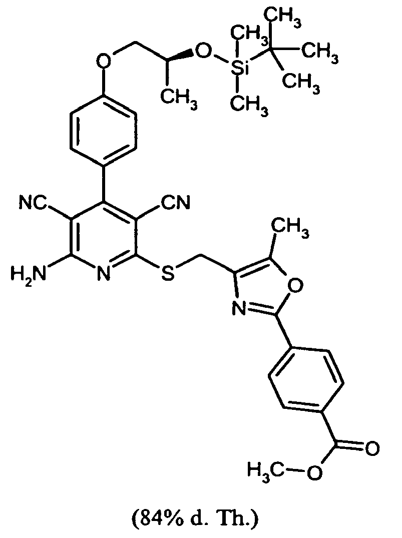 | 5.04 min (4); m/z = 670 | 8.37 (s, 1H), 8.29-7.91 (br. s, 2H), 7.97 (d, 2H), 7.61 (d, 2H), 7.47 (d, 2H), 7.12 (d, 2H), 4.48-4.39 (m, 1H), 4.42 (s, 2H), 4.16-4.03 (m, 3H), 3.77 (dd, 1H), 1.37 (s, 3H), 1.31 (s, 3H). |
| **97A** | 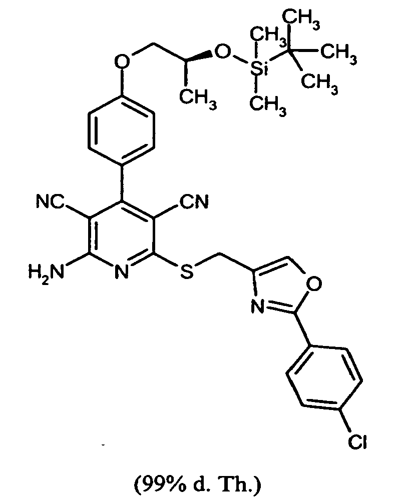 | 5.14 min (4); m/z = 632 [Mr | |

### Beispiel 98A

2-Anüno-4-(4-{[(4*S*)-2,2-dimethyl-1,3-dioxolan-4-yl]methoxy)phenyl)-6-(phenylthio)pyridin-3,5-dicarbonitril 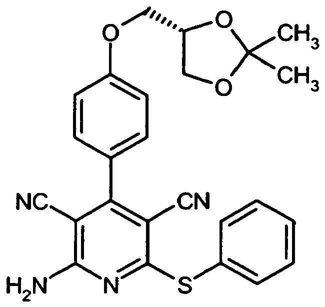 1.63 g (6.90 mmol) der Verbindung aus Beispiel 9A werden in 25 ml trockenem Ethanol vorgelegt und sukzessive mit 957 mg (14.49 mmol) Malonsäuredinitril, 798 mg (7.24 mmol) Thiophenol und 21 mg (0.21 mmol) Triethylamin versetzt. Das Reaktionsgemisch wird 2 h unter Rückfluss erhitzt. Nach Abkühlen auf RT wird das Lösungsmittel am Rotationsverdampfer entfernt und der Rückstand direkt mittels Säulenchromatographie an Kieselgel 60 aufgereinigt (Laufrnittel: Dichlormethan/Methanol 60:1). Es folgt eine weitere Reinigung mittels präparativer HPLC (Säule: YMC GEL ODS-AQ S-5 / 15 um; Laufmittel-Gradient: Acetonitril/Wasser 10:90 → 95:5).

Ausbeute: 850 mg (27% d. Th.)

LC-MS (Methode 3): Rₜ = 2.63 min; MS (ESIpos): m/z = 459 [M+H]⁺.

### Beispiel 99A

2-Amino-4-[4-(2-hydroxyethoxy)phenyl]-6-(phenylsulfanyl)pyridin-3,5-dicarbonitril

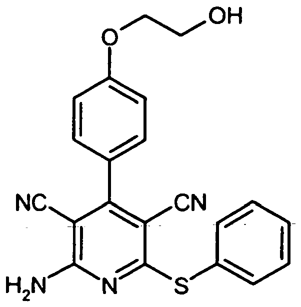

Die Titelverbindung wird ausgehend von 4-(2-Hydroxyethoxy)-benzaldehyd analog zu Beispiel 98A hergestellt.

Ausbeute: 21 % d. Th.

¹H-NMR (400 MHz, DMSO-d₆): δ = 7.83-7.19 (br. s, 2H), 7.64-7.58 (m, 2H), 7.53-7.48 (m, 5H), 7.12 (d, 2H), 5.10-4.75 (br. s, 1H), 4.10 (t, 2H), 3.75 (t, 2H).

LC-MS (Methode 7): R, = 1.76 min; MS (ESIpos): m/z = 389 [M+H]⁺.

### Beispiel 100A

[2-(4-Chlorphenyl)-1,3-oxazol-4-yl]methanol 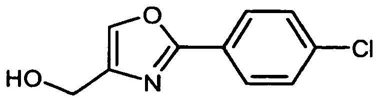 2.00 g (8.77 mmol) der Verbindung aus Beispiel 17A werden in 175 ml (17.54 mmol) 0.1 N Natronlauge suspendiert. Das Reaktionsgemisch wird 2 h unter Rückfluss gerührt. Der Ansatz wird dann mit Eis auf 0°C gekühlt, wobei langsam ein Niederschlag ausfällt. Das Gemisch wird mit ca. 100 ml Dichlormethan und 5 ml Ethanol versetzt. Die Phasen werden getrennt. Die wässrige Phase wird auf pH 7 gestellt und zweimal mit je 50 ml Dichlormethan (mit jeweils 3 ml Ethanol) extrahiert. Die vereinigten organischen Phasen werden über Magnesiumsulfat getrocknet. Nach Entfernen des Lösungsmittels am Rotationsverdampfer wird der Rückstand im Vakuum getrocknet.

Ausbeute: 1.20 g (65% d. Th.)

LC-MS (Methode 8): Rₜ = 3.05 min; MS (ESIpos): m/z = 210 [M+H]⁺.

### Beispiel 101A

2-Amino-6-{[2-(4-chlorphenyl)-1,3-oxazol-4-yl]methoxy)-4-(4-([(4*S*)-2,2-dimethyl-1,3-dioxolan-4-yl]methoxy}phenyl)pyridin-3,5-dicarbonitril 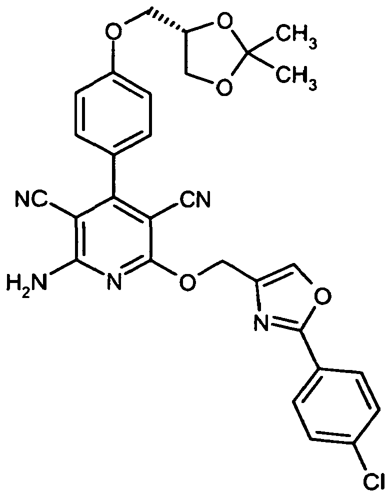 122 mg (1.09 mmol) Kalium-tert.-butylat werden in 2 ml trockenem 1,2-Dimethoxyethan suspendiert. Dann werden nacheinander 229 mg (1.09 mmol) der Verbindung aus Beispiel 100A und 100 mg (0.22 mmol) der Verbindung aus Beispiel 98A zugegeben. Das Reaktionsgemisch wird 2 h bei 60°C und nach Abkühlen weitere 10 h bei RT gerührt. Der Ansatz wird dann mit 5 ml Wasser versetzt. Der ausfallende Niederschlag wird abgesaugt und einmal mit ca. 2 ml kaltem Wasser gewaschen. Es folgt eine Reinigung mittels präparativer HPLC (Säule: YMC GEL ODS-AQ S-5 / 15 µm; Laufinittel-Gradient: Acetonitril/Wasser 10:90 → 95:5).

Ausbeute: 70 mg (58% d. Th.)

¹H-NMR (400 MHz, DMSO-d₆): δ = 8.47 (s, 1H), 8.18-7.89 (br. s, 2H), 8.01 (d, 2H), 7.62 (d, 2H), 7.48 (d, 2H), 7.12 (d, 2H), 5.41 (s, 2H), 4.48-4.41 (m, 1H), 4.15-4.04 (m, 3H), 3.79 (dd, 1H), 1.37 (s, 3H), 1.31 (s, 3H).

LC-MS (Methode 3): Rₜ = 2.82 min; MS (ESIpos): m/z = 558 [M+H]⁺.

### Beispiel 102A

6-Amino-4-[4-(2-hydroxyethoxy)phenyl]-2-oxo-1,2-dihydropyridin-3,5-dicarbonitril 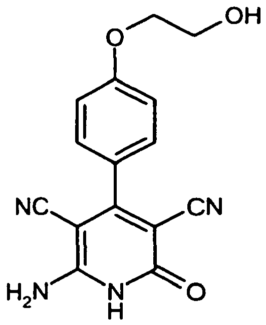 500 mg (1.29 mmol) der Verbindung aus Beispiel 99A werden in 6.4 ml Ethanol vorgelegt. Nach Zugabe von 2.57 g (28.96 mmol) Natriumhydroxid wird 30 min bei 80°C gerührt, wobei eine klare Lösung entsteht. Nach Abkühlen auf RT wird das Lösungsmittel am Rotationsverdampfer entfernt. Der Rückstand wird in 3 ml Wasser aufgenommen und mit 1 N Salzsäure sauer gestellt, bis ein gelblicher Niederschlag ausfällt. Die Suspension wird 3 h bei RT gerührt. Der Niederschlag wird abfiltriert, mit ca. 5 ml Wasser und wenig Ethanol gewaschen und dann noch einmal aus ca. 10 ml Ethanol umkristallisiert. Das so erhaltene Produkt wird ohne weitere Reinigung in den Folgereaktionen eingesetzt.

Ausbeute: 153 mg (36% d. Th., 89% Reinheit)

¹H-NMR (400 MHz, DMSO-d₆): δ = 11.85-11.72 (br. s, 1H), 7.87-7.60 (br. s, 2H), 7.43 (d, 2H), 7.10 (d, 2H), 5.08-4.52 (br. s, 1H), 4.08 (t, 2H), 3.74 (t, 2H).

LC-MS (Methode 3): Rₜ = 1.29 min; MS (ESIpos): m/z = 297 [M+H]⁺.

### Beispiel 103A

2-Chlor-6-({[2-(4-chlorphenyl)-1,3-oxazol-4-yl]methyl}thio)-4-(4-{[(2*S*)-2,3-dihydroxypropyl]-oxy}phenyl)pyridin-3,5-dicarbonitril 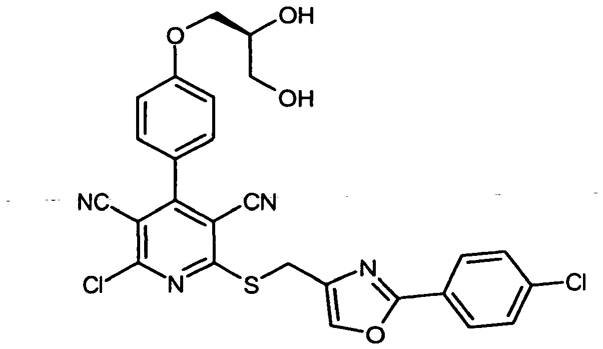 569 mg (4.86 mmol) Isopentylnitrit und 653 mg (4.86 mmol) Kupfer(II)chlorid werden in 20 ml trockenem Acetonitril vorgelegt und mit 465 mg (0.81 mmol) der Verbindung aus Beispiel 58A versetzt. Das Reaktionsgemisch wird 3 h bei 60°C gerührt. Nach Abkühlen auf RT wird der Ansatz mit 20 ml 1 N Salzsäure versetzt. Die wässrige Phase wird zweimal mit je 30 ml Ethylacetat extrahiert. Die vereinigten organischen Phasen werden je einmal mit 10 ml ges. wässriger Natriumhydrogencarbonat-Lösung und 10 ml ges. wässriger Natriumchlorid-Lösung gewaschen. Nach Trocknen über Magnesiumsulfat wird das Lösungsmittel am Rotationsverdampfer entfernt. Der Rückstand wird mittels präparativer HPLC aufgereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt, welches ohne weitere Reinigung in der Folgereaktion eingesetzt wird.

Ausbeute: 108 mg (18% d. Th., 73% Reinheit)

LC-MS (Methode 5): Rₜ = 3.85 min; MS (ESIpos): m/z = 553 [M+H]⁺.

Die in Tabelle 6 aufgeführten Beispiele werden aus den entsprechenden Ausgangsverbindungen analog zu Beispiel 46A hergestellt:

**Tabelle 6**

| **Beispiel Nr.** | **Struktur (Ausbeute)** | **LC-MS: Rₜ [min] (Methode); MS (ESI): m/z [M+H]⁺** | **¹H-NMR (DMSO-d₆): δ=** |
|---|---|---|---|
| **104A** | 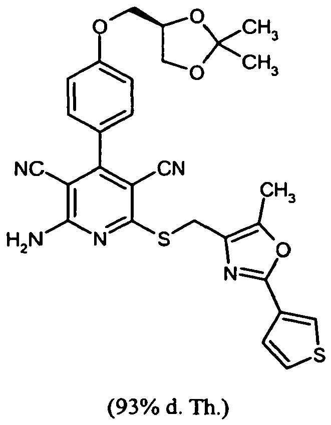 | 2.82 min (3); m/z = 560 | 8.16-7.95 (br. s, 2H), 8.11 (d, 1H), 7.71 (dd, 1H), 7.51 (d, 1H), 7.49 (d, 2H), 7.12 (d, 2H), 4.48 (s, 2H), 4.47-4.40 (m, 1H), 4.16-4.03 (m, 3H), 3.79 (dd, 1H), 2.45 (s, 3H), 1.38 (s, 3H), 1.31 (s, 3H). |
| **105A** | 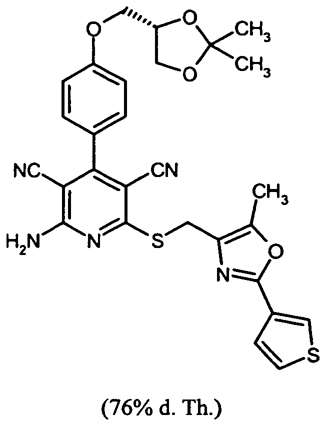 | 2.82 min (3); m/z = 560 | 8.17-7.95 (br. s, 2H), 8.11 (d, 1H), 7.70 (dd, 1H), 7.52 (d, 1H), 7.49 (d, 2H), 7.12 (d, 2H), 4.49 (s, 2H), 4.47-4.40 (m, 1H), 4.15-4.03 (m, 3H), 3.78 (dd, 1H), 2.45 (s, 3H), 1.38 (s, 3H), 1.31 (s, 3H). |

Die in Tabelle 7 aufgeführten Verbindungen können aus den entsprechenden Ausgangsmaterialien analog zu den Vorschriften der Beispiele 16A und 34A hergestellt werden:

**Tabelle 7**

| **Beispiel Nr.** | **Struktur** | **LC-MS: Rₜ [min] (Methode); MS (ESI): m/z [M+H]⁺** | **¹H-NMR (400 MHz, DMSO-d₆):** δ **=** |
|---|---|---|---|
| **106A** | 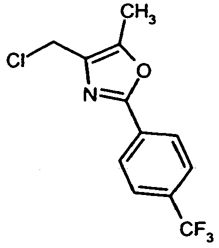 | 2.73 min (3); m/z = 276 | 8.13 (d, 2H), 7.90 (d, 2H), 4.79 (s, 2H), 2.48 (s, 3H). |
| **107A** | 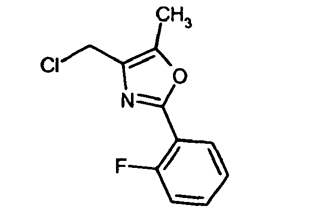 | 2.31 min (3); m/z = 226 | 7.98 (dt, 1H), 7.62-7.54 (m, 1H), 7.44-7.33 (m, 2H), 4.78 (s, 2H), 2.46 (s, 3H). |
| **108A** | 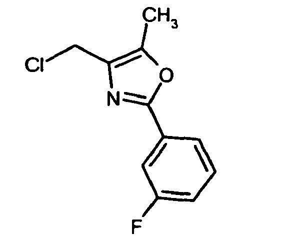 | 2.45 min (3); m/z = 226 | 7.78 (d, 1H), 7.67 (d, 1H), 7.59 (q, 1H), 7.37 (dt, 1H), 4.77 (s, 2H), 2.46 (s, 3H). |
| **109A** | 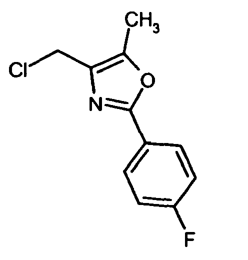 | 2.42 min (3); m/z = 226 | 7.98 (dd, 2H), 7.37 (pseudo-t, 2H), 4.75 (s, 2H), 2.44 (s, 3H). |
| **110A** | 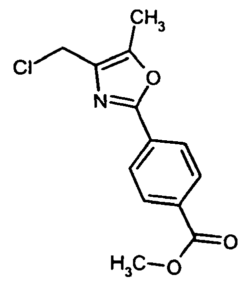 | 2.42 min (3); m/z = 266 | 8.12-8.04 (m, 4H), 4.79 (s, 2H), 3.89 (s, 3H), 2.48 (s, 3H). |
| **111A** | 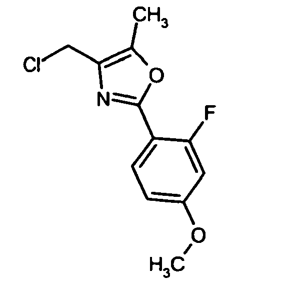 | 2.38 min (3); m/z = 256 | 7.88 (t, 1H), 7.02 (dd, 1H), 6.94 (dd, 1H), 4.76 (s, 2H), 3.84 (s, 3H), 2.42 (s, 3H). |
| **112A** | 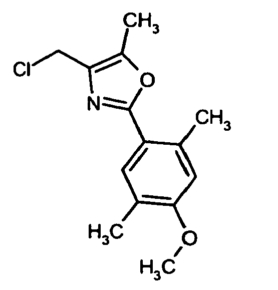 | 2.79 min (3); m/z = 266 | 7.65 (s, 1H), 6.91 (s, 1H), 4.73 (s, 2H), 3.83 (s, 3H), 2.59 (s, 3H), 2.41 (s, 3H), 2.16 (s, 3H). |
| **113A** | 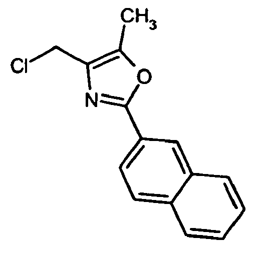 | 2.75 min (3); m/z = 258 | 8.54 (s, 1H), 8.13-8.08 (m, 1H), 8.05 (d, 2H), 8.02-7.96 (m, 1H), 7.63-7.58 (m, 2H), 4.80 (s, 2H), 2.49 (s, 3H). |
| **114A** | 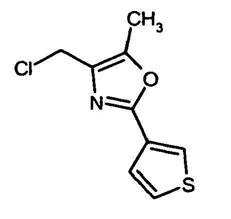 | 2.21 min (3); m/z = 214 | 8.14 (d, 1H), 7.72 (dd, 1H), 7.53 (d, 1H), 4.73 (s, 2H), 2.41 (s, 3H). |
| **115A** | 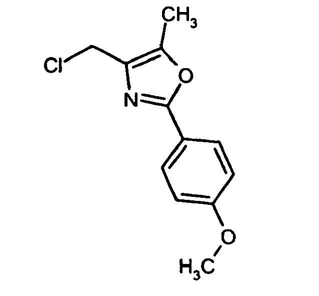 | 2.36 min (3); m/z = 238 | 7.87 (d, 2H), 7.08 (d, 2H), 4.72 (s, 2H), 3.82 (s, 3H), 2.42 (s, 3H). |
| **116A** | 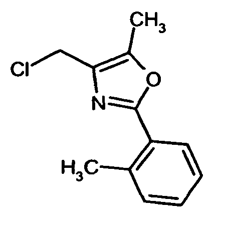 | 2.60 min (3); m/z = 221 | 7.88 (d, 1H), 7.43-7.31 (m, 3H), 4.78 (s, 2H), 2.61 (s, 3H), 2.43 (s, 3H). |
| **117A** | 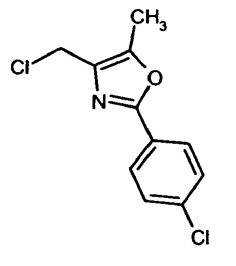 | 2.63 min (3); m/z = 242 | 7.94 (d, 2H), 7.60 (d, 2H), 4.77 (s, 2H), 2.45 (s, 3H). |
| **118A** | 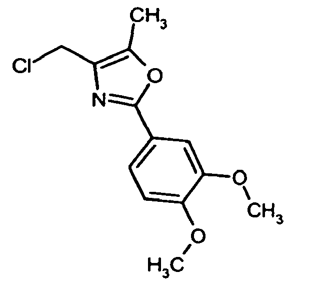 | 2.19 min (3); m/z = 268 | 7.50 (dd, 1H), 7.42 (s, 1H), 7.09 (d, 1H), 4.73 (s, 2H), 3.85 (s, 3H), 3.83 (s, 3H), 2.42 (s, 3H). |
| **119A** | 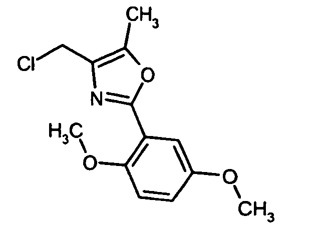 | 2.21 min (3); m/z = 268 | 7.30 (d, 1H), 7.13 (d, 1H), 7.08 (dd, 1H), 4.75 (s, 2H), 3.81 (s, 3H), 3.77 (s, 3H), 2.42 (s, 3H). |
| **120A** | 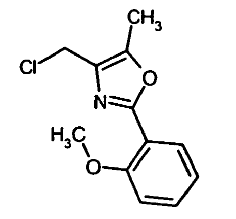 | 2.19 min (3); m/z = 238 | 7.78 (d, 1H), 7.49 (dt, 1H), 7.19 (d, 1H), 7.06 (t, 1H), 4.75 (s, 2H), 3.86 (s, 3H), 2.42 (s, 3H). |
| **121A** | 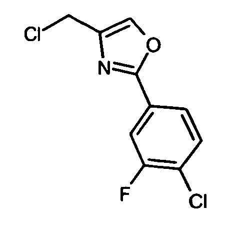 | 1.28 min (14); m/z = 246 | |
| **122A** | 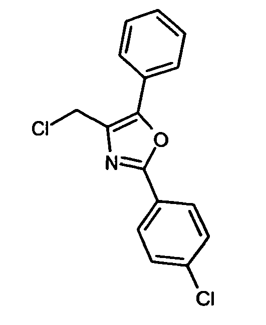 | 2.84 min (6); m/z = 304 | 8.10 (d, 2H), 7.84 (d, 2H), 7.65 (d, 2H), 7.60 (t, 2H), 7.50 (t, 1H), 4.98 (s, 2H). |

### Beispiel 123A

2-Chlor-6-({[2-(4-chlorphenyl)-1,3-oxazol-4-yl]methyl}sulfanyl)-4-[4-(2-hydroxyethoxy)phenyl]-pyridin-3,5-dicarbonitril 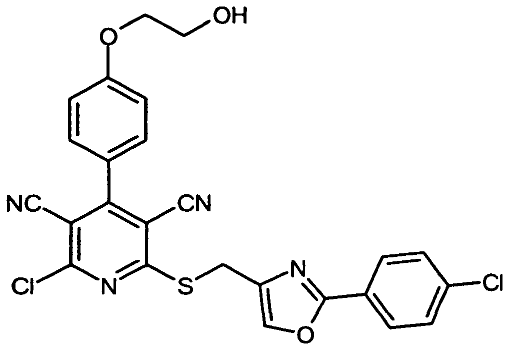 156 mg (1.33 mmol) Isopentylnitrit und 179 mg (1.33 mmol) Kupfer(II)chlorid werden in 17 ml trockenem Acetonitril vorgelegt und mit 336 mg (0.67 mmol) der Verbindung aus Beispiel 15 versetzt. Das Reaktionsgemisch wird 3 h bei 60°C gerührt. Der Ansatz wird danach mit 17 ml 1N Salzsäure versetzt und zweimal mit je 30 ml Ethylacetat extrahiert. Die vereinigten organischen Phasen werden über Magnesiumsulfat getrocknet. Nach Entfernen des Lösungsmittels wird das Rohprodukt ohne weitere Reinigung in der Folgereaktion eingesetzt.

Ausbeute: 410 mg (78% d. Th., 67% Reinheit).

Ein 85 mg-Aliquot des Rohprodukts wird mittels präparativer HPLC gereinigt (Säule: YMC GEL ODS-AQ S-5 / 15 µm; Laufmittel-Gradient: Acetonitril/Wasser 10:90 → 95:5). Man erhält so 14 mg der reinen Zielverbindung.

¹H-NMR (400 MHz, DMSO-d₆): δ = 8.18 (s, 1H), 7.98 (d, 2H), 7.62 (dd, 4H), 7.19 (d, 2H), 4.98-4.88 (br. s, 1H), 4.58 (s, 2H), 4.11 (t, 2H), 3.79-3.71 (br. s, 2H).

LC-MS (Methode 14): Rₜ = 1.46 min; MS (ESIpos): m/z = 523 [M+H]⁺.

### Beispiel 124A

2-Chlor-6-{[2-(4-chlorphenyl)-1,3-oxazol-4-yl]methoxy}-4-[4-(2-hydroxyethoxy)phenyl]pyridin-3,5-dicarbonitril

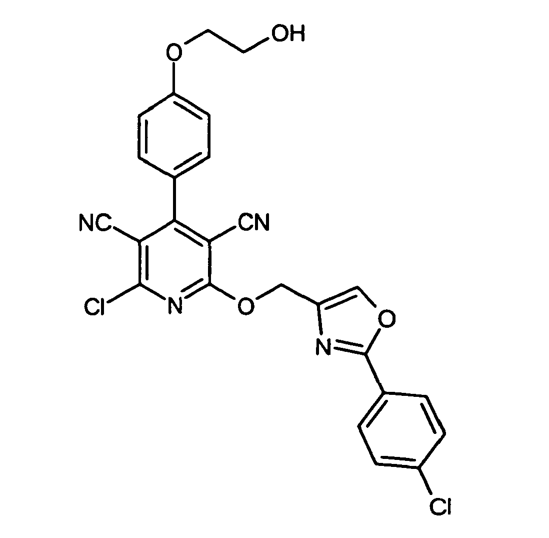

Die Titelverbindung wird auf analoge Weise zu Beispiel 123A ausgehend von Beispiel 110 hergestellt.

LC-MS (Methode 3): Rₜ = 2.76 min; MS (ESIpos): m/z = 507 [M+H]⁺.

### Beispiel 125A

2-Chlor-6-({[2-(4-chlorphenyl)-1,3-oxazol-4-yl]methyl}sulfanyl)-4-[3-fluor-4-(2-hydroxyethoxy)-phenyl]pyridin-3,5-dicarbonitril 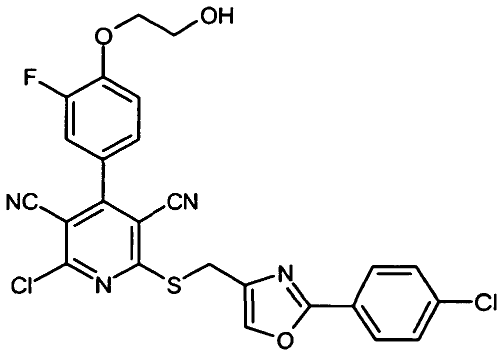 150 mg (0.287 mmol) 2-Amino-6-({[2-(4-chlorphenyl)-1,3-oxazol-4-yl]methyl}sulfanyl)-4-[3-fluor-4-(2-hydroxyethoxy)phenyl]pyridin-3,5-dicarbonitril (Beispiel 20) werden in 20 ml Acetonitril vorgelegt, mit 258 µl (1.724 mmol) Isoamylnitrit sowie 232 mg (1.724 mmol) Kupfer(II)-chlorid versetzt und über Nacht bei Raumtemperatur gerührt. Das Reaktionsgemisch wird danach auf gesättigte Ammoniumchlorid-Lösung gegeben und mit Essigsäureethylester extrahiert. Die organische Phase wird mit gesättigter Natriumchlorid-Lösung gewaschen, über Natriumsulfat getrocknet und eingeengt. Der Rückstand wird mittels präparativer HPLC gereinigt (Laufmittel-Gradient: Acetonitril/Wasser 10:90 → 95:5). Man erhält 25 mg (16% d. Th.) der Zielverbindung.

LC-MS (Methode 7): Rₜ = 2.43 min; MS (ESIpos): m/z = 541 [M+H]⁺.

### Ausführungsbeispiele:

### Beispiel 1

2-Amino-6-({[2-(4-chlor-3-methylphenyl)-1,3-oxazol-4-yl]methyl}thio)-4-[4-(2-hydroxyethoxy)-phenyl]pyridin-3,5-dicarbonitril 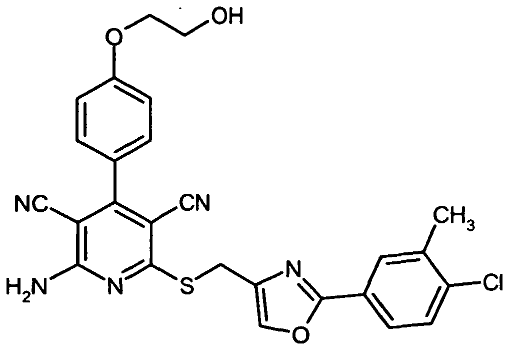 52 mg (0.17 mmol) der Verbindung aus Beispiel 1A und 89 mg (0.18 mmol) der Verbindung aus Beispiel 28A werden zusammen mit 42 mg (0.50 mmol) Natriumhydrogencarbonat in 1.8 ml trockenem DMF suspendiert. Das Reaktionsgemisch wird 12 h bei RT gerührt. Der Ansatz wird danach filtriert und das Filtrat direkt mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 16 mg (38% d. Th.)

¹H-NMR (400 MHz, DMSO-d₆): δ = 8.36 (s, 1H), 8.30-7.90 (br. s, 2H), 7.96 (s, 1H), 7.79 (d, 1H), 7.58 (d, 1H), 7.47 (d, 2H), 7.10 (d, 2H), 4.91 (t, 1H), 4.41 (s, 2H), 4.12-4.05 (m, 2H), 3.73 (dt, 2H), 2.41 (s, 3H).

LC-MS (Methode 6): Rₜ = 2.36 min; MS (ESIpos): m/z = 518 [M+H]⁺.

Die in Tabelle 8 aufgeführten Beispiele werden aus den entsprechenden Ausgangsverbindungen analog zu Beispiel 1 hergestellt:

**Tabelle 8**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ**=** |
|---|---|---|---|
| **2** | 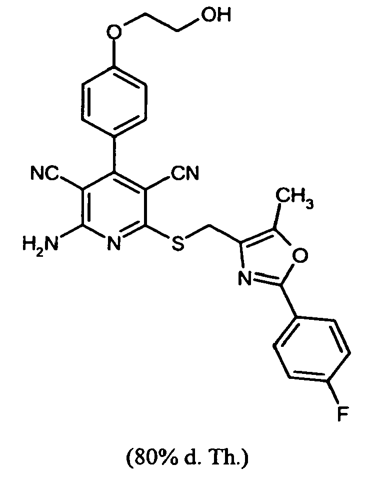 | 3.63 min (4); m/z = 502 | 8.22-7.93 (br. s, 2H), 7.97 (d, 1H), 7.95 (d, 1H), 7.49 (d, 2H), 7.36 (t, 2H), 7.11 (d, 2H), 4.99 (t, 1H), 4.50 (s, 2H), 4.08 (t, 2H), 3.73 (dt, 2H), 2.45 (s, 3H). |
| **3** | 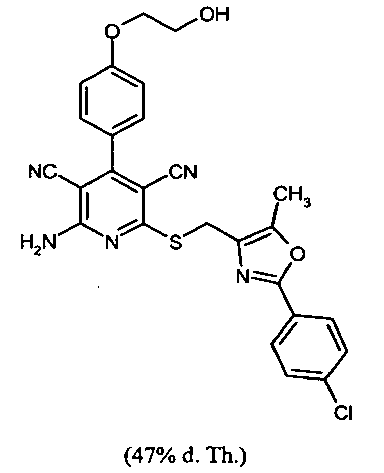 | 2.15 min (7); m/z = 518 | 8.18-7.96 (br. s, 2H), 7.92 (d, 2H), 7.59 (d, 2H), 7.48 (d, 2H), 7.10 (d, 2H), 4.91 (t, 1H), 4.50 (s, 2H), 4.07 (t, 2H), 3.73 (dt, 2H), 2.47 (s, 3H). |
| **4** | 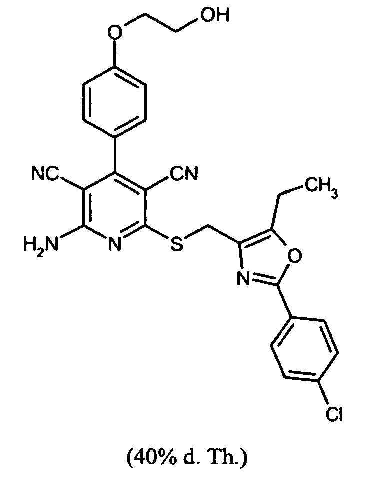 | 2.43 min (6); m/z = 532 | 8.23-7.89 (br. s, 2H), 7.95 (d, 2H), 7.59 (d, 2H), 7.48 (d, 2H), 7.10 (d, 2H), 4.91 (t, 1H), 4.51 (s, 2H), 4.07 (t, 2H), 3.74 (q, 2H), 2.38 (dt, 2H), 1.20 (t, 3H). |
| **5** | 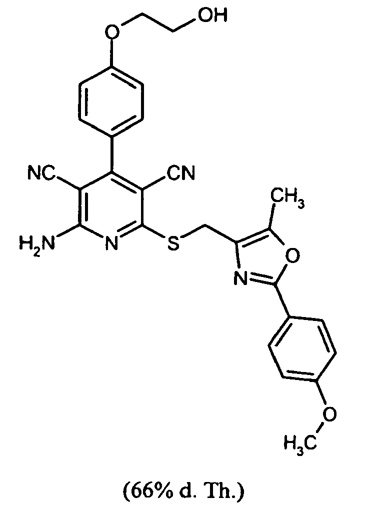 | 2.53 min (3); m/z = 514 | 8.17-7.92 (br. s, 2H), 7.85 (d, 2H), 7.48 (d, 2H), 7.12 (d, 2H), 7.05 (d, 2H), 4.91 (t, 1H), 4.49 (s, 2H), 4.08 (t, 2H), 3.81 (s, 3H), 3.73 (dt, 2H), 2.44 (s, 3H). |
| **6** | 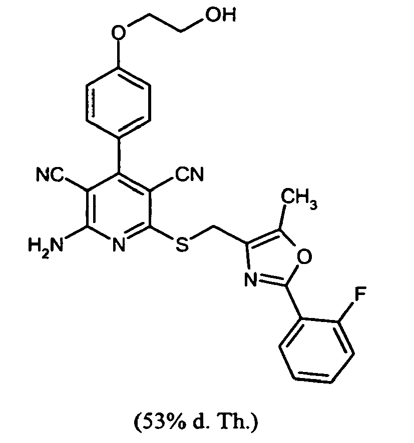 | 2.50 min (3); m/z = 502 | 8.19-7.92 (br. s, 2H), 7.96 (t, 1H), 7.61-7.52 (m, 1H), 7.48 (d, 2H), 7.93-7.31 (m, 2H), 7.11 (d, 2H), 4.92 (t, 1H), 4.53 (s, 2H), 4.08 (t, 2H), 3.78-3.71 (m, 2H), 2.48 (s, 3H). |
| **7** | 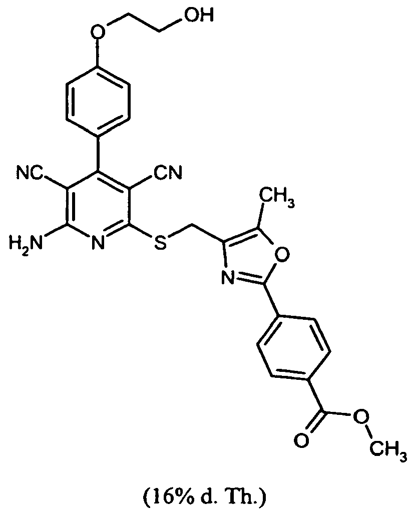 | 3.44 min (5); m/z = 542 | 8.12-7.98 (m, 6H), 7.48 (d, 2H), 7.10 (d, 2H), 4.90 (t, 1H), 4.53 (s, 2H), 4.07 (t, 2H), 3.88 (s, 3H), 3.73 (dt, 2H), 2.50 (s, 3H). |
| **8** | 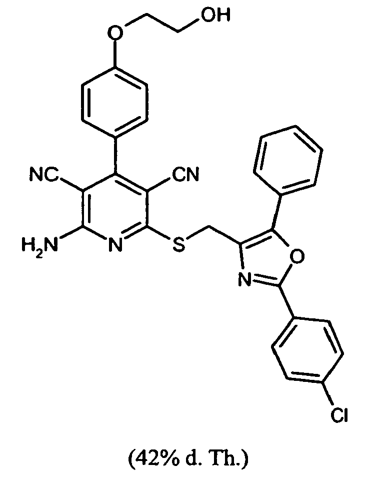 | 3.10 min (3); m/z = 580 | 8.10 (d, 2H), 7.99-7.89 (br. s, 2H), 7.85 (d, 2H), 7.64 (d, 2H), 7.59 (d, 2H), 7.52-7.47 (m, 3H), 7.10 (d, 2H), 4.91 (t, 1H), 4.81 (s, 2H), 4.07 (t, 2H), 3.73 (dt, 2H). |
| **9** | 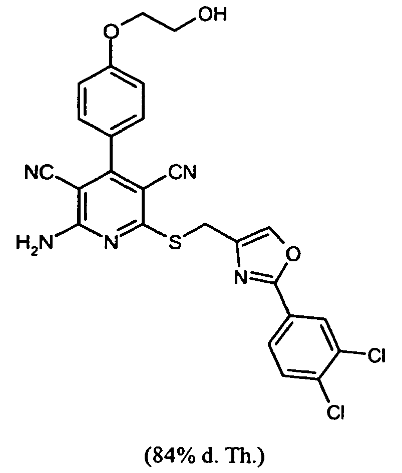 | 2.84 min (3); m/z= 538 | 8.41 (s, 1H), 8.28-7.97 (br. s, 2H), 8.11 (d, 1H), 7.93 (dd, 1H), 7.80 (d, 1H), 7.48 (d, 2H), 7.10 (d, 2H), 4.91 (t, 1H), 4.42 (s, 2H), 4.08 (t, 2H), 3.75 (dt, 2H). |
| **10** | 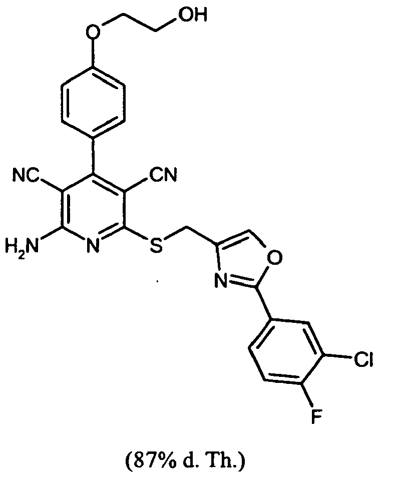 | 2.70 min (3); m/z = 522 | 8.39 (s, 1H), 8.28-7.91 (br. s, 2H), 8.10 (dd, 1H), 8.00-7.94 (m, 1H), 7.59 (pseudo-t, 1H), 7.47 (d, 2H), 7.09 (d, 2H), 4.91 (t, 1H), 4.41 (s, 2H), 4.07 (t, 2H), 3.73 (dt, 2H). |
| **11** | 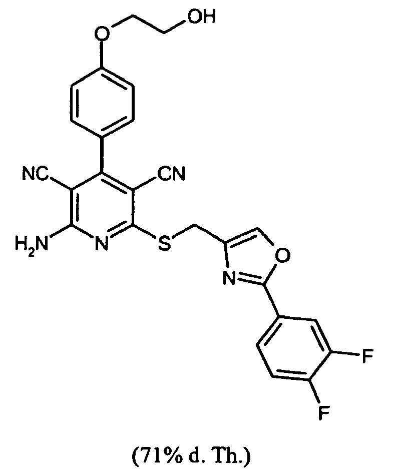 | 2.43 min (9); m/z = 506 | 8.39 (s, 1H), 8.30-7.91 (br. s, 2H), 7.96 (dt, 1H), 7.87-7.78 (m, 1H), 7.62 (q, 1H), 7.47 (d, 2H), 7.10 (d, 2H), 4.90 (t, 1H), 4.42 (s, 2H), 4.11-4.03 (m, 2H), 3.73 (q, 2H). |
| **12** | 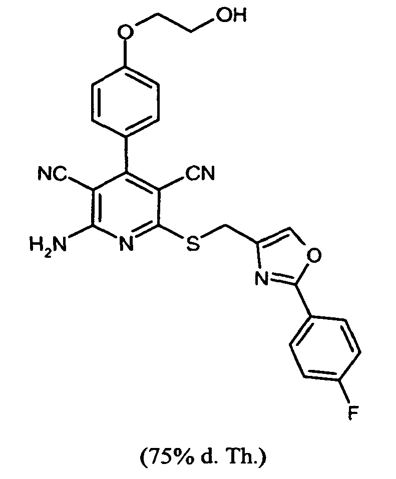 | 2.44 min (9); m/z = 488 | 8.34 (s, 1H), 8.26-7.93 (br. s, 2H), 8.02 (d, 1H), 8.01 (d, 1H), 7.47 (d, 2H), 7.37 (t, 2H), 7.10 (d, 2H), 4.91 (t, 1H), 4.41 (s, 2H), 4.08 (t, 2H), 3.74 (q, 2H). |
| **13** | 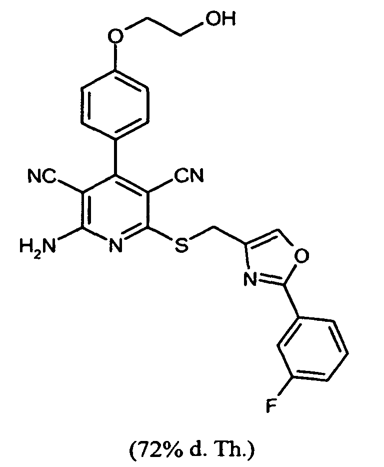 | 2.41 min (9); m/z = 488 | 8.48 (s, 1H), 8.27-7.93 (br. s, 2H), 7.83 (d, 1H), 7.72 (d, 1H), 7.60 (q, 1H), 7.47 (d, 2H), 7.39 (dt, 1H), 7.11 (d, 2H), 4.97 (t, 1H), 4.43 (s, 2H), 4.08 (t, 2H), 3.75 (q, 2H). |
| **14** | 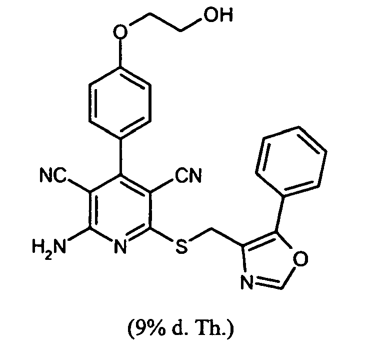 | 2.29 min (9); m/z = 470 | 8.48 (s, 1H), 7.98-7.82 (br. s, 2H), 7.72 (d, 2H), 7.58-7.43 (m, 5H), 7.11 (d, 2H), 4.93 (t, 1H), 4.73 (s, 2H), 4.09 (t, 2H), 3.74 (q,2H). |
| **15** | 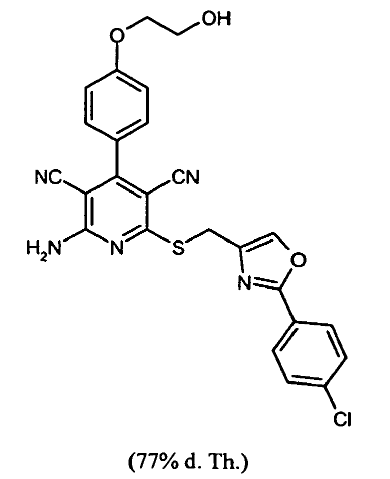 | 2.53 min (3); m/z = 504 | 8.37 (s, 1H), 8.31-7.89 (br. s, 2H), 7.97 (d, 2H), 7.60 (d, 2H), 7.46 (d, 2H), 7.10 (d, 2H), 4.91 (t, 1H), 4.41 (s, 2H), 4.08 (t, 2H), 3.74 (q, 2H). |
| 1**6** | 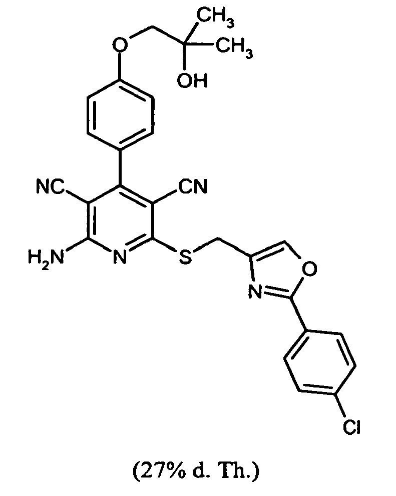 | 2.83 min (3); m/z = 532 | 8.38 (s, 1H), 8.27-7.93 (br. s, 2H), 7.97 (d, 2H), 7.61 (d, 2H), 7.45 (d, 2H), 7.09 (d, 2H), 4.69 (s, 1H), 4.42 (s, 2H), 3.80 (s, 2H), 1.23 (s, 6H). |
| **17** | 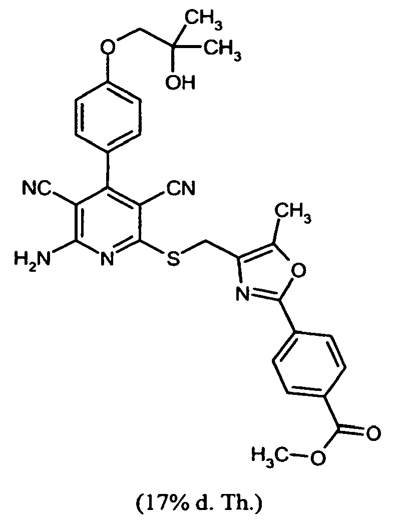 | 3.83 min (4); m/z = 570 | 8.20-7.94 (br. s, 2H), 8.13-8.05 (m, 4H), 7.48 (d, 2H), 7.10 (d, 2H), 4.68 (s, 1H), 4.53 (s, 2H), 3.90 (s, 3H), 3.79 (s, 2H), 1.21 (s, 6H). |
| **18** | 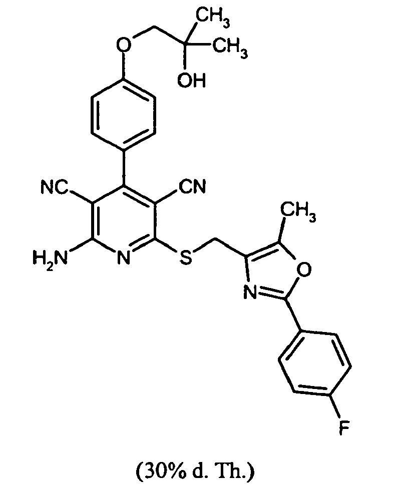 | 2.76 min (3); m/z = 530 | 8.20-7.93 (br. s, 2H), 7.97 (dd, 2H), 7.48 (d, 2H), 7.35 (pseudo-t, 2H), 7.10 (d, 2H), 4.68 (s, 1H), 4.51 (s, 2H), 3.80 (s, 2H), 2.46 (s, 3H), 1.21 (s, 6H). |
| **19** | 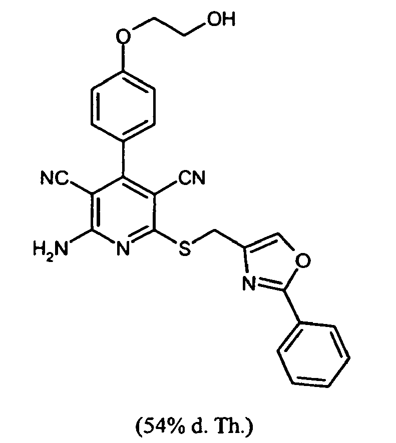 | 2.49 min (3); m/z = 470 | 8.34 (s, 1H), 8.27-7.89 (br. s, 2H), 7.99-7.93 (m, 2H), 7.59-7.50 (m, 3H), 7.47 (d, 2H), 7.10 (d, 2H), 4.91 (t, 1H), 4.43 (s, 2H), 4.08 (t, 2H), 3.73 (q, 2H). |
| **20** | 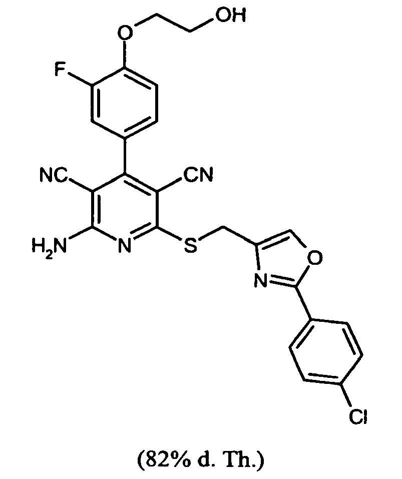 | 1.30 min (14); m/z = 522 | 8.36 (s, 1H), 8.31-8.02 (br. s, 2H), 7.98 (d, 2H), 7.60 (d, 2H), 7.50 (dd, 1H), 7.39-7.29 (m, 2H), 4.96 (t, 1H), 4.43 (s, 2H), 4.17 (t, 2H), 3.76 (q, 2H). |
| **21** | 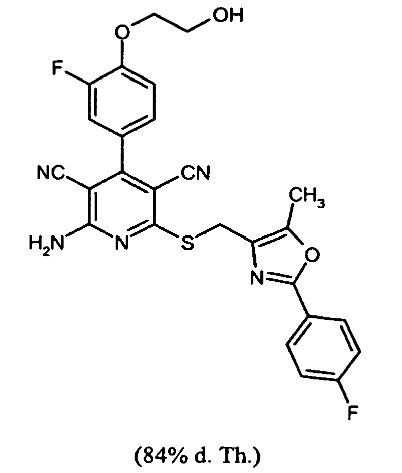 | 1.26 min (14); m/z = 520 | 8.25-8.00 (br. s, 2H), 7.98 (d, 1H), 7.96 (d, 1H), 7.52 (dd, 1H), 7.40-7.30 (m, 4H), 4.96 (t, 1H), 4.51 (s, 2H), 4.17 (t, 2H), 3.75 (q, 2H), 2.47 (s, 3H). |
| **22** | 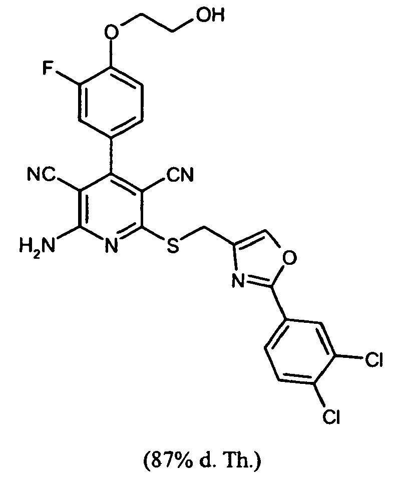 | 1.39 min (14); m/z = 556 | 8.42 (s, 1H), 8.34-7.98 (br. s, 2H), 8.12 (s, 1H), 7.93 (dd, 1H), 7.81 (d, 1H), 7.49 (dd, 1H), 7.49-7.39 (m, 4H), 4.97 (t, 1H), 4.42 (s, 2H), 4.17 (t, 2H), 3.78 (q, 2H). |
| **23** | 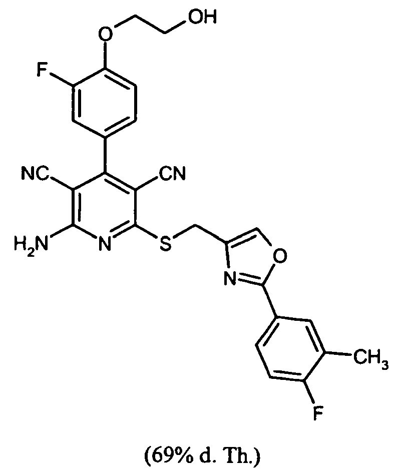 | 1.29 min (14); m/z = 520 | 8.33 (s, 1H), 8.29-7.97 (br. s, 2H), 7.91 (dd, 1H), 7.85-7.78 (m, 1H), 7.49 (dd, 1H), 7.38-7.27 (m, 3H), 4.96 (t, 1H), 4.41 (s, 2H), 4.15 (t, 2H), 3.76 (q, 2H), 2.31 (s, 3H). |
| **24** | 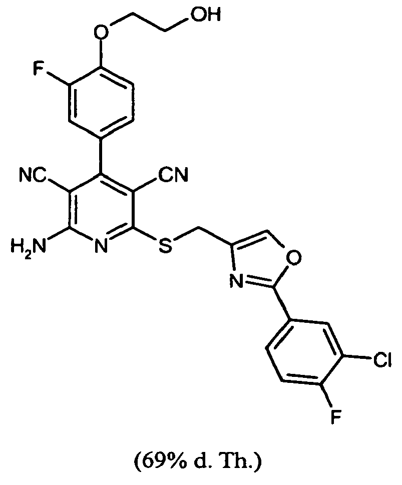 | 2.76 min (3); m/z = 540 | 8.39 (s, 1H), 8.33-7.90 (br. s, 2H), 8.10 (dd, 1H), 8.00-7.93 (m, 1H), 7.59 (t, 1H), 7.49 (dd, 1H), 7.35 (q, 1H), 7.33 (d, 1H), 4.96 (t, 1H), 4.42 (s, 2H), 4.16 (t, 2H), 3.77 (q, 2H). |
| **25** | 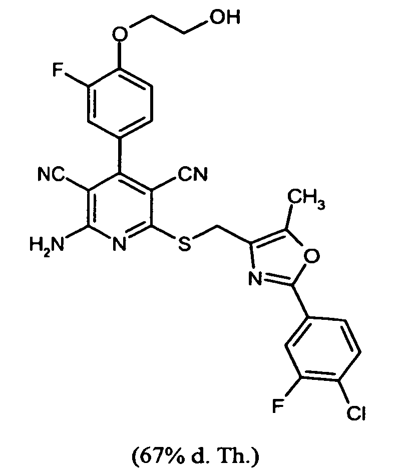 | 2.87 min (3); m/z = 554 | 8.27-7.98 (br. s, 2H), 7.86 (d, 1H), 7.80-7.72 (m, 2H), 7.52 (dd, 1H), 7.40-7.31 (m, 2H), 4.97 (t, 1H), 4.52 (s, 2H), 4.17 (t, 2H), 3.77 (q, 2H), 2.49 (s,3H). |
| **26** | 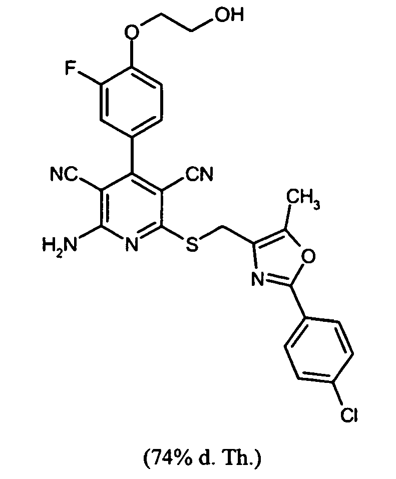 | 2.81 min (3); m/z = 536 | 8.23-7.99 (br. s, 2H), 7.92 (d, 2H), 7.59 (d, 2H), 7.52 (dd, 1H), 7.36 (q, 1H), 7.35 (s, 1H), 4.96 (t, 1H), 4.51 (s, 2H), 4.17 (t, 2H), 3.77 (q, 2H), 2.46 (s, 3H). |
| **27** | 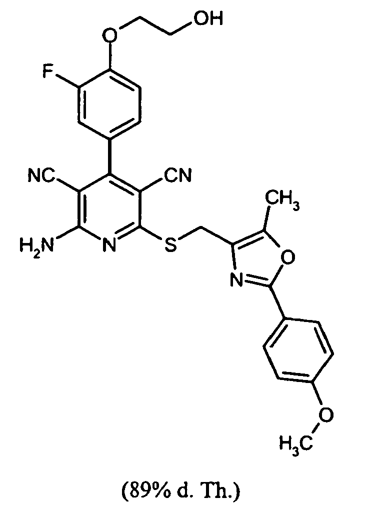 | 2.61 min (3); m/z = 532 | 8.24-8.03 (br. s, 2H), 7.86 (d, 2H), 7.51 (dd, 1H), 7.36 (q, 1H), 7.34 (s, 1H), 7.07 (d, 2H), 4.96 (t, 1H), 4.49 (s, 2H), 4.18 (t, 2H), 3.81 (s, 3H), 3.76 (q, 2H), 2.45 (s, 3H). |
| **28** | 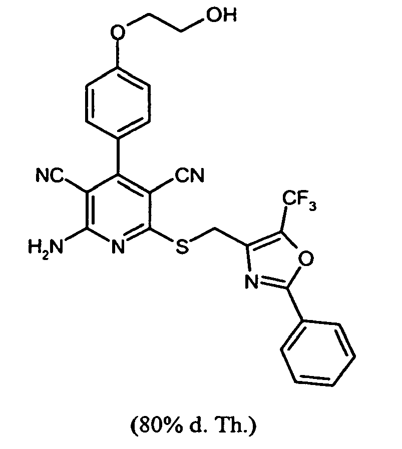 | 2.77 min (3); m/z = 538 | 8.11-7.92 (br. s, 2H), 8.05 (d, 2H), 7.69-7.57 (m, 3H), 7.50 (d, 2H), 7.12 (d, 2H), 4.91 (t, 1H), 4.71 (s, 2H), 4.09 (t, 2H), 3.74 (q, 2H). |
| **29** | 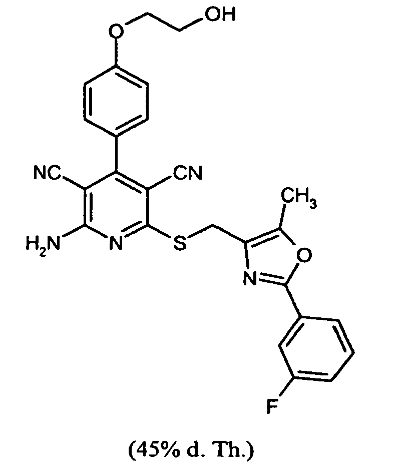 | 2.20 min (6); m/z = 502 | 8.19-7.95 (br. s, 2H), 7.78 (d, 1H), 7.65 (dd, 1H), 7.57 (q, 1H), 7.49 (d, 2H), 7.36 (dt, 1H), 7.10 (d, 2H), 4.91 (t, 1 H), 4.52 (s, 2H), 4.08 (t, 2H), 3.75 (q, 2H), 2.48 (s, 3H). |
| **30** | 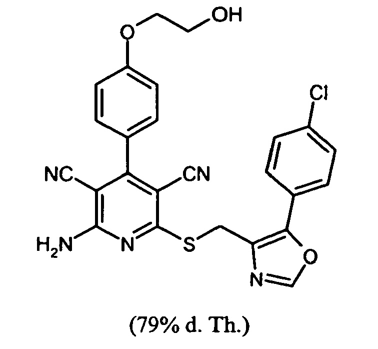 | 1.22 min (14); m/z = 504 | 8.49 (s, 1H), 8.06-7.89 (br. s, 2H), 7.73 (d, 2H), 7.61 (d, 2H), 7.48 (d, 2H), 7.10 (d, 2H), 4.91 (t, 1H), 4.74 (s, 2H), 4.08 (t, 2H), 3.74 (q, 2H). |
| **31** | 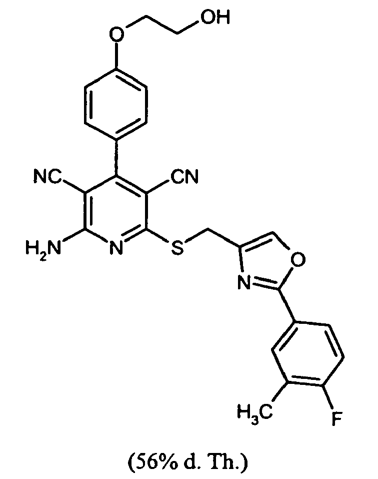 | 2.23 min (6); m/z = 502 | 8.33 (s, 1H), 8.23-7.98 (br. s, 2H), 7.92 (d, 1H), 7.85-7.78 (m, 1H), 7.47 (d, 2H), 7.30 (t, 1H), 7.10 (d, 2H), 4.91 (t, 1H), 4.41 (s, 2H), 4.07 (t, 2H), 3.73 (q, 2H), 2.31 (s, 3H). |
| **32** | 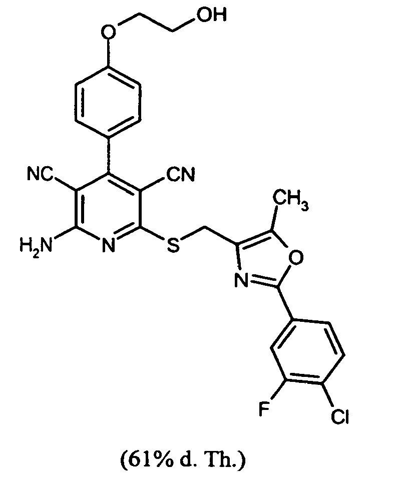 | 2.83 min (3); m/z = 536 | 8.22-7.93 (br. s, 2H), 7.85 (dd, 1H), 7.79-7.71 (m, 2H), 7.48 (d, 2H), 7.12 (d, 2H), 4.91 (t, 1H), 4.52 (s, 2H), 4.07 (t, 2H), 3.73 (q, 2H), 2.48 (s, 3H). |
| **33** | 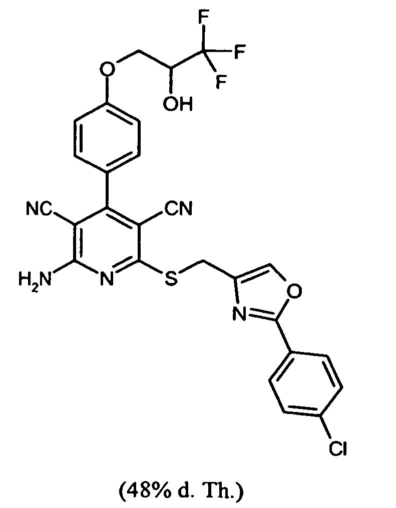 | 2.34 min (7); m/z = 572 | 8.37 (s, 1H), 8.27-7.91 (br. s, 2H), 7.97 (d, 2H), 7.60 (d, 2H), 7.48 (d, 2H), 7.14 (d, 2H), 6.69 (d, 1H), 4.49-4.38 (m, 1H), 4.43 (s, 2H), 4.27 (dd, 1H), 4.17 (dd, 1H). |
| **34** | 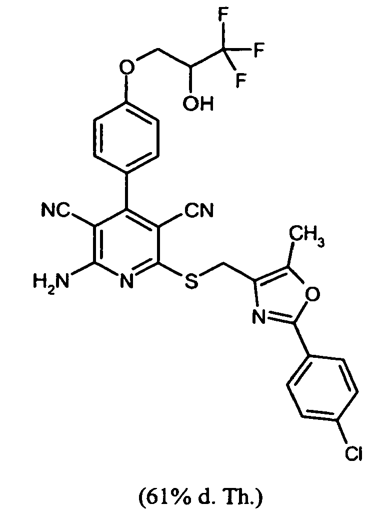 | 2.41 min (7); m/z = 586 | 8.21-7.98 (br. s, 2H), 7.92 (d, 2H), 7.58 (d, 2H), 7.50 (d, 2H), 7.14 (d, 2H), 6.69 (d, 1H), 4.52 (s, 2H), 4.48-4.37 (m, 1H), 4.28 (dd, 1H), 4.16 (dd, 1H), 2.48 (s, 3H). |
| **35** | 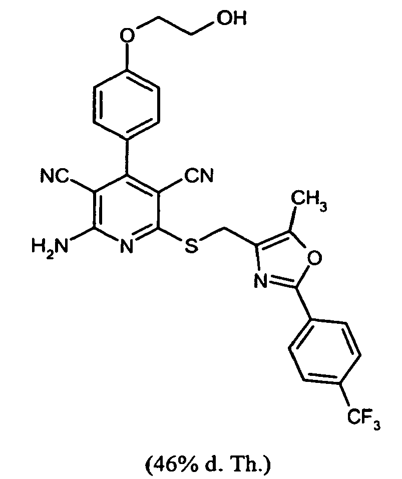 | 2.20 min (7); m/z = 552 | 8.17-7.95 (br. s, 2H), 8.11 (d, 2H), 7.88 (d, 2H), 7.48 (d, 2H), 7.10 (d, 2H), 4.90 (t, 1H), 4.53 (s, 2H), 4.08 (t, 2H), 3.75 (q, 2H), 2.50 (s, 3H). |
| **36** | 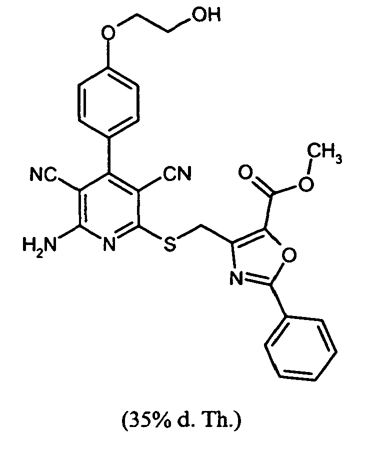 | 2.60 min (3); m/z = 528 | 8.11-7.91 (br. s, 2H), 8.05 (d, 2H), 7.67-7.06 (m, 3H), 7.50 (d, 2H), 7.11 (d, 2H), 4.92 (t, 1H), 4.83 (s, 2H), 4.09 (t, 2H), 3.93 (s, 3H), 3.76 (q, 2H). |
| **37** | 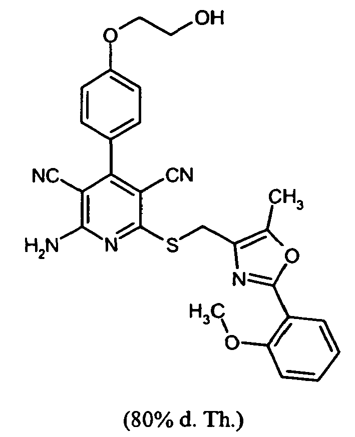 | 1.16 min (14); m/z = 514 | 8.17-7.96 (br. s, 2H), 7.76 (dd, 1H), 7.52-7.46 (m, 1H), 7.50 (d, 2H), 7.19 (d, 1H), 7.11 (d, 2H), 7.06 (t, 1H), 4.91 (t, 1H), 4.52 (s, 2H), 4.08 (t, 2H), 3.85 (s, 3H), 3.75 (q, 2H), 2.45 (s, 3H). |
| **38** | 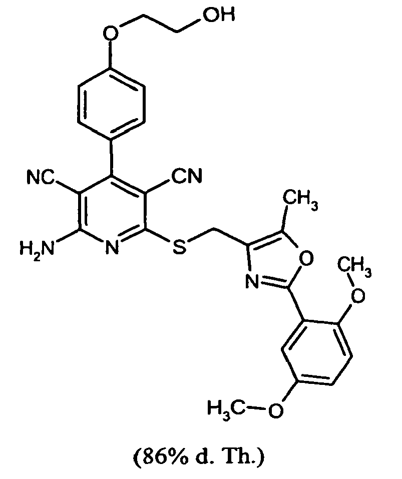 | 1.16 min (14); m/z = 544 | 8.13-7.99 (br.s, 2H), 7.49 (d, 2H), 7.29 (d, 1H), 7.15-7.08 (m, 3H), 7.06 (dd, 1H), 4.91 (t, 1H), 4.51 (s, 2H), 4.08 (t, 2H), 3.80 (s, 3H), 3.77-3.71 (m, 2H), 3.76 (s, 3H), 2.43 (s, 3H). |
| **39** | 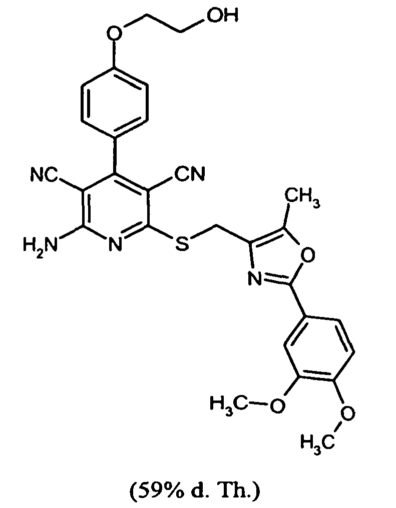 | 2.29 min (3); m/z = 544 | 8.15-7.96 (br. s, 2H), 7.49 (d, 2H), 7.48 (d, 1H), 7.41 (s, 1H), 7.11 (d, 2H), 7.07 (d, 1H), 4.91 (t, 1H), 4.50 (s, 2H), 4.08 (t, 2H), 3.84 (s, 3H), 3.83 (s, 3H), 3.74 (q, 2H), 2.45 (s, 3H). |
| **40** | 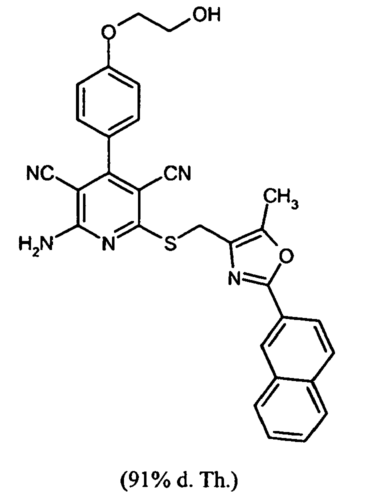 | 1.35 min (14); m/z = 534 | 8.53 (s, 1H), 8.22-7.94 (br. s, 2H), 8.15-8.08 (m, 1H), 8.04 (s, 2H), 8.01-7.95 (m, 1H), 7.65-7.58 (m, 2H), 7.50 (d, 2H), 7.11 (d, 2H), 4.92 (t, 1H), 4.57 (s, 2H), 4.08 (t, 2H), 3.73 (q, 2H), 2.49 (s, 3H). |
| **41** | 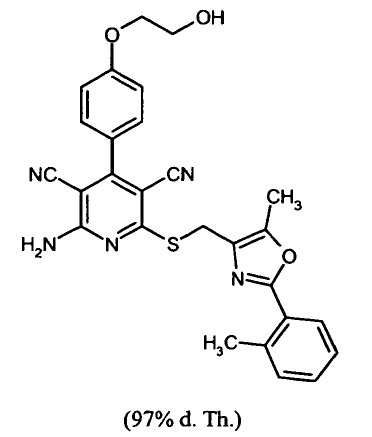 | 1.30 min (14); m/z = 498 | 8.17-7.93 (br. s, 2H), 7.87 (d, 1H), 7.50 (d, 2H), 7.41-7.30 (m, 3H), 7.11 (d, 2H), 4.91 (t, 1H), 4.53 (s, 2H), 4.08 (t, 2H), 3.74 (q, 2H), 2.60 (s, 3H), 2.48 (s, 3H). |
| **42** | 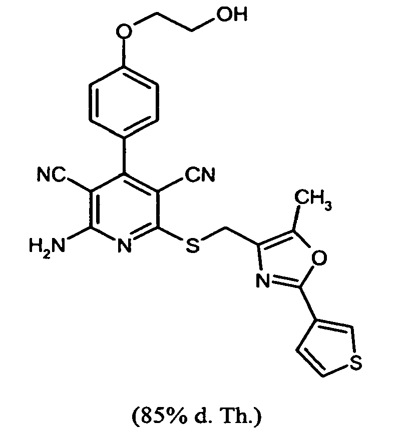 | 2.17 min (15); m/z = 490 | 8.17-7.94 (br. s, 2H), 8.11 (d, 1H), 7.71 (dd, 1H), 7.52 (d, 1H), 7.49 (d, 2H), 7.10 (d, 2H), 4.91 (t, 1H), 4.49 (s, 2H), 4.08 (t, 2H), 3.74 (q, 2H), 2.45 (s, 3H). |
| **43** | 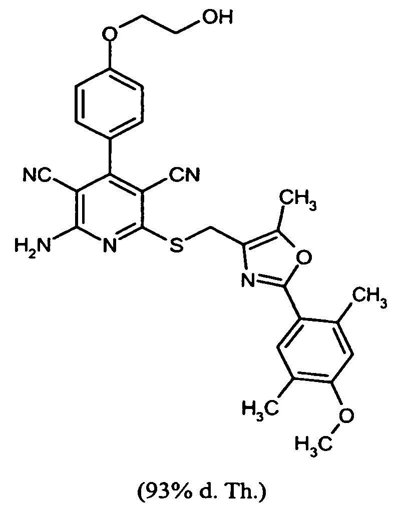 | 2.21 min (7); m/z = 542 | 8.17-7.95 (br. s, 2H), 7.63 (s, 1H), 7.49 (d, 2H), 7.11 (d, 2H), 6.91 (s, 1H), 4.90 (t, 1H), 4.50 (s, 2H), 4.08 (t, 2H), 3.83 (s, 3H), 3.73 (q, 2H), 2.58 (s, 3H), 2.44 (s, 3H), 2.16 (s, 3H). |

### Beispiel 44

(+)-2-Amino-6-({[2-(4-chlorphenyl)-1,3-oxazol-4-yl]methyl}sulfanyl)-4-[4-(3,3,3-trifluor-2-hydroxypropoxy)phenyl]pyridin-3,5-dicarbonitril

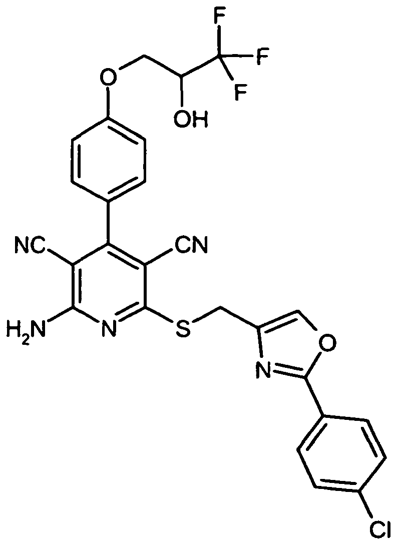

Die racemische Verbindung aus Beispiel 33 (67 mg) wird durch HPLC-Chromatographie an chiraler Phase in die beiden Enantiomere aufgetrennt (*siehe auch Beispiel 45)* [Säule: Daicel Chiralpak AD-H, 5 µm, 250 mm x 20 mm; Eluent: Ethanol; Fluss: 10 ml/min; Temperatur: 35°C; Detektion: 220 nm].

### (+)-Enantiomer:

Ausbeute: 33 mg

Rₜ = 6.86 min [Säule: Daicel Chiralpak AD-H, 5 µm, 250 mm x 4.6 mm; Eluent: Ethanol; Fluss: 1 ml/min; Temperatur: 40°C]

Spezifischer Drehwert: +1.2° (c = 0.43 g/100 ml, Methanol, n = 589 nm, T = 20.4°C)

¹H-NMR (400 MHz, DMSO-d₆): δ = 8.37 (s, 1H), 8.28-7.91 (br. s, 2H), 7.98 (d, 2H), 7.61 (d, 2H), 7.49 (d, 2H), 7.15 (d, 2H), 6.70 (d, 1H), 4.49-4.39 (m, 1H), 4.43 (s, 2H), 4.28 (dd, 1H), 4.16 (dd, 1H).

LC-MS (Methode 7): Rₜ = 2.34 min; MS (ESIpos): m/z = 572 [M+H]⁺.

### Beispiel 45

(-)-2-Amino-6-({[2-(4-chlorphenyl)-1,3-oxazol-4-yl]methyl}sulfanyl)-4-[4-(3,3,3-trifluor-2-hydroxypropoxy)phenyl]pyridin-3,5-dicarbonitril

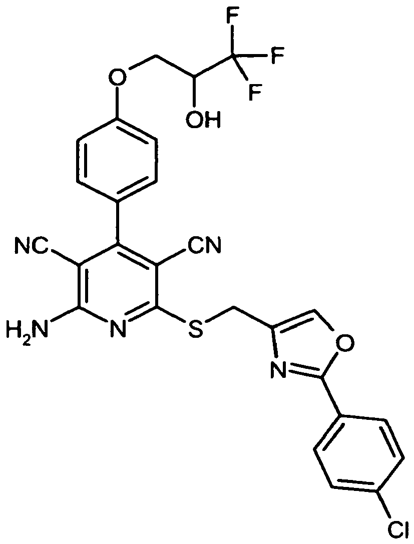

Die racemische Verbindung aus Beispiel 33 (67 mg) wird durch HPLC-Chromatographie an chiraler Phase in die beiden Enantiomere aufgetrennt (*siehe auch Beispiel 44*) [Säule: Daicel Chiralpak AD-H, 5 µm, 250 mm x 20 mm; Eluent: Ethanol; Fluss: 10 ml/min; Temperatur: 35°C; Detektion: 220 nm].

### (-)-Enantiomer:

Ausbeute: 33 mg

Rₜ = 8.73 min [Säule: Daicel Chiralpak AD-H, 5 µm, 250 mm x 4.6 mm; Eluent: Ethanol; Fluss: 1 ml/min; Temperatur: 40°C]

¹H-NMR (400 MHz, DMSO-d₆): δ = 8.37 (s, 1H), 8.29-7.90 (br. s, 2H), 7.98 (d, 2H), 7.61 (d, 2H), 7.49 (d, 2H), 7.16 (d, 2H), 6.70 (d, 1H), 4.49-4.39 (m, 1H), 4.43 (s, 2H), 4.28 (dd, 1H), 4.16 (dd, 1H).

LC-MS (Methode 7): Rₜ = 2.35 min; MS (ESIpos): m/z = 572 [M+H]⁺.

### Beispiel 46

(+)-2-Amino-6-({[2-(4-chlorphenyl)-5-methyl-1,3-oxazol-4-yl]methyl}sulfanyl)-4-[4-(3,3,3-trifluor-2-hydroxypropoxy)phenyl]pyridin-3,5-dicarbonitril

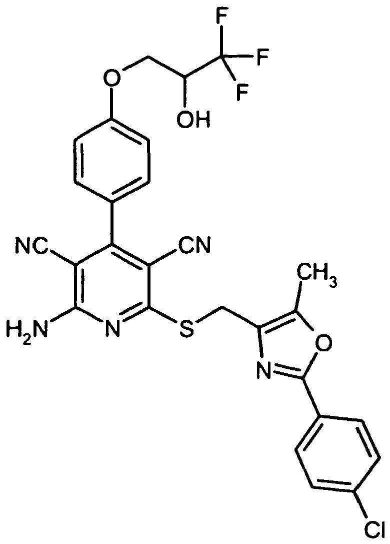

Die racemische Verbindung aus Beispiel 34 (87 mg) wird durch HPLC-Chromatographie an chiraler Phase in die beiden Enantiomere aufgetrennt (*siehe auch Beispiel 47*) [Säule: Daicel Chiralpak AD-H, 5 µm, 250 mm x 20 mm; Eluent: Isohexan/Isopropanol 1:1 (v/v); Fluss: 15 ml/ min; Temperatur: 40°C; Detektion: 220 nm].

### (+)-Enantiomer:

Ausbeute: 30 mg

Rₜ = 4.58 min [Säule: Daicel Chiralpak AD-H, 5 µm, 250 mm x 4.6 mm; Eluent: Isohexan/Isopropanol 1:1 (v/v); Fluss: 1 ml/min; Temperatur: 40°C]

Spezifischer Drehwert: +11.1° (c = 0.435 g/100 ml, DMF, n = 589 nm, T = 19.6°C)

¹H-NMR (400 MHz, DMSO-d₆): δ = 8.20-7.88 (br. s, 2H), 7.92 (d, 2H), 7.58 (d, 2H), 7.49 (d, 2H), 7.15 (d, 2H), 6.70 (d, 1H), 4.52 (s, 2H), 4.48-4.37 (m, 1H), 4.28 (dd, 1H), 4.17 (dd, 1H), 2.48 (s, 3H).

LC-MS (Methode 7): Rₜ = 2.43 min; MS (ESIpos): m/z = 586 [M+H]⁺.

### Beispiel 47

(-)-2-Amino-6-({[2-(4-chlorphenyl)-5-methyl-1,3-oxazol-4-yl]methyl}sulfanyl)-4-[4-(3,3,3-trifluor-2-hydroxypropoxy)phenyl]pyridin-3,5-dicarbonitril

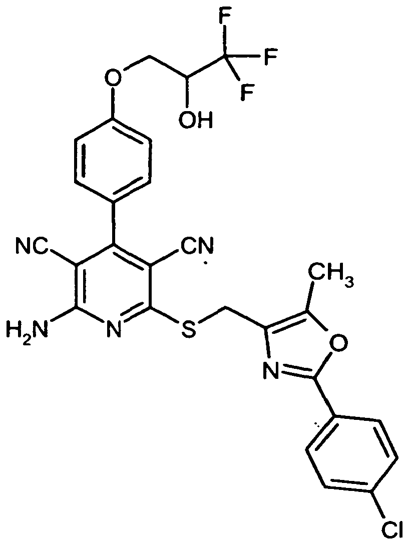

Die racemische Verbindung aus Beispiel 34 (87 mg) wird durch HPLC-Chromatographie an chiraler Phase in die beiden Enantiomere aufgetrennt *(siehe auch Beispiel 46)* [Säule: Daicel Chiralpak AD-H, 5 µm, 250 mm x 20 mm; Eluent: Isohexan/Isopropanol 1:1 (v/v); Fluss: 15 ml/ min; Temperatur: 40°C; Detektion: 220 nm].

### (-)-Enantiomer:

Ausbeute: 31 mg

Rₜ = 5.56 min [Säule: Daicel Chiralpak AD-H, 5 µm, 250 mm x 4.6 mm; Eluent: Isohexan/Isopropanol 1:1 (v/v); Fluss: 1 ml/min; Temperatur: 40°C]

¹H-NMR (400 MHz, DMSO-d₆): δ = 8.21-7.89 (br. s, 2H), 7.92 (d, 2H), 7.58 (d, 2H), 7.50 (d, 2H), 7.16 (d, 2H), 6.70 (d, 1H), 4.52 (s, 2H), 4.48-4.39 (m, 1H), 4.29 (dd, 1H), 4.17 (dd, 1H), 2.48 (s, 3H).

LC-MS (Methode 7): Rₜ = 2.43 min; MS (ESIpos): m/z = 586 [M+H]⁺.

### Beispiel 48

2-Amino-6-({[2-(4-chlorphenyl)-1,3-oxazol-4-yl]methyl}sulfanyl)-4-(4-{[(2S)-2,3-dihydroxypropyl]oxy}phenyl)pyridin-3,5-dicarbonitril 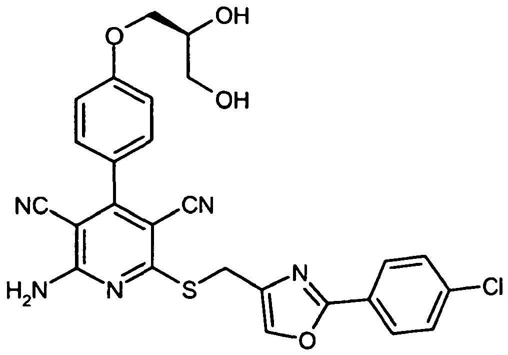 400 mg (0.70 mmol) der Verbindung aus Beispiel 46A werden in 17 ml Essigsäure vorgelegt und anschließend vorsichtig mit 8.6 ml Wasser versetzt. Es wird 12 h bei RT gerührt. Nach Einengen des Reaktionsgemisches am Rotationsverdampfer wird der Rückstand direkt mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 340 mg (91% d. Th.)

¹H-NMR (400 MHz, DMSO-d₆): δ = 8.37 (s, 1H), 8.27-7.91 (br. s, 2H), 7.98 (d, 2H), 7.60 (d, 2H), 7.47 (d, 2H), 7.10 (d, 2H), 5.00 (d, 1H), 4.70 (t, 1H), 4.42 (s, 2H), 4.09 (dd, 1H), 3.96 (dd, 1H), 3.70 (q, 1H), 3.46 (t, 2H).

LC-MS (Methode 3): Rₜ = 2.48 min; MS (ESIpos): m/z = 534 [M+H]⁺.

### Beispiel 49

2-Amino-6-({[2-(4-chlorphenyl)-1,3-oxazol-4-yl]methyl}sulfanyl)-4-(4-{[(2*R*)-2,3-dihydroxypropyl]oxy}phenyl)pyridin-3,5-dicarbonitril 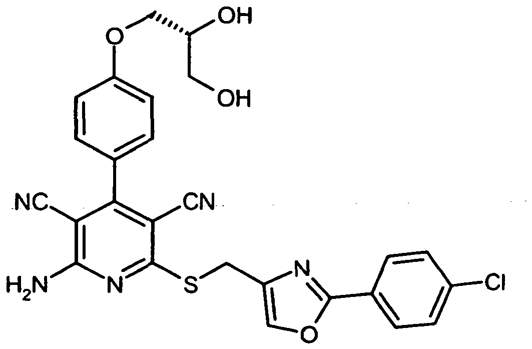 403 mg (80% Reinheit, 0.56 mmol) der Verbindung aus Beispiel 47A werden in 23.5 ml Essigsäure vorgelegt und anschließend vorsichtig mit 23.5 ml Wasser versetzt. Das Reaktionsgemisch wird über Nacht bei RT gerührt und dann am Rotationsverdampfer eingeengt. Der Rückstand wird in wenig DMF aufgenommen und mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 259 mg (86% d. Th.)

¹H-NMR (400 MHz, DMSO-d₆): δ = 8.37 (s, 1H), 8.30-7.89 (br. s, 2H), 7.98 (d, 2H), 7.61 (d, 2H), 7.48 (d, 2H), 7.10 (d, 2H), 5.00 (d, 1H), 4.70 (t, 1H), 4.42 (s, 2H), 4.09 (dd, 1H), 3.98-3.92 (m, 1H), 3.81 (q, 1H), 3.50-3.43 (m, 2H).

LC-MS (Methode 3): Rₜ = 2.51 min; MS (ESIpos): m/z = 534 [M+H]⁺.

Die in Tabelle 9 aufgeführten Beispiele werden aus den entsprechenden Ausgangsverbindungen analog zu den Beispielen 48 und 49 hergestellt:

**Tabelle 9**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ**=** |
|---|---|---|---|
| **50** | 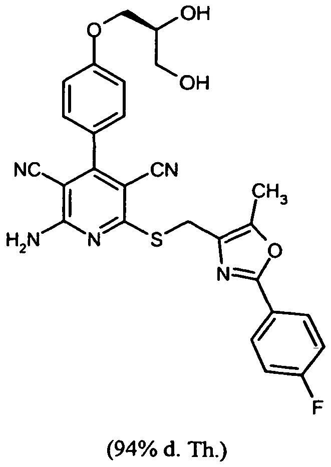 | 2.43 min (3); m/z = 532 | 8.20-7.92 (br. s, 2H), 7.97 (d, 1H), 7.96 (d, 1H), 7.48 (d, 2H), 7.35 (pseudo-t, 2H), 7.10 (d, 2H), 5.00 (d, 1H), 4.69 (t, 1H), 4.50 (s, 2H), 4.09 (dd, 1H), 3.96 (dd, 1H), 3.82 (q, 1H), 3.47 (t, 2H), 2.43 (s, 3H). |
| **51** | 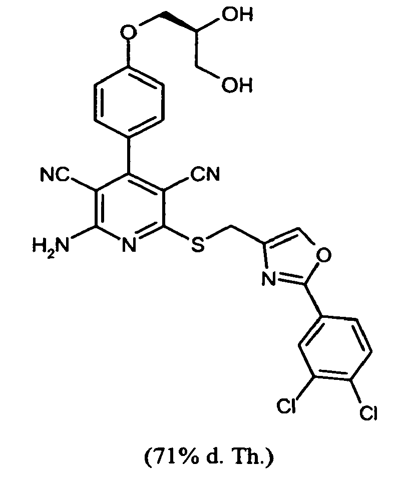 | 2.65 min (3); m/z = 568 | 8.41 (s, 1H), 8.32-7.97 (br. s, 2H), 8.12 (d, 1H), 7.92 (dd, 1H), 7.70 (d, 1H), 7.47 (d, 2H), 7.10 (d, 2H), 5.00 (d, 1H), 4.69 (t, 1H), 4.42 (s, 2H), 4.09 (dd, 1H), 3.94 (dd, 1H), 3.86-3.77 (m, 1H), 3.47 (t, 2H). |
| **52** | 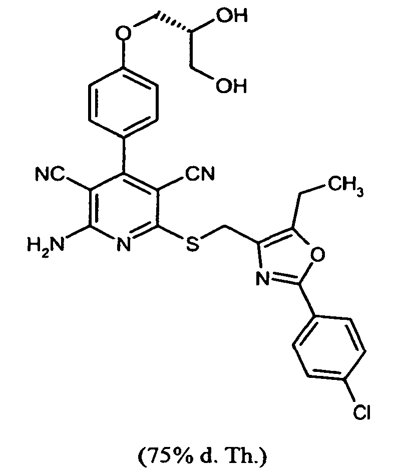 | 2.69 min (3); m/z = 562 | 8.24-7.89 (br. s, 2H), 7.95 (d, 2H), 7.59 (d, 2H), 7.48 (d, 2H), 7.10 (d, 2H), 5.01 (d, 1H), 4.70 (t, 1H), 4.51 (s, 2H), 4.09 (dd, 1H), 3.96 (dd, 1H), 3.86-3.78 (m, 1H), 3.48 (t, 2H), 2.89 (q, 2H), 1.21 (t, 3H). |
| **53** | 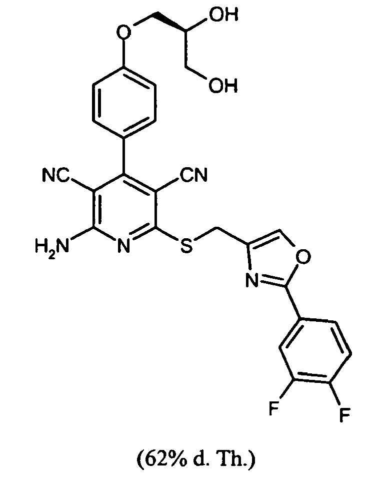 | 2.43 min (3); m/z = 536 | 8.39 (s, 1H), 8.32-7.91 (br. s, 2H), 7.97 (dd, 1H), 7.86-7.80 (m, 1H), 7.63 (q, 1H), 7.46 (d, 2H), 7.10 (d, 2H), 5.05-4.58 (2 br. s, 2H), 4.41 (s, 2H), 4.09 (dd, 1H), 3.95 (dd, 1H), 3.84-3.79 (m, 1H), 3.46 (d, 2H). |
| **54** | 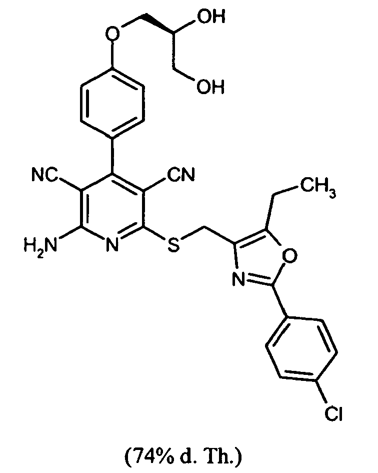 | 2.68 min (3); m/z = 562 | 8.21-7.88 (br. s, 2H), 7.94 (d, 2H), 7.58 (d, 2H), 7.47 (d, 2H), 7.09 (d, 2H), 5.00 (d, 1H), 4.70 (t, 1H), 4.52 (s, 2H), 4.08 (dd, 1H), 3.98-3.92 (m, 1H), 3.86-3.77 (m, 1H), 3.47 (t, 2H), 2.88 (q, 2H), 1.19 (t, 3H). |
| **55** | 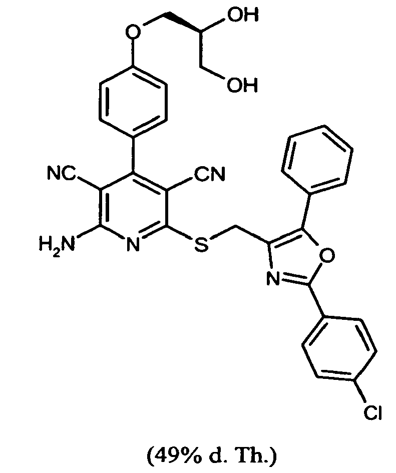 | 2.48 min (6); m/z = 610 [M]⁺ | 8.10 (d, 2H), 7.99-7.87 (br. s, 2H), 7.85 (d, 2H), 7.64 (d, 2H), 7.61-7.51 (m, 2H), 7.50-7.44 (m, 3H), 7.11 (d, 2H), 5.01 (d, 1H), 4.81 (s, 2H), 4.69 (t, 1H), 4.09 (dd, 1H), 3.96 (dd, 1H), 3.87-3.78 (m, 1H), 3.47 (t, 2H). |
| **56** | 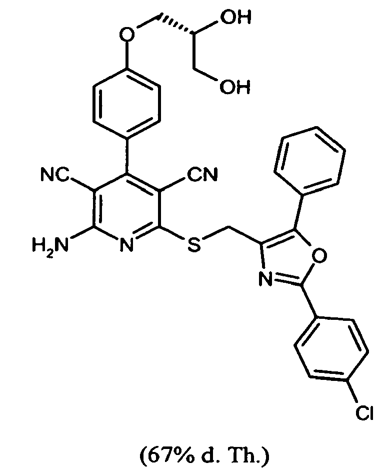 | 2.48 min (6); m/z = 610 [M]⁺ | 8.10 (d, 2H), 7.98-7.87 (br. s, 2H), 7.86 (d, 2H), 7.64 (d, 2H), 7.61-7.53 (m, 2H), 7.51-7.44 (m, 3H), 7.11 (d, 2H), 5.01 (d, 1H), 4.81 (s, 2H), 4.69 (t, 1H), 4.09 (dd, 1H), 3.96 (dd, 1H), 3.87-3.78 (m, 1H), 3.48 (t, 2H). |
| **57** | 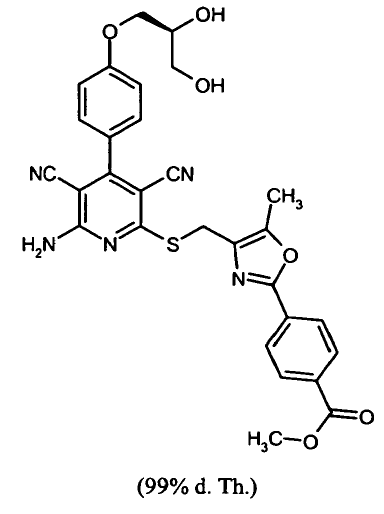 | 3.26 min (5); m/z = 572 | 8.17-7.98 (m, 6H), 7.49 (d, 2H), 7.10 (d, 2H), 5.00 (d, 1H), 4.70 (t, 1H), 4.53 (s, 2H), 4.08 (dd, 1H), 3.99-3.92 (m, 1H), 3.88 (s, 3H), 3.85-3.79 (m, 1H), 3.46 (t, 2H), 2.49 (s, 3H). |
| **58** | 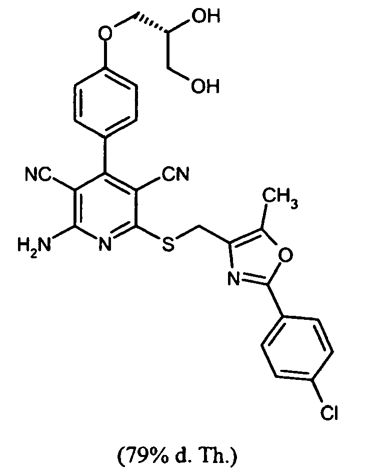 | 2.58 min (3); m/z = 548 | 8.24-7.89 (br. s, 2H), 7.92 (d, 2H), 7.58 (d, 2H), 7.49 (d, 2H), 7.10 (d, 2H), 5.00 (d, 1H), 4.69 (t, 1H), 4.51 (s, 2H), 4.08 (dd, 1H), 3.95 (dd, 1H), 3. 81 (q, 1H), 3.47 (t, 2H), 2.48 (s, 3H). |
| **59** | 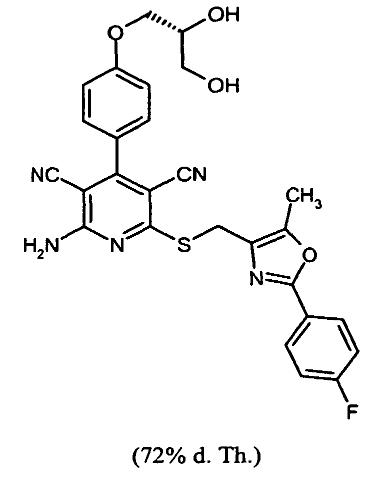 | 2.42 min (3); m/z = 532 | 8.19-7.91 (br. s, 2H), 7.98 (d, 1H), 7.97 (d, 1H), 7.49 (d, 2H), 7.36 (pseudo-t, 2H), 7.10 (d, 2H), 4.50 (s, 2H), 4.10 (dd, 1H), 3.97 (dd, 1H), 3.81 (q, 1H), 3.71-3.26 (br. s, 2H), 3.47 (t, 2H), 2.47 (s, 3H). |
| **60** | 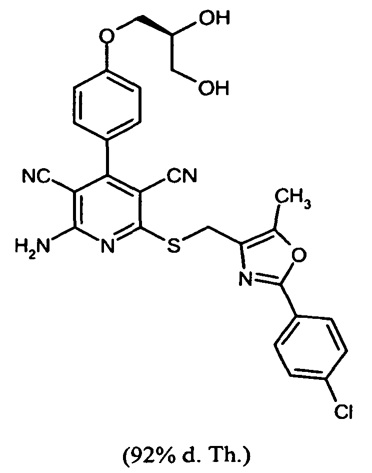 | 2.58 min (3); m/z = 548 | 8.27-7.96 (br. s, 2H), 7.93 (d, 2H), 7.58 (d, 2H), 7.49 (d, 2H), 7.10 (d, 2H), 5.00 (d, 1H), 4.70 (t, 1H), 4.51 (s, 2H), 4.09 (dd, 1H), 3.96 (dd, 1H), 3.84-3.78 (m, 1H), 3.47 (t, 2H), 2.49 (s, 3H). |
| **61** | 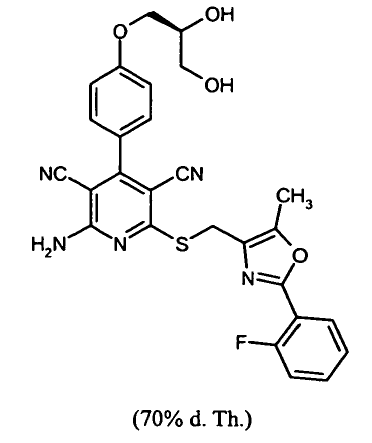 | 1.79 min (7); m/z = 532 | 8.20-7.92 (br. s, 2H), 7.97 (dt, 1H), 7.60-7.53 (m, 1H), 7.48 (d, 2H), 7.43-7.31 (m, 2H), 7.09 (d, 2H), 5.00 (d, 1H), 4.70 (t, 1H), 4.53 (s, 2H), 4.09 (dd, 1H), 3.95 (dd, 1H), 3.72 (q, 1H), 3.47 (t, 2H), 2.47 (s, 3H). |
| **62** | 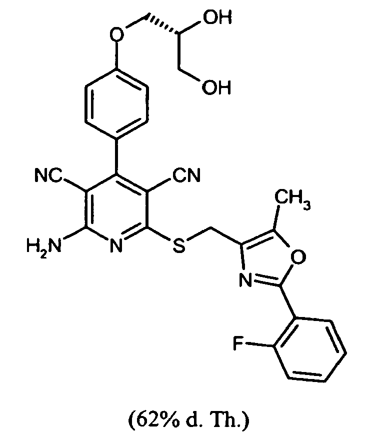 | 1.79 min (7); m/z = 532 | 8.19-7.90 (br. s, 2H), 7.97 (dt, 1H), 7.60-7.53 (m, 1H), 7.49 (d, 2H), 7.43-7.32 (m, 2H), 7.10 (d, 2H), 5.00 (d, 1H), 4.69 (t, 1H), 4.52 (s, 2H), 4.09 (dd, 1H), 3.95 (dd, 1H), 3.71 (q, 1H), 3.47 (t, 2H), 2.47 (s, 3H). |
| **63** | 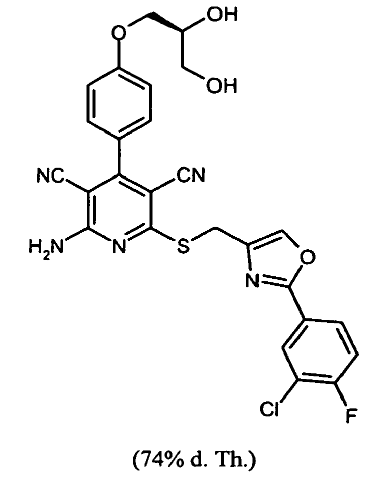 | 2.54 min (3); m/z = 552 | 8.39 (s, 1H), 8.31-7.89 (br. s, 2H), 8.10 (dd, 1H), 8.00-7.94 (m, 1H), 7.60 (pseudo-t, 1H), 7.47 (d, 2H), 7.09 (d, 2H), 5.00 (d, 1H), 4.70 (t, 1H), 4.41 (s, 2H), 4.08 (dd, 1H), 3.95 (dd, 1H), 3.81 (q, 1H), 3.46 (t, 2H). |
| **64** | 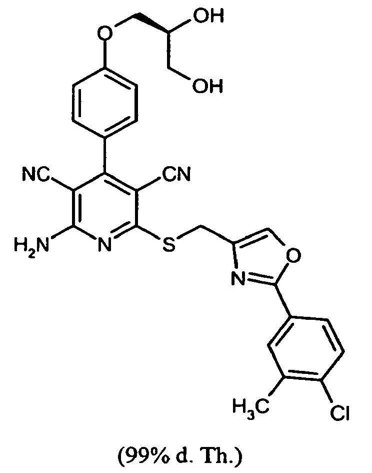 | 2.64 min (3); m/z = 548 | 8.36 (s, 1H), 8.30-7.90 (br. s, 2H), 7.97 (s, 1H), 7.79 (dd, 1H), 7.57 (d, 1H), 7.46 (d, 2H), 7.09 (d, 2H), 5.00 (d, 1H), 4.69 (t, 1H), 4.42 (s, 2H), 4.09 (dd, 1H), 3.95 (dd, 1H), 3.86-3.77 (m, 1H), 3.47 (t, 2H), 2.41 (s, 3H). |
| **65** | 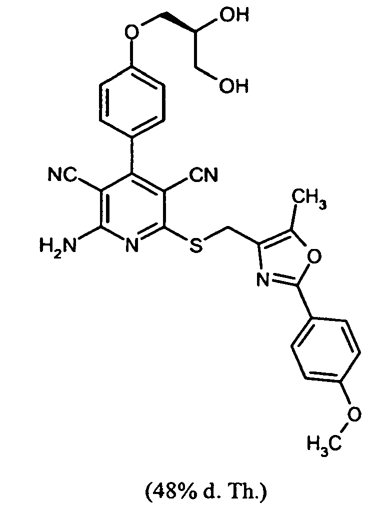 | 2.39 min (3); m/z = 544 | 8.21-7.91 (br. s, 2H), 7.86 (d, 2H), 7.48 (d, 2H), 7.10 (d, 2H), 7.05 (d, 2H), 5.00 (d, 1H), 4.70 (t, 1H), 4.49 (s, 2H), 4.09 (dd, 1H), 3.95 (dd, 1H), 3.85-3.79 (m, 1H), 3.81 (s, 3H), 3.48 (t, 2H), 2.43 (s, 3H). |
| **66** | 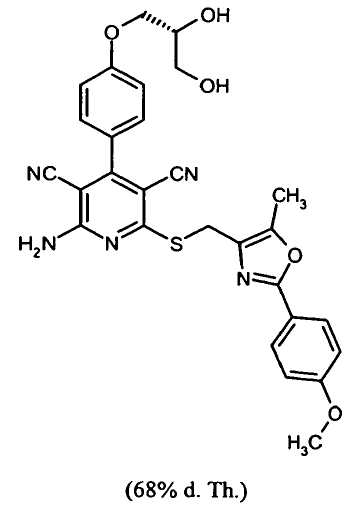 | 2.39 min (3); m/z = 544 | 8.18-7.94 (br. s, 2H), 7.86 (d, 2H), 7.49 (d, 2H), 7.10 (d, 2H), 7.07 (d, 2H), 5.00 (d, 1H), 4.70 (t, 1H), 4.49 (s, 2H), 4.09 (dd, 1H), 3.95 (dd, 1H), 3.85-3.79 (m, 1H), 3.83 (s, 3H), 3.47 (t, 2H), 2.45 (s, 3H). |
| **67** | 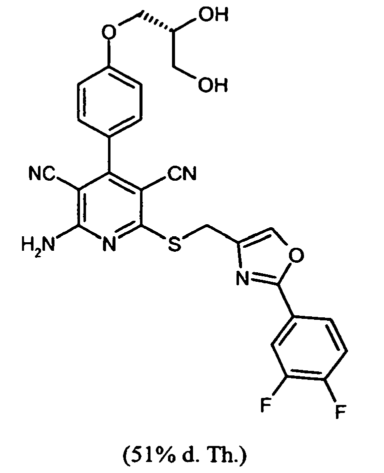 | 2.02 min (6); m/z = 536 | 8.39 (s, 1H), 8.34-7.91 (br. s, 2H), 7.96 (dd, 1H), 7.86-7.80 (m, 1H), 7.63 (q, 1H), 7.47 (d, 2H), 7.09 (d, 2H), 5.00 (s, 1H), 4.70 (s, 1H), 4.41 (s, 2H), 4.09 (dd, 1H), 3.95 (dd, 1H), 3.84-3.78 (m, 1H), 3.45 (d, 2H). |
| **68** | 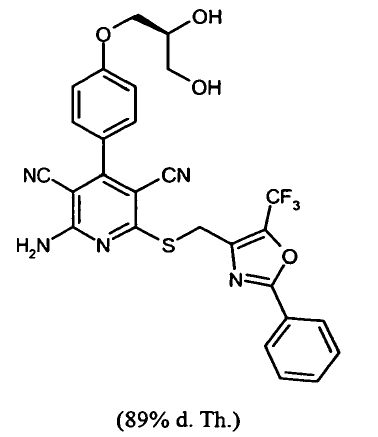 | 2.22 min (6); m/z = 568 | 8.09-7.93 (br. s, 2H), 8.04 (d, 2H), 7.68-7.57 (m, 3H), 7.50 (d, 2H), 7.11 (d, 2H), 5.00 (d, 1H), 4.71 (s, 2H), 4.70 (t, 1H), 4.09 (dd, 1H), 3.97 (dd, 1H), 3.87-3.79 (m, 1H), 3.47 (t, 2H). |
| **69** | 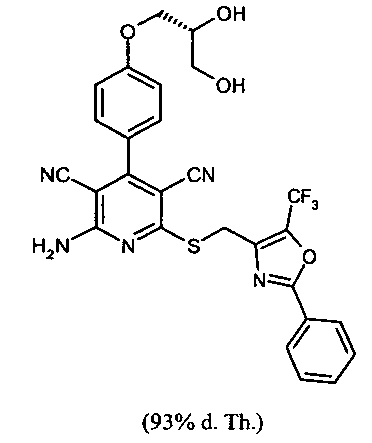 | 2.22 min (6); m/z = 568 | 8.09-7.90 (br. s, 2H), 8.03 (d, 2H), 7.68-7.56 (m, 3H), 7.49 (d, 2H), 7.11 (d, 2H), 5.00 (d, 1H), 4.71 (s, 2H), 4.70 (t, 1H), 4.09 (dd, 1H), 3.97 (dd, 1H), 3.86-3.79 (m, 1H), 3.48 (t, 2H). |
| **70** | 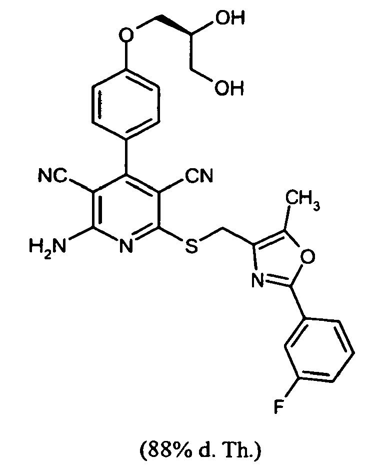 | 2.05 min (6); m/z = 532 | 8.20-7.90 (br. s, 2H), 7.77 (d, 1H), 7.66 (dd, 1H), 7.58 (q, 1H), 7.49 (d, 2H), 7.38 (dt, 1H), 7.11 (d, 2H), 5.01 (d, 1H), 4.70 (t, 1H), 4.52 (s, 2H), 4.10 (dd, 1H), 3.96 (dd, 1H), 3.85-3.79 (m, 1H), 3.47 (t, 2H), 2.48 (s, 3H). |
| **71** | 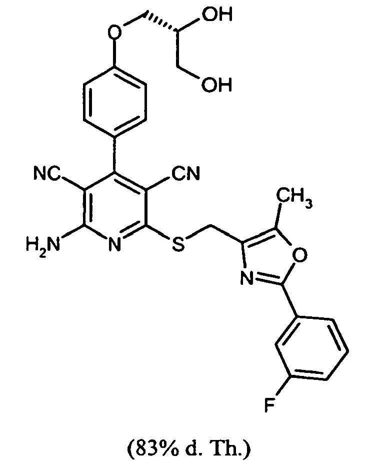 | 2.05 min (6); m/z = 532 | 8.19-7.92 (br. s, 2H), 7.77 (d, 1H), 7.65 (dd, 1H), 7.57 (q, 1H), 7.48 (d, 2H), 7.37 (dt, 1H), 7.10 (d, 2H), 5.00 (d, 1H), 4.70 (t, 1H), 4.52 (s, 2H), 4.09 (dd, 1H), 3.95 (dd, 1H), 3.86-3.78 (m, 1H), 3.47 (t, 2H), 2.48 (s, 3H). |
| **72** | 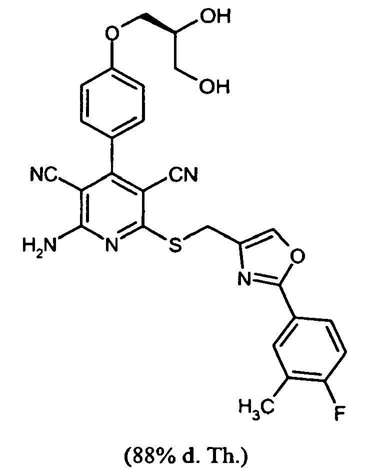 | 2.49 min (3); m/z = 532 | 8.32 (s, 1H), 8.26-7.96 (br. s, 2H), 7.91 (d, 1H), 7.85-7.79 (m, 1H), 7.47 (d, 2H), 7.30 (t, 1H), 7.10 (d, 2H), 5.01 (d, 1H), 4.70 (t, 1H), 4.41 (s, 2H), 4.08 (dd, 1H), 3.95 (dd, 1H), 3.85-3.77 (m, 1H), 3.46 (t, 2H), 2.32 (s, 3H). |
| **73** | 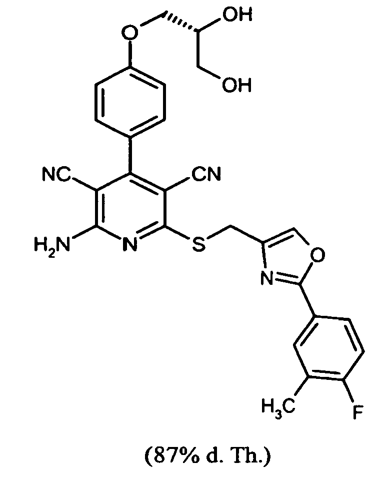 | 2.50 min (3); m/z = 532 | 8.32 (s, 1H), 8.27-7.97 (br. s, 2H), 7.91 (dd, 1H), 7.84-7.79 (m, 1H), 7.47 (d, 2H), 7.30 (t, 1H), 7.10 (d, 2H), 5.00 (d, 1H), 4.71 (t, 1H), 4.41 (s, 2H), 4.09 (dd, 1H), 3.95 (dd, 1H), 3.85-3.78 (m, 1H), 3.46 (t, 2H), 2.32 (s, 3H). |
| **74** | 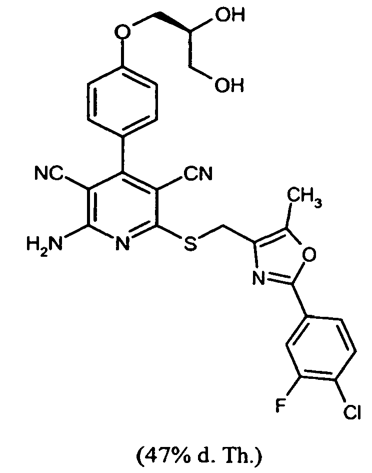 | 2.04 min (7); m/z = 566 | 8.20-7.92 (br. s, 2H), 7.85 (d, 1H), 7.77 (s, 1H), 7.76 (q, 1H), 7.49 (d, 2H), 7.10 (d, 2H), 5.00 (d, 1H), 4.69 (t, 1H), 4.51 (s, 2H), 4.08 (dd, 1H), 3.95 (dd, 1H), 3.86-3.78 (m, 1H), 3.47 (t, 2H), 2.50 (s, 3H). |
| **75** | 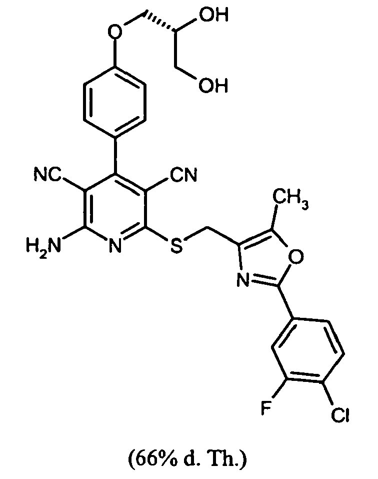 | 2.05 min (7); m/z = 566 | 8.22-7.95 (br. s, 2H), 7.86 (d, 1H), 7.78 (s, 1H), 7.77 (q, 1H), 7.49 (d, 2H), 7.11 (d, 2H), 5.00 (d, 1H), 4.70 (t, 1H), 4.52 (s, 2H), 4.09 (dd, 1H), 3.97 (dd, 1H), 3.86-3.78 (m, 1H), 3.47 (t, 2H), 2.50 (s, 3H). |
| **76** | 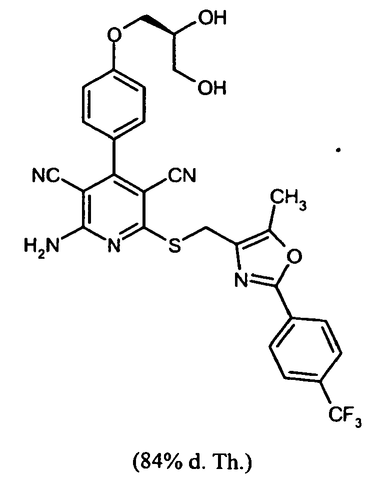 | 2.57 min (3); m/z = 582 | 8.18-7.96 (br. s, 2H), 8.12 (d, 2H), 7.88 (d, 2H), 7.49 (d, 2H), 7.10 (d, 2H), 5.00 (d, 1H), 4.69 (t, 1H), 4.53 (s, 2H), 4.09 (dd, 1H), 3.95 (dd, 1H), 3.85-3.78 (m, 1H), 3.47 (t, 2H), 2.49 (s, 3H). |
| **77** | 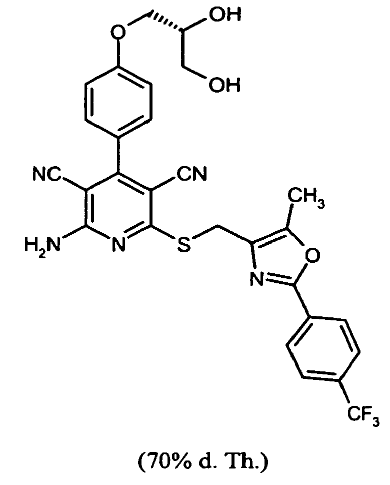 | 2.58 min (3); m/z = 582 | 8.18-7.97 (br. s, 2H), 8.13 (d, 2H), 7.88 (d, 2H), 7.49 (d, 2H), 7.10 (d, 2H), 5.00 (d, 1H), 4.69 (t, 1H), 4.53 (s, 2H), 4.09 (dd, 1H), 3.95 (dd, 1H), 3.85-3.78 (m, 1H), 3.47 (t, 2H), 2.49 (s, 3H). |
| **78** | 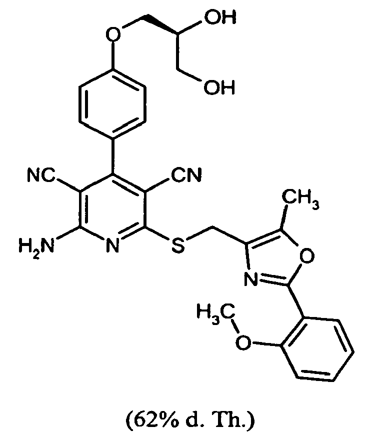 | 1.10 min (14); m/z = 544 | 8.22-7.95 (br. s, 2H), 7.76 (dd, 1H), 7.53-7.45 (m, 1H), 7.49 (d, 2H), 7.20 (d, 1H), 7.10 (d, 2H), 7.05 (t, 1H), 5.02 (d, 1H), 4.71 (t, 1H), 4.51 (s, 2H), 4.09 (dd, 1H), 3.95 (dd, 1H), 3.89-3.78 (m, 1H), 3.86 (s, 3H), 3.50-3.42 (m, 2H), 2.43 (s, 3H). |
| **79** | 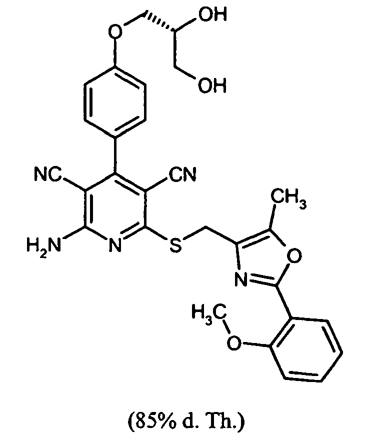 | 1.10 min (14); m/z = 544 | 8.18-7.96 (br. s, 2H), 7.76 (dd, 1H), 7.52-7.44 (m, 1H), 7.49 (d, 2H), 7.19 (d, 1H), 7.10 (d, 2H), 7.05 (t, 1H), 5.02 (d, 1H), 4.71 (t, 1H), 4.51 (s, 2H), 4.09 (dd, 1H), 3.95 (dd, 1H), 3.88-3.78 (m, 1H), 3.86 (s, 3H), 3.46 (t, 2H), 2.43 (s, 3H). |
| **80** | 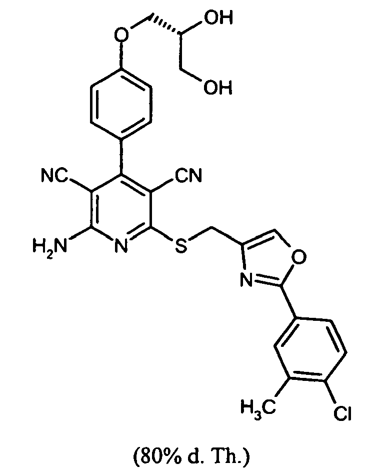 | 2.67 min (3); m/z = 548 | 8.36 (s, 1H), 8.27-7.98 (br. s, 2H), 7.97 (s, 1H), 7.79 (dd, 1H), 7.57 (d, 1H), 7.46 (d, 2H), 7.10 (d, 2H), 5.00 (d, 1H), 4.69 (t, 1H), 4.42 (s, 2H), 4.09 (dd, 1H), 3.96 (dd, 1H), 3.86-3.78 (m, 1H), 3.47 (t, 2H), 2.41 (s, 3H). |
| **81** | 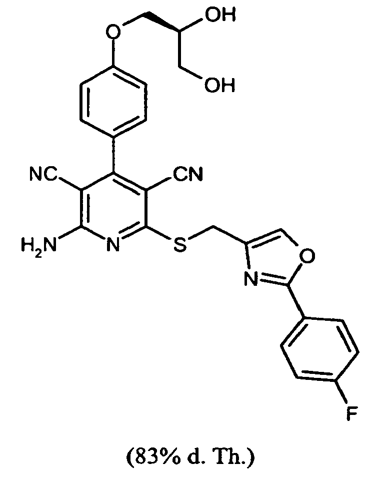 | 2.38 min (3); m/z = 518 | 8.34 (s, 1H), 8.27-7.91 (br. s, 2H), 8.01 (dd, 2H), 7.46 (d, 2H), 7.38 (t, 2H), 7.10 (d, 2H), 5.00 (d, 1H), 4.70 (t, 1H), 4.42 (s, 2H), 4.08 (dd, 1H), 3.96 (dd, 1H), 3.85-3.77 (m, 1H), 3.47 (t, 2H). |
| **82** | 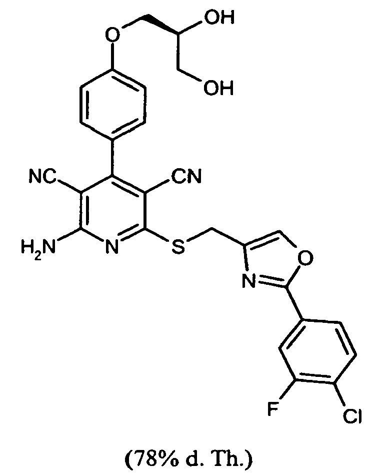 | 2.44 min (3); m/z = 552 | 8.41 (s, 1H), 8.31-7.96 (br. s, 2H), 7.91 (dd, 1H), 7.84-7.73 (m, 2H), 7.46 (d, 2H), 7.09 (d, 2H), 5.00 (d, 1H), 4.69 (t, 1H), 4.52 (s, 2H), 4.08 (dd, 1H), 3.95 (dd, 1H), 3.86-3.78 (m, 1H), 3.48 (t, 2H). |
| **83** | 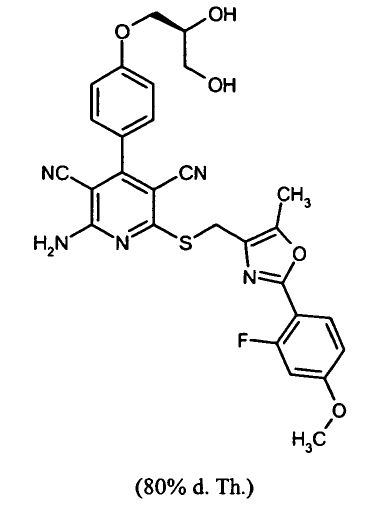 | 2.28 min (3); m/z = 562 | 8.15-7.96 (br. s, 2H), 7.87 (t, 1H), 7.49 (d, 2H), 7.10 (d, 2H), 7.01 (dd, 1H), 6.92 (dd, 1H), 5.00 (d, 1H), 4.69 (t, 1H), 4.51 (s, 2H), 4.09 (dd, 1H), 3.96 (dd, 1H), 3.86-3.78 (m, 1H), 3.84 (s, 3H), 3.47 (t, 2H), 2.45 (s, 3H). |
| **84** | 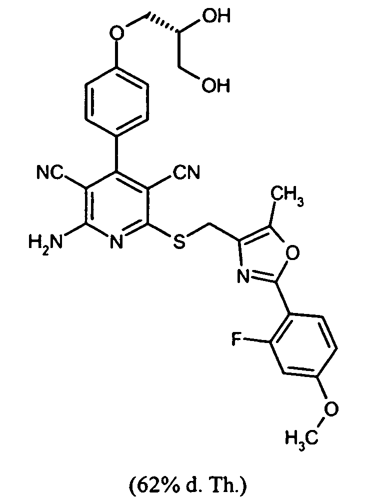 | 2.28 min (3); m/z = 562 | 8.14-7.95 (br. s, 2H), 7.87 (t, 1H), 7.49 (d, 2H), 7.10 (d, 2H), 7.01 (dd, 1H), 6.92 (dd, 1H), 5.00 (d, 1H), 4.70 (t, 1H), 4.51 (s, 2H), 4.09 (dd, 1H), 3.96 (dd, 1H), 3.86-3.78 (m, 1H), 3.84 (s, 3H), 3.47 (t, 2H), 2.45 (s, 3H). |
| **85** | 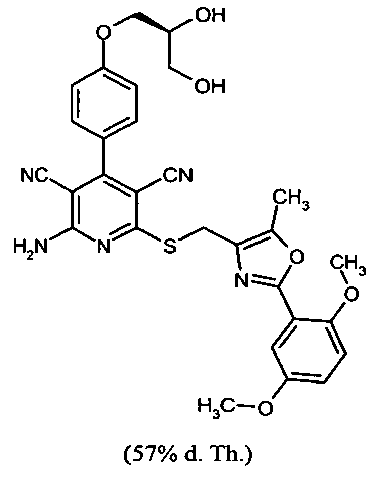 | 2.19 min (3); m/z = 574 | 8.14-7.97 (br. s, 2H), 7.49 (d, 2H), 7.29 (d, 1H), 7.15-7.09 (m, 3H), 7.06 (dd, 1H), 5.00 (d, 1H), 4.69 (t, 1H), 4.51 (d, 2H), 4.09 (dd, 1H), 3.96 (dd, 1H), 3.87-3.79 (m, 1H), 3.81 (s, 3H), 3.76 (s, 3H), 3.48 (t, 2H), 2.44 (s, 3H). |
| **86** | 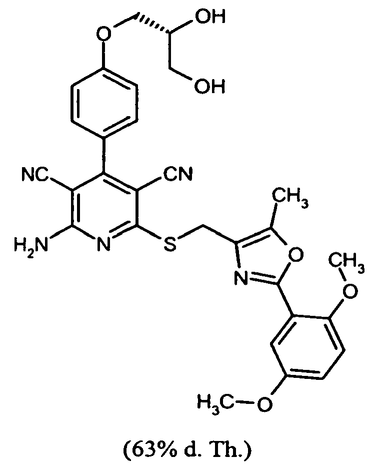 | 2.19 min (3); m/z=574 | 8.13-7.97 (br. s, 2H), 7.49 (d, 2H), 7.29 (d, 1H), 7.15-7.09 (m, 3H), 7.05 (dd, 1H), 5.00 (d, 1H), 4.70 (t, 1H), 4.51 (d, 2H), 4.09 (dd, 1H), 3.96 (dd, 1H), 3.87-3.79 (m, 1H), 3.80 (s, 3H), 3.76 (s, 3H), 3.47 (t, 2H), 2.45 (s, 3H). |
| **87** | 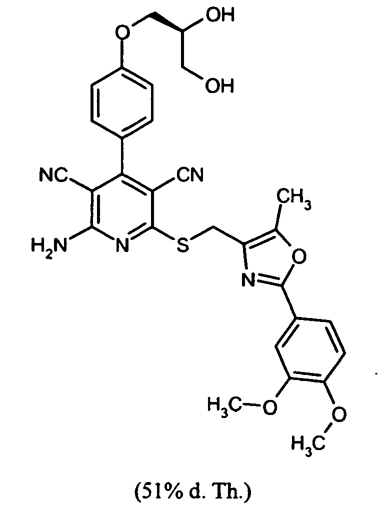 | 1.69 min (7); m/z = 574 | 8.15-7.98 (br. s, 2H), 7.49 (d, 2H), 7.48 (d, 1H), 7.40 (s, 1H), 7.11 (d, 2H), 7.08 (d, 1H), 5.00 (d, 1H), 4.70 (t, 1H), 4.50 (s, 2H), 4.09 (dd, 1H), 3.96 (dd, 1H), 3.85 (s, 3H), 3.83 (s, 3H), 3.83-3.78 (m, 1H), 3.47 (t, 2H), 2.46 (s, 3H). |
| **88** | 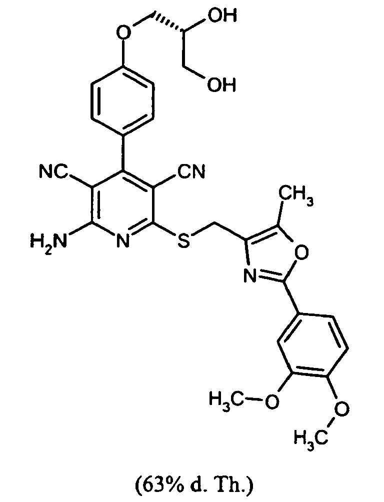 | 1.68 min (7); m/z = 574 | 8.17-7.98 (br. s, 2H), 7.49 (d, 2H), 7.48 (d, 1H), 7.40 (s, 1H), 7.11 (d, 2H), 7.09 (d, 1H), 5.00 (d, 1H), 4.70 (t, 1H), 4.50 (s, 2H), 4.09 (dd, 1H), 3.96 (dd, 1H), 3.84 (s, 3H), 3.82 (s, 3H), 3.82-3.78 (m, 1H), 3.47 (t, 2H), 2.46 (s, 3H). |
| **89** | 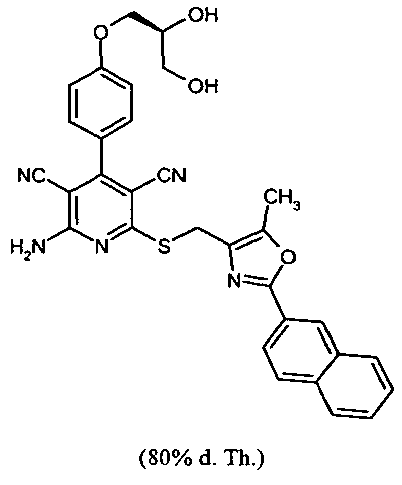 | 2.61 min (3); m/z = 564 | 8.52 (s, 1H), 8.18-7.95 (br. s, 2H), 8.14-8.07 (m, 1H), 8.03 (s, 2H), 8.00-7.95 (m, 1H), 7.59 (d, 2H), 7.48 (d, 2H), 7.10 (d, 2H), 5.00 (d, 1H), 4.70 (t, 1H), 4.54 (s, 2H), 4.09 (dd, 1H), 3.95 (dd, 1H), 3.85-3.78 (m, 1H), 3.47 (t, 2H), 2.50 (s, 3H). |
| 90 | 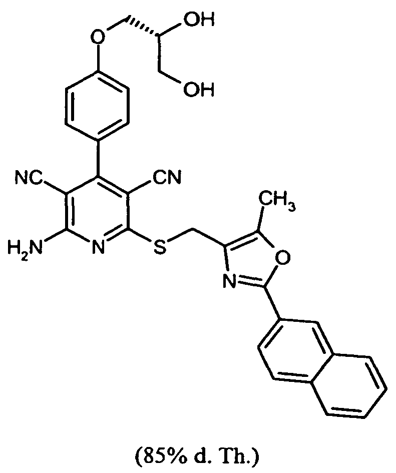 | 2.61 min (3); m/z = 564 | 8.52 (s, 1H), 8.20-7.94 (br. s, 2H), 8.13-8.07 (m, 1H), 8.04 (s, 2H), 8.00-7.94 (m, 1H), 7.62-7.57 (m, 2H), 7.49 (d, 2H), 7.10 (d, 2H), 5.00 (d, 1H), 4.70 (t, 1H), 4.55 (s, 2H), 4.09 (dd, 1H), 3.95 (dd, 1H), 3.86-3.78 (m, 1H), 3.46 (t, 2H), 2.50 (s, 3H). |
| **91** | 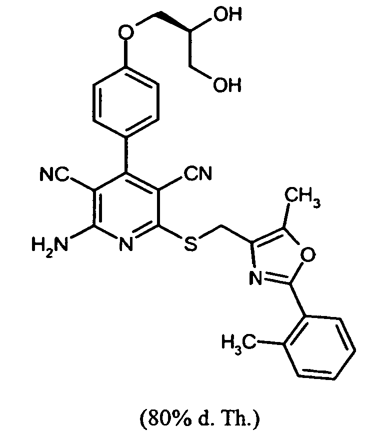 | 1.95 min (7); m/z = 528 | 8.18-7.95 (br. s, 2H), 7.86 (d, 1H), 7.48 (d, 2H), 7.41-7.29 (m, 3H), 7.10 (d, 2H), 5.01 (d, 1H), 4.70 (t, 1H), 4.52 (s, 2H), 4.10 (dd, 1H), 3.95 (dd, 1H), 3.85-3.78 (m, 1H), 3.48 (t, 2H), 2.60 (s, 3H), 2.47 (s, 3H). |
| **92** | 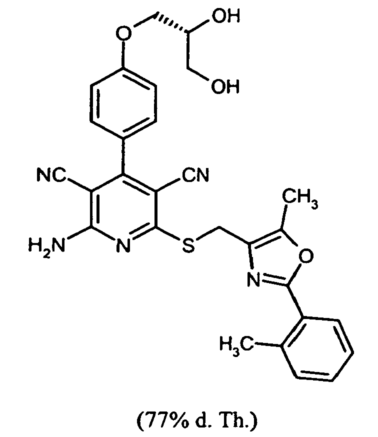 | 1.95 min (7); m/z = 528 | 8.17-7.94 (br. s, 2H), 7.86 (d, 1H), 7.49 (d, 2H), 7.41-7.30 (m, 3H), 7.11 (d, 2H), 5.01 (d, 1H), 4.70 (t, 1H), 4.52 (s, 2H), 4.09 (dd, 1H), 3.95 (dd, 1H), 3.85-3.78 (m, 1H), 3.47 (t, 2H), 2.60 (s, 3H), 2.48 (s, 3H). |
| **93** | 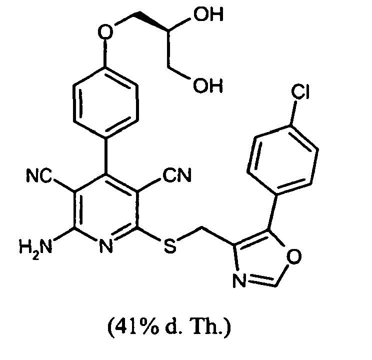 | 2.15 min (15); m/z = 534 | 8.51 (s, 1H), 8.04-7.89 (br. s, 2H), 7.74 (d, 2H), 7.61 (d, 2H), 7.50 (d, 2H), 7.10 (d, 2H), 5.00 (d, 1H), 4.75 (s, 2H), 4.69 (t, 1H), 4.09 (dd, 1H), 3.97 (dd, 1H), 3.87-3.78 (m, 1H), 3.48 (t, 2H). |
| **94** | 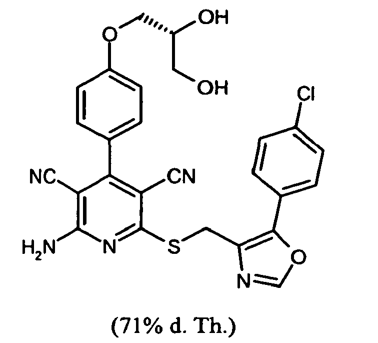 | 2.15 min (15); m/z = 534 | 8.51 (s, 1H), 8.04-7.89 (br. s, 2H), 7.74 (d, 2H), 7.61 (d, 2H), 7.49 (d, 2H), 7.11 (d, 2H), 5.00 (d, 1H), 4.75 (s, 2H), 4.69 (t, 1H), 4.09 (dd, 1H), 3.96 (dd, 1H), 3.86-3.78 (m, 1H), 3.47 (t, 2H). |

### Beispiel 95

4-{4-[({6-Amino-3,5-dicyano-4-[4-(2-hydroxy-2-methylpropoxy)phenyl]pyridin-2-yl}thio)-methyl]-5-methyl-1,3-oxazol-2-yl}benzoesäure 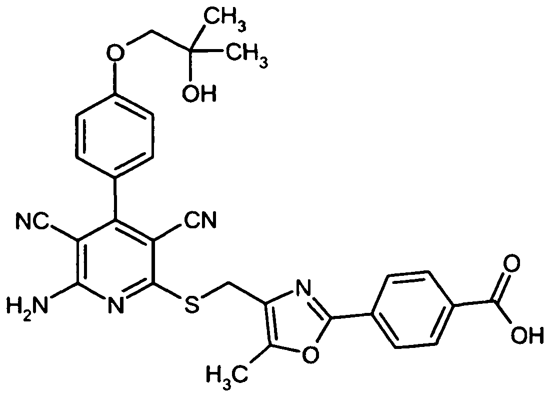 40 mg (0.07 mmol) der Verbindung aus Beispiel 17 und 11 mg (0.28 mmol) Natriumhydroxid werden in 12.8 ml 1,2-Dimethoxyethan, 0.7 ml Methanol und 2.8 ml Wasser gelöst. Die Reaktionslösung wird 3 h bei RT gerührt. Anschließend wird das Gemisch am Rotationsverdampfer eingeengt. Der Rückstand wird mit 5 ml Wasser versetzt. Durch Zugabe von 1 N Salzsäure wird der pH-Wert auf 4 eingestellt. Der dabei ausfallende Niederschlag wird abfiltriert und mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 19 mg (47% d. Th.)

¹H-NMR (400 MHz, DMSO-d₆): δ = 13.19 (s, 1H), 8.23-7.96 (br. s, 2H), 8.09-8.02 (m, 4H), 7.48 (d, 2H), 7.10 (d, 2H), 4.69 (s, 1H), 4.53 (s, 2H), 3.80 (s, 2H), 2.50 (s, 3H), 1.21 (s, 6H).

LC-MS (Methode 3): Rₜ = 2.41 min; MS (ESIpos): m/z = 556 [M+H]⁺.

Die in Tabelle 10 aufgeführten Beispiele werden aus den entsprechenden Ausgangsverbindungen analog zu Beispiel 95 hergestellt:

**Tabelle 10**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ **=** |
|---|---|---|---|
| **96** | 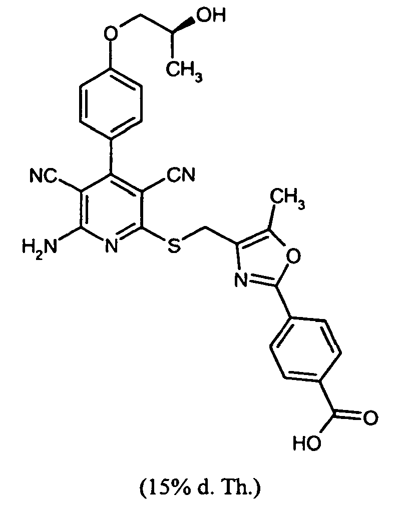 | 2.32 min (3); m/z = 542 | 13.20 (s, 1H), 8.18-7.98 (br. s, 2H), 8.08-8.01 (m, 4H), 7.48 (d, 2H), 7.09 (d, 2H), 4.92 (d, 1H), 4.53 (s, 2H), 4.02-3.94 (m, 1H), 3.93-3.86 (m, 2H), 2.49 (s, 3H), 1.18 (d, 3H). |
| **97** | 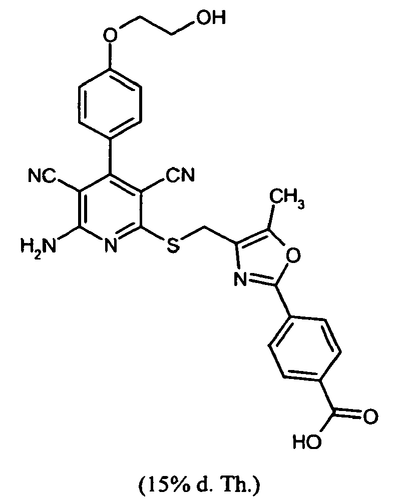 | 1.73 min (17) | 13.19 (s, 1H), 8.29-7.95 (br. s, 2H), 8.10-7.99 (m, 4H), 7.48 (d, 2H), 7.10 (d, 2H), 4.95-4.88 (m, 1H), 4.52 (s, 2H), 4.08 (t, 2H), 3.78-3.70 (m, 2H), 2.48 (s, 3H). |

### Beispiel 98

4-[4-({[6-Amino-3,5-dicyano-4-(4-{[(2*S*)-2,3-dihydroxypropyl]oxy}phenyl)pyridin-2-yl]thio}-methyl)-5-methyl-1,3-oxazol-2-yl]benzoesäure 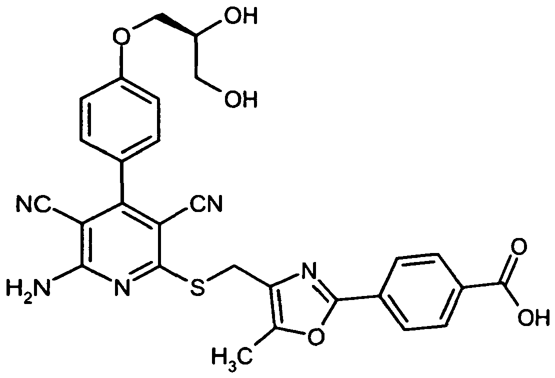 63 mg (0.11 mmol) der Verbindung aus Beispiel 57 werden in 3 ml THF gelöst und mit 220 µl (0.22 mmol) einer 1 M wässrigen Lithiumhydroxid-Lösung versetzt. Das Reaktionsgemisch wird in einer Mikrowelle auf 140°C aufgeheizt und 15 min bei dieser Temperatur gerührt. Das Lösungsmittel wird dann am Rotationsverdampfer entfernt. Der Rückstand wird in 3 ml Wasser aufgenommen und mit ca. 0.5 ml 1 N Salzsäure auf pH 4 eingestellt. Es fällt ein Niederschlag aus, der abfiltriert und mittels präparativer HPLC gereinigt wird (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 3 mg (5% d. Th.)

¹H-NMR (400 MHz, DMSO-d₆): δ = 13.19 (br. s, 1H), 8.19-7.91 (m, 6H), 7.49 (d, 2H), 7.11 (d, 2H), 5.00 (d, 1H), 4.70 (t, 1H), 4.53 (s, 2H), 4.09 (dd, 1H), 3.95 (dd, 1H), 3.82 (q, 1H), 3.46 (t, 2H), 2.50 (s, 3H).

LC-MS (Methode 11): Rₜ = 1.56 min; MS (ESIpos): m/z = 558 [M+H]⁺.

### Beispiel 99

2-Amino-4-(4-{[(2*S*)-2,3-dihydroxypropyl]oxy}phenyl)-6-({[2-(3-fluorphenyl)-1,3-oxazol-4-yl]-methyl}thio)pyridin-3,5-dicarbonitril 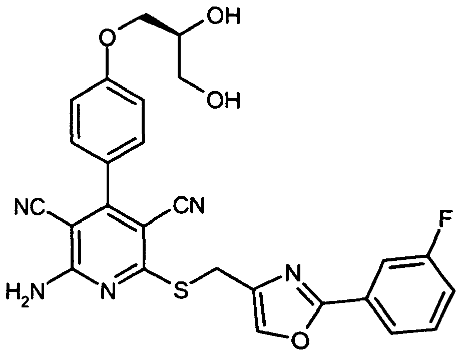 75 mg (0.17 mmol) der Verbindung aus Beispiel 10A und 38 mg (0.18 mmol) der Verbindung aus Beispiel 21A werden in 2 ml trockenem DMF gelöst, mit 50 mg (0.36 mmol) Kaliumcarbonat versetzt und 8 h bei RT gerührt. Anschließend werden 0.82 ml (1.65 mmol) 2 N Salzsäure zugetropft und das Gemisch nochmals 1 h bei RT gerührt. Nach Filtration wird das Lösungsmittel am Rotationsverdampfer entfernt. Der Rückstand wird mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 17 mg (20% d. Th.)

¹H-NMR (400 MHz, DMSO-d₆): δ = 8.39 (s, 1H), 8.27-7.89 (br. s, 2H), 7.81 (d, 1H), 7.70 (d, 1H), 7.59 (q, 1H), 7.46 (d, 2H), 7.39 (dt, 1H), 7.09 (d, 2H), 5.00 (d, 1H), 4.69 (t, 1H), 4.42 (s, 2H), 4.09 (dd, 1H), 3.94 (dd, 1H), 3.81 (q, 1H), 3.47 (t, 2H).

LC-MS (Methode 2): Rₜ = 2.06 min; MS (ESIpos): m/z = 518 [M+H]⁺.

Die in Tabelle 11 aufgeführten Beispiele werden aus den entsprechenden Ausgangsverbindungen analog zu Beispiel 99 hergestellt:

**Tabelle 11**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ **=** |
|---|---|---|---|
| **100** | 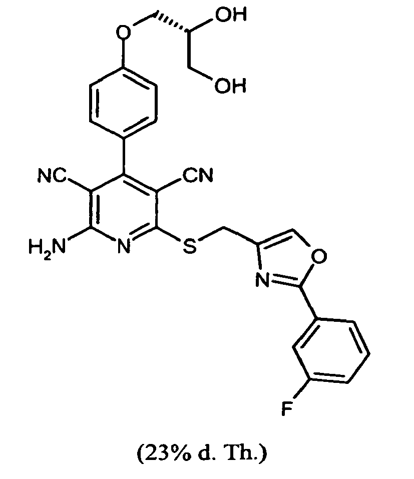 | 2.39 min (10); m/z = 518 | 8.38 (s, 1H), 8.29-7.90 (br. s, 2H), 7.81 (d, 1H), 7.71 (d, 1H), 7.59 (q, 1H), 7.48 (d, 2H), 7.40 (dt, 1H), 7.09 (d, 2H), 5.00 (d, 1H), 4.69 (t, 1H), 4.42 (s, 2H), 4.08 (dd, 1H), 3.94 (dd, 1H), 3.81 (q, 1H), 3.46 (t, 2H). |
| **101** | 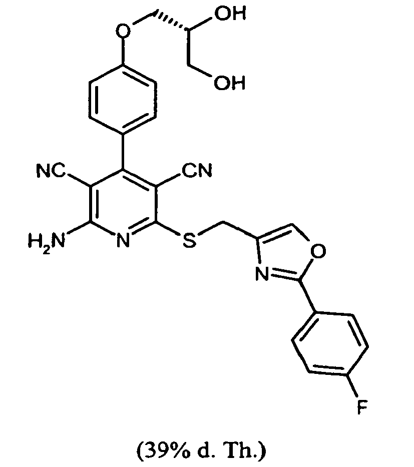 | 2.08 min (2); m/z = 518 | 8.33 (s, 1H), 8.27-7.93 (br. s, 2H), 8.03 (d, 1H), 8.01 (d, 1H), 7.48 (d, 2H), 7.38 (t, 2H), 7.10 (d, 2H), 5.00 (d, 1H), 4.70 (t, 1H), 4.42 (s, 2H), 4.09 (dd, 1H), 3.96 (dd, 1H), 3.87-3.78 (m, 1H), 3.47 (t, 2H). |
| **102** | 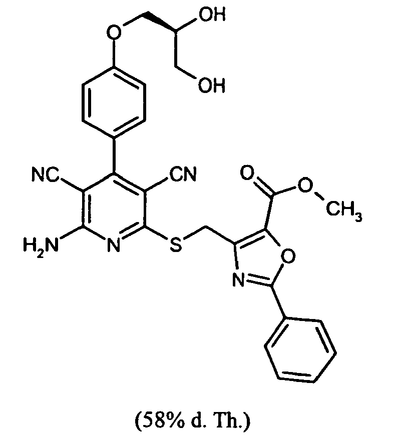 | 1.83 min (7); m/z = 558 | 8.09-7.94 (br. s, 2H), 8.05 (d, 2H), 7.68-7.57 (m, 3H), 7.50 (d, 2H), 7.11 (d, 2H), 5.01 (d, 1H), 4.83 (s, 2H), 4.70 (t, 1H), 4.10 (dd, 1H), 3.99-3.91 (m, 1H), 3.93 (s, 3H), 3.86-3.78 (m, 1H), 3.47 (t, 2H). |
| **103** | 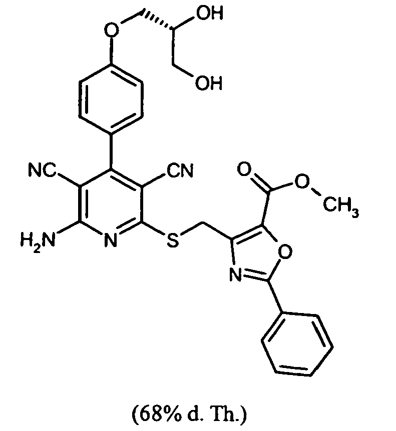 | 1.84 min (7); m/z = 558 | 8.08-7.93 (br. s, 2H), 8.05 (d, 2H), 7.67-7.57 (m, 3H), 7.50 (d, 2H), 7.11 (d, 2H), 5.01 (d, 1H), 4.82 (s, 2H), 4.70 (t, 1H), 4.10 (dd, 1H), 3.99-3.91 (m, 1H), 3.93 (s, 3H), 3.85-3.79 (m, 1H), 3.47 (t, 2H). |

### Beispiel 104

2-Amino-6-({[2-(4-fluorphenyl)-5-methyl-1,3-oxazol-4-yl]methyl}thio)-4-(4-{((2*S*)-2-hydroxypropyl]oxy}phenyl)pyridin-3,5-dicarbonitril 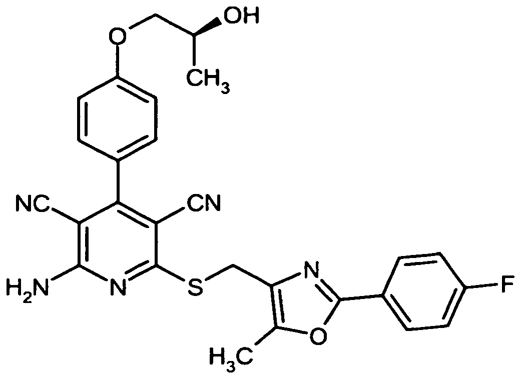 65 mg (0.10 mmol) der Verbindung aus Beispiel 95A werden in 4 ml Methanol gelöst und mit 1.5 ml 1 N Salzsäure versetzt. Es wird 12 h bei RT gerührt. Anschließend wird das Lösungsmittel am Rotationsverdampfer entfernt und der Rückstand mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 48 mg (91% d. Th.)

¹H-NMR (400 MHz, DMSO-d₆): δ = 8.20-7.91 (br. s, 2H), 7.49 (d, 2H), 7.35 (pseudo-t, 2H), 7.09 (d, 2H), 4.91 (d, 1H), 4.50 (s, 2H), 4.02-3.94 (m, 1H), 3.92-3.86 (m, 2H), 2.48 (s, 3H), 1.18 (s, 3H).

LC-MS (Methode 3): Rₜ = 2.67 min; MS (ESIpos): m/z = 516 [M+H]⁺.

Die in Tabelle 12 aufgeführten Beispiele werden aus den entsprechenden Ausgangsverbindungen analog zu Beispiel 104 hergestellt:

**Tabelle 12**

| **Biespiel Nr.** | **Struktur (Ausbeute)** | **LC-MS: R₁ [min] (Methode); MS (ESI): m/z [M+H]⁺** | **¹H-NMR (DMSO-d₆): δ** |
|---|---|---|---|
| 105 | 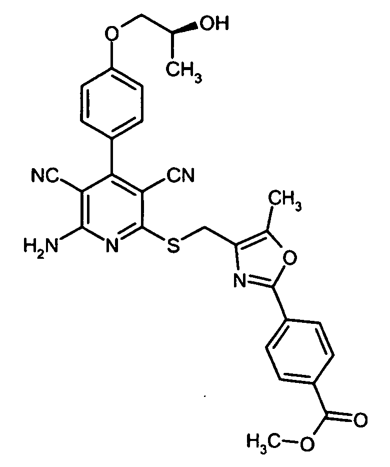 | 2.64 min (3); m/z = 556 | 8.20-7.95 (br.s, 2H), 8.11-8.03 (m,4H), 7.48 (d,2H), 7.10 (d, 2H), 4.92 (d,1H), 4.54 (s, 2H), 4.03-3.94 (m, 1H), 3.91-3.85 (m,2H) 3.89 (s, 3H), 1.18 (d, 3H). |
| | (74% d. Th.) | | |
| 106 | 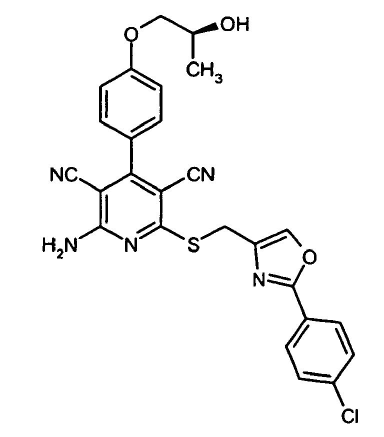 | 2.76 min (3); m/z = 518 | 8.37 (s, 1H), 8.27-7.91 (br.s, 2H), 7.97 (d, 2H), 7.61 (d,2H), 7.47 (d, 2H), 7.09 (d, 2H), 4.92 (d, 1H), 4.42 (s, 2H), 4.02-3.93 (m, 1H), 3.92-3.85 (m, 2H), 1.18 (d, 3H). |
| | (55% d.Th.) | | |

### Beispiel 107

4-({[6-Amino-3,5-dicyano-4-(4-{[(2*R*)-2,3-dihydroxypropyl]oxy}phenyl)pyridin-2-yl]sulfanyl}-methyl)-2-phenyl-1,3-oxazol-5-carbonsäure 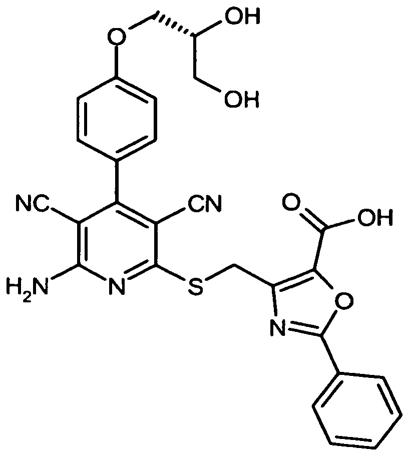 30 mg (0.05 mmol) der Verbindung aus Beispiel 103 und 0.22 ml (0.22 mmol) 1 N Natronlauge werden in 2 ml 1,2-Dimethoxyethan, 2 ml Wasser und 0.5 ml Methanol gelöst und 3 h bei RT gerührt. Die Lösungsmittel werden danach am Rotationsverdampfer entfernt und der Rückstand in 2 ml Wasser aufgenommen. Der pH-Wert wird durch Zugabe von 1 N Salzsäure auf 4 eingestellt. Es fällt ein weißer Niederschlag aus, der abgesaugt und im Vakuum getrocknet wird.

Ausbeute: 8 mg (26% d. Th.)

¹H-NMR (400 MHz, DMSO-d₆): δ = 14.15-13.93 (br. s, 1H), 8.08-7.92 (br. s, 2H), 8.04 (d, 2H), 7.67-7.57 (m, 3H), 7.50 (d, 2H), 7.11 (d, 2H), 5.05-4.97 (br. s, 1H), 4.71 (s, 2H), 4.74-4.66 (br. s, 1H), 4.09 (dd, 1H), 3.99-3.89 (m, 1H), 3.87-3.79 (br. s, 1H), 3.51-3.43 (m, 2H).

LC-MS (Methode 3): Rₜ = 2.19 min; MS (ESIpos): m/z = 544 [M+H]⁺.

### Beispiel 108

Methyl *N*-[6-({[2-(4-chlorphenyl)-1,3-oxazol-4-yl]methyl)thio)-3,5-dicyano-4-(4-{[(2*S*)-2,3-dihydroxypropyl]oxy}phenyl)pyridin-2-yl]-*N*-methylglycinat 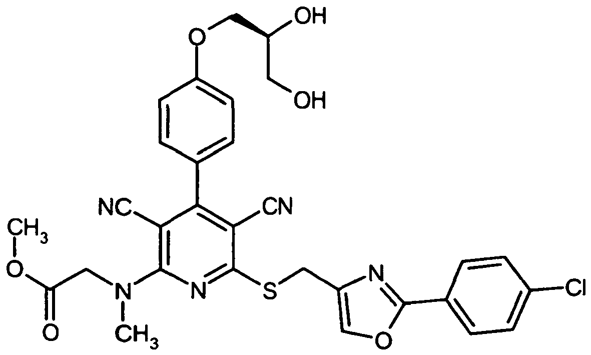 108 mg (0.20 mmol) der Verbindung aus Beispiel 103A werden in 3 ml trockenem DMF gelöst und mit 54 mg (0.39 mmol) Methyl N-methylglycinat-Hydrochlorid sowie 59 mg (0.59 mmol) Triethylamin versetzt. Das Reaktionsgemisch wird 8 h bei RT gerührt. Das Lösungsmittel wird anschließend am Rotationsverdampfer entfernt und der Rückstand direkt mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 13 mg (11 % d. Th.)

¹H-NMR (400 MHz, DMSO-d₆): δ = 8.19 (s, 1H), 7.98 (d, 2H), 7.62 (d, 2H), 7.55 (d, 2H), 7.11 (d, 2H), 5.01 (d, 1H), 4.71 (t, 1H), 4.61 (s, 2H), 4.41 (s, 2H), 4.09 (dd, 1H), 3.97 (dd, 1H), 3.87-3.78 (m, 1H), 3.65 (s, 3H), 3.51-3.43 (m, 5H).

LC-MS (Methode 5): Rₜ = 3.65 min; MS (ESIpos): m/z = 620 [M+H]⁺.

### Beispiel 109

2-Amino-6-{[2-(4-chlorphenyl)-1,3-oxazol-4-yl]methoxy}-4-(4-{[(2*R*)-2,3-dihydroxypropyl]oxy}-phenyl)pyridin-3,5-dicarbonitril 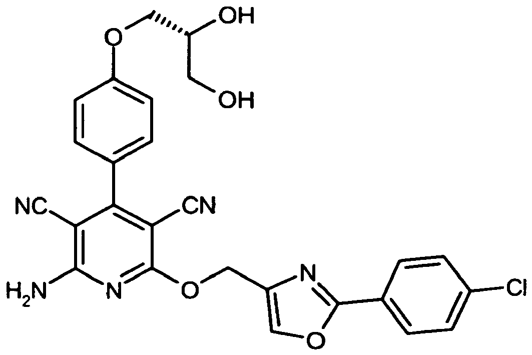 65 mg (0.12 mmol) der Verbindung aus Beispiel 101A werden in 6 ml Essigsäure gelöst und mit 3 ml Wasser versetzt. Es wird 30 min bei RT gerührt. Anschließend wird auf 70°C erhitzt und weitere 30 min bei dieser Temperatur gerührt. Es entsteht eine klare Lösung. Der Ansatz wird dann am Rotationsverdampfer vom Lösungsmittel befreit und der Rückstand mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 60 mg (100% d. Th.)

¹H-NMR (400 MHz, DMSO-d₆): δ = 8.48 (s, 1H), 8.25-7.80 (br. s, 2H), 8.01 (d, 2H), 7.61 (d, 2H), 7.47 (d, 2H), 7.10 (d, 2H), 5.42 (s, 2H), 5.01 (d, 1H), 4.70 (t, 1H), 4.09 (dd, 1H), 3.95 (dd, 1H), 3.87-3.77 (m, 1H), 3.48 (t, 2H).

LC-MS (Methode 3): Rₜ = 2.30 min; MS (ESIpos): m/z = 518 [M+H]⁺.

### Beispiel 110

2-Amino-6-{[2-(4-chlorphenyl)-1,3-oxazol-4-yl]methoxy}-4-[4-(2-hydroxyethoxy)phenyl]pyridin-3,5-dicarbonitril 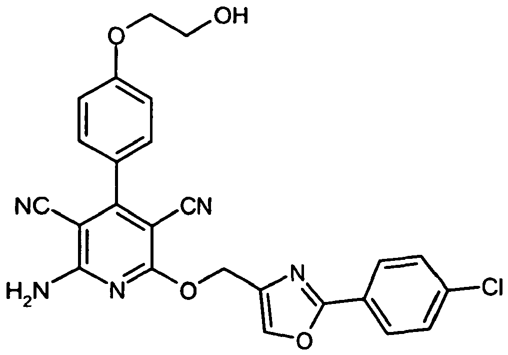 72 mg (0.64 mmol) Kalium-tert.-butylat werden in 1 ml trockenem 1,2-Dimethoxyethan suspendiert. Dann werden nacheinander 270 mg (1.29 mmol) der Verbindung aus Beispiel 100A und 50 mg (0.13 mmol) der Verbindung aus Beispiel 99A zugegeben. Das Reaktionsgemisch wird 2 h bei 60°C gerührt, anschließend auf RT abgekühlt und weitere 8 h bei dieser Temperatur nachgerührt. Der Ansatz wird dann mit 5 ml Wasser und 1 ml 2 N Essigsäure versetzt. Es fällt ein Niederschlag aus, der abgesaugt und mittels präparativer HPLC gereinigt wird (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als gelben Feststoff.

Ausbeute: 44 mg (70% d. Th.)

¹H-NMR (400 MHz, DMSO-d₆): δ = 8.48 (s, 1H), 8.18-7.85 (br. s, 2H), 8.00 (d, 2H), 7.62 (d, 2H), 7.48 (d, 2H), 7.11 (d, 2H), 5.41 (s, 2H), 4.91 (t, 1H), 4.08 (t, 2H), 3.73 (q, 2H).

LC-MS (Methode 14): Rₜ = 1.22 min; MS (ESIpos): m/z = 488 [M+H]⁺.

Die in Tabelle 13 aufgeführten Beispiele werden aus den entsprechenden Ausgangsverbindungen analog zu Beispiel 110 hergestellt:

**Tabelle 13**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ **=** |
|---|---|---|---|
| **111** | 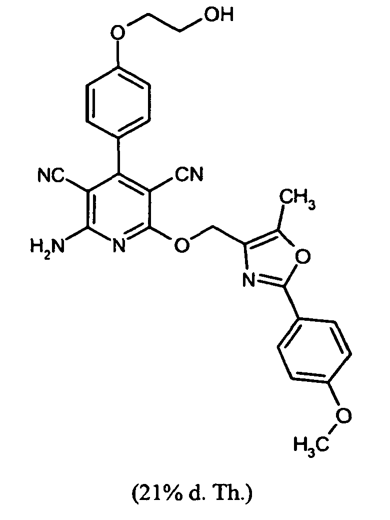 | 1.15 min (14); m/z = 498 | 8.11-7.85 (br. s, 2H), 7.89 (d, 2H), 7.48 (d, 2H), 7.11 (d, 2H), 7.09 (d, 2H), 5.39 (s, 2H), 4.91 (t, 1H), 4.08 (t, 2H), 3.82 (s, 3H), 3.74 (q, 2H), 2.50 (s, 3H). |
| **112** | 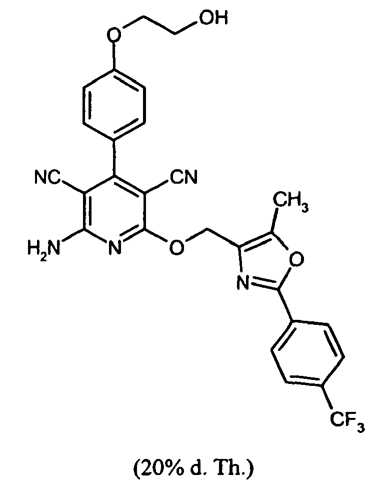 | 2.41 min (15); m/z = 536 | 8.16 (d, 2H), 8.10-7.83 (br. s, 2H), 7.90 (d, 2H), 7.48 (d, 2H), 7.10 (d, 2H), 5.48 (s, 2H), 4.91 (t, 1H), 4.08 (t, 2H), 3.74 (q, 2H), 2.47 (s, 3H). |

### Beispiel 113

2-Amino-6-{[2-(4-fluorphenyl)-5-methyl-1,3-oxazol-4-yl]methoxy}-4-[4-(2-hydroxyethoxy)-phenyl]pyridin-3,5-dicarbonitril 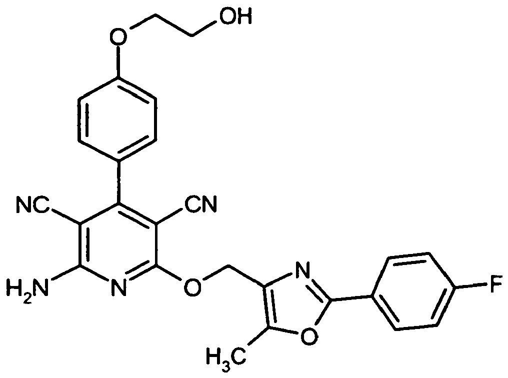 250 mg (0.81 mmol) der Verbindung aus Beispiel 102A, 228 mg (1.013 mmol) 4-(Chlormethyl)-2-(4-fluorphenyl)-5-methyl-1,3-oxazol und 224 mg (1.62 mmol) Kaliumcarbonat werden in 8.6 ml trockenem DMF vorgelegt und 2 h bei 70°C gerührt. Das Lösungsmittel wird danach am Rotationsverdampfer entfernt und der Rückstand mittels präparativer HPLC aufgereinigt (Säule: YMC GEL ODS-AQ S-5 / 15 µm; Laufmittel-Gradient: Acetonitril/Wasser 10:90 → 95:5). Anschließend erfolgt eine nochmalige Reinigung mittels HPLC (Säule: Waters Sunfire C 18 5 µm, 250 mm x 20 mm; Laufmittel-Gradient: Wasser/Ethanol 55:45 → 5:95; Fluss: 25 ml/min; Temperatur: 30°C; Detektion: 210 nm).

Ausbeute: 56 mg (14% d. Th.)

¹H-NMR (400 MHz, DMSO-d₆): δ = 8.07-7.87 (br. s, 2H), 8.03-7.98 (m, 2H), 7.48 (d, 2H), 7.37 (t, 2H), 7.10 (d, 2H), 5.40 (s, 2H), 4.91 (t, 1H), 4.08 (t, 2H), 3.74 (q, 2H), 2.49 (s, 3H).

LC-MS (Methode 14): Rₜ = 1.18 min; MS (ESIpos): m/z = 486 [M+H]⁺.

Die in Tabelle 14 aufgeführten Beispiele werden aus den entsprechenden Ausgangsverbindungen analog zu Beispiel 113 hergestellt:

**Tabelle 14**

| **Beispiel Nr.** | **Struktur (Ausbeute)** | **LC-MS: Rₜ [min] (Methode); MS (ESI): m/z [M+H]⁺** | **¹H-NMR(DMSO-d₆):** δ **=** |
|---|---|---|---|
| **114** | 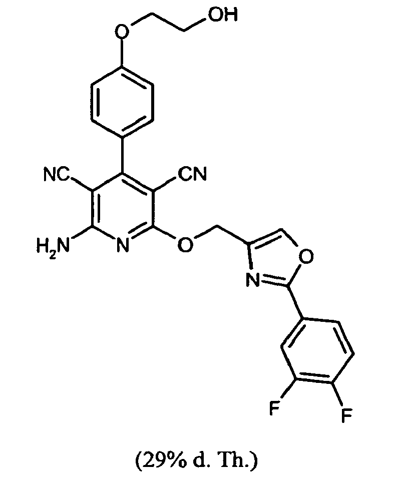 | 2.33 min (9); m/z = 490 | 8.51 (s, 1H), 8.25-7.80 (br. s, 2H), 8.01 (dt, 1H), 7.90-7.82 (m, 1H), 7.62 (q, 1H), 7.48 (d, 2H), 7.11 (d, 2H), 5.41 (s, 2H), 4.93 (t, 1H), 4.08 (t, 2H), 3.23 (q, 2H). |
| **115** | 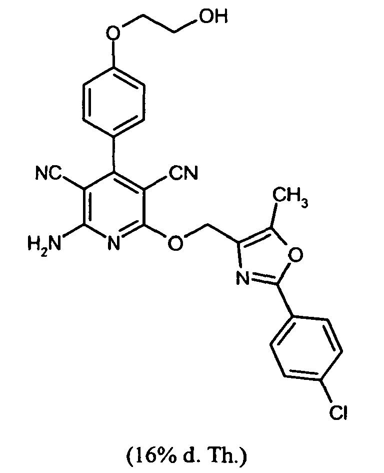 | 1.26 min (14); m/z = 502 | 8.08-7.95 (br. s, 2H), 7.96 (d, 2H), 7.60 (d, 2H), 7.48 (d, 2H), 7.11 (d, 2H), 5.41 (s, 2H), 4.90 (t, 1H), 4.07 (t, 2H), 3.73 (q, 2H), 2.50 (s, 3H). |

### Beispiel 116

2-({[2-(4-Chlorphenyl)-1,3-oxazol-4-yl]methyl}sulfanyl)-4-[4-(2-hydroxyethoxy)phenyl]-6-(pyrrolidin-1-yl)pyridin-3,5-dicarbonitril 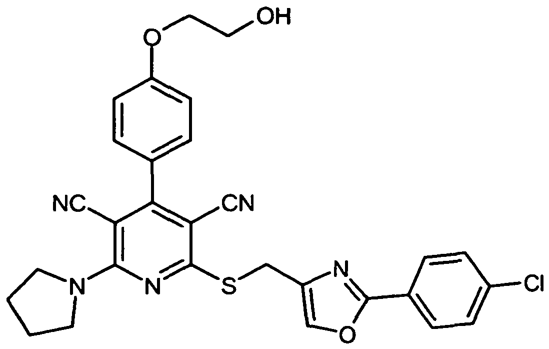 80 mg (0.15 mmol) der Verbindung aus Beispiel 123A werden in 2 ml trockenem THF vorgelegt und mit 22 mg (0.31 mmol) Pyrrolidin versetzt. Das Reaktionsgemisch wird 10 h bei RT gerührt. Der Ansatz wird danach mit ca. 2 ml Wasser versetzt und direkt mittels präparativer HPLC gereinigt (Säule: YMC GEL ODS-AQ S-5 / 15 µm; Laufmittel-Gradient: Acetonitril/Wasser 10:90 → 95:5). Nach Entfernen des Lösungsmittels am Rotationsverdampfer erhält man das Produkt als weißen Feststoff.

Ausbeute: 26 mg (30% d. Th.)

¹H-NMR (400 MHz, DMSO-d₆): δ = 8.20 (s, 1H), 7.97 (d, 2H), 7.61 (d, 2H), 7.49 (d, 2H), 7.11 (d, 2H), 4.91 (t, 1H), 4.51 (s, 2H), 4.09 (t, 2H), 3.91-3.81 (br. s, 4H), 3.74 (q, 2H), 2.02-1.91 (br. s, 4H).

LC-MS (Methode 3): Rₜ = 3.02 min; MS (ESIpos): m/z = 558 [M+H]⁺.

Die in Tabelle 15 aufgeführten Beispiele werden aus den entsprechenden Ausgangsverbindungen analog zu Beispiel 116 hergestellt:

**Tabelle 15**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ **=** |
|---|---|---|---|
| **117** | 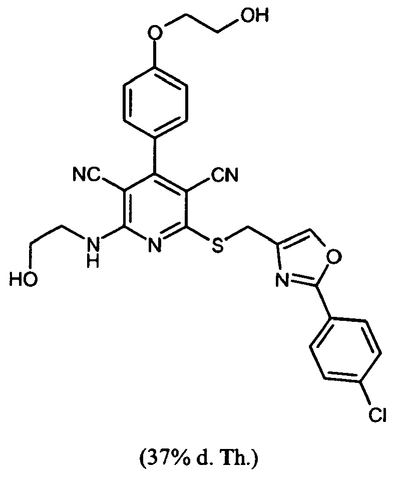 | 2.53 min (3); m/z = 548 | 8.20 (s, 1H), 8.04-7.92 (m, 1H), 7.97 (d, 2H), 7.62 (d, 2H), 7.49 (d, 2H), 7.12 (d, 2H), 4.91 (t, 1H), 4.83 (t, 1H), 4.51 (s, 2H), 4.08 (t, 2H), 3.73 (q, 2H), 3.67-3.61 (m, 2H), 3.61-3.53 (m, 2H). |
| **118** | 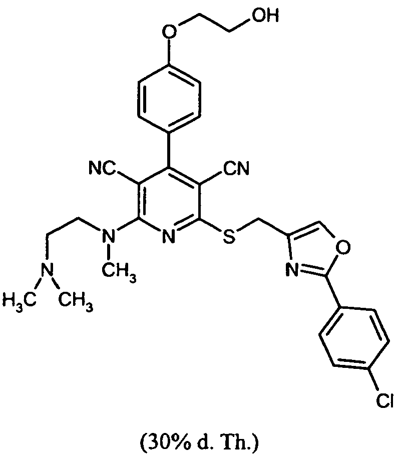 | 1.75 min (3); m/z = 589 | 8.19 (s, 1H), 7.97 (d, 2H), 7.61 (d, 2H), 7.50 (d, 2H), 7.10 (d, 2H), 4.91 (t, 1H), 4.51 (s, 2H), 4.08 (t, 2H), 3.89 (t, 2H), 3.75 (q, 2H), 3.40 (s, 3H), 3.35-3.29 (m, 2H), 2.14 (s, 6H). |
| **119** | 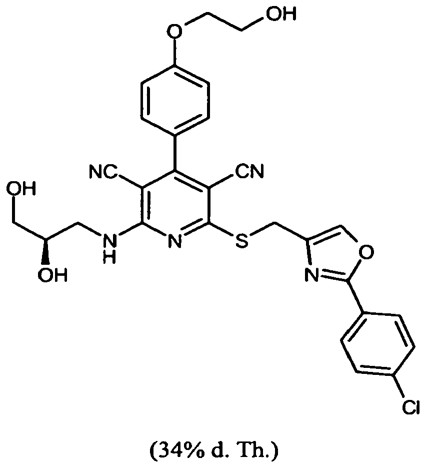 | 2.36 min (3); m/z = 578 | 8.21 (s, 1H), 7.98 (d, 2H), 7.90 (t, 1H), 7.60 (d, 2H), 7.48 (d, 2H), 7.11 (d, 2H), 4.95 (d, 1H), 4.91 (t, 1H), 4.73 (t, 1H), 4.57 (d, 1H), 4.50 (d, 1H), 4.08 (t, 2H), 3.72-3.69 (m, 4H), 3.59-3.48 (m, 1H), 3.45-3.34 (m, 2H). |

Die in Tabelle 16 aufgeführten Beispiele werden aus den entsprechenden Ausgangsverbindungen analog zu Beispiel 48 hergestellt:

**Tabelle 16**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ **=** |
|---|---|---|---|
| **120** | 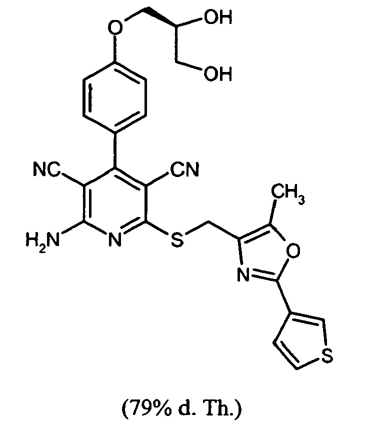 | 2.25 min (3); m/z = 520 | 8.19-7.93 (br. s, 2H), 8.12 (d, 1H), 7.71 (dd, 1H), 7.53 (d, 1H), 7.48 (d, 2H), 7.10 (d, 2H), 5.00 (d, 1H), 4.70 (t, 1H), 4.49 (s, 2H), 4.09 (dd, 1H), 3.95 (dd, 1H), 3.87-3.78 (m, 1H), 3.47 (t, 2H), 2.45 (s, 3H). |
| **121** | 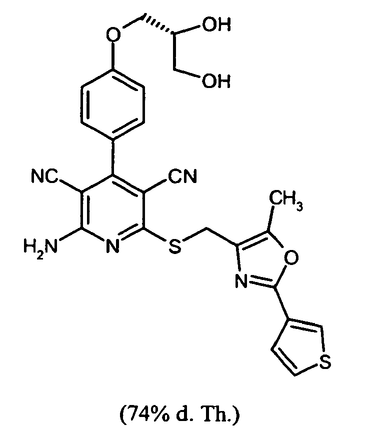 | 2.25 min (3); m/z = 520 | 8.18-7.93 (br. s, 2H), 8.11 (d, 1H), 7.71 (dd, 1H), 7.52 (d, 1H), 7.48 (d, 2H), 7.11 (d, 2H), 5.01 (d, 1H), 4.70 (t, 1H), 4.49 (s, 2H), 4.09 (dd, 1H), 3.94 (dd, 1H), 3.85-3.78 (m, 1H), 3.46 (t, 2H), 2.44 (s, 3H). |

### Beispiel 122

2-({[2-(4-Chlorphenyl)-1,3-oxazol-4-yl]methyl}sulfanyl)-4-[3-fluor-4-(2-hydroxyethoxy)phenyl]-6-[(2-hydroxyethyl)amino]pyridin-3,5-dicarbonitril 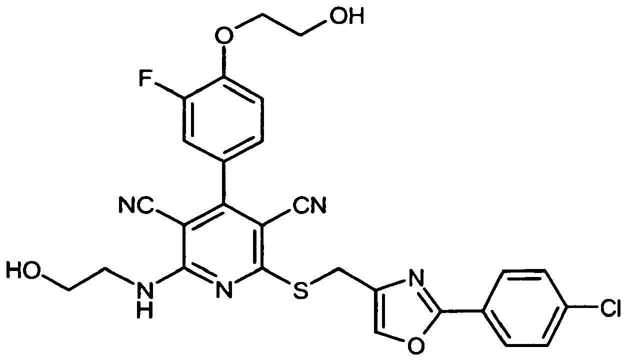 25 mg (0.046 mmol) 2-Chlor-6-({[2-(4-chlorphenyl)-1,3-oxazol-4-yl]methyl}sulfanyl)-4-[3-fluor-4-(2-hydroxyethoxy)phenyl]pyridin-3,5-dicarbonitril (Beispiel 125A) werden in 1 ml THF vorgelegt, mit 6 µl 2-Aminoethanol versetzt und 30 Minuten bei Raumtemperatur gerührt. Das Reaktionsgemisch wird danach direkt mittels präparativer HPLC gereinigt (Laufmittel-Gradient: Acetonitril/Wasser 10:90 → 95:5). Es werden 24 mg (94% d. Th.) der Zielverbindung erhalten.

¹H-NMR (400 MHz, DMSO-d₆): δ = 8.20 (s, 1H), 8.08 (t, 1H), 7.98 (d, 2H), 7.61 (d, 2H), 7.51 (dd, 1H), 7.39-7.31 (m, 2H), 4.51 (s, 2H), 4.16 (t, 2H), 3.76 (t, 2H), 3.67-3.62 (m, 2H), 3.58-3.55 (m, 2H).

LC-MS (Methode 3): Rₜ = 2.52 min; MS (ESIpos): m/z = 566 [M+H]⁺.

### Beispiel 123

4-[({6-Amino-3,5-dicyano-4-[4-(2-hydroxyethoxy)phenyl]pyridin-2-yl}sulfanyl)methyl]-2-phenyl-1,3-oxazol-5-carbonsäure 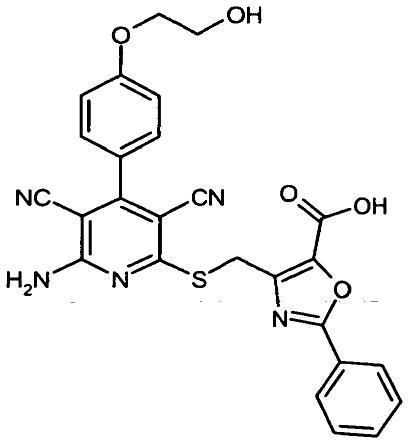 100 mg (0.190 mmol) Methyl 4-[({6-amino-3,5-dicyano-4-[4-(2-hydroxyethoxy)phenyl]pyridin-2-yl}sulfanyl)methyl]-2-phenyl-1,3-oxazol-S-carboxylat (Beispiel 36) werden in 6 ml THF vorgelegt, mit 379 µl (0.379 mmol) 1 N wässrige Lithiumhydroxid-Lösung versetzt und 30 Minuten bei Raumtemperatur gerührt. Das Reaktionsgemisch wird danach eingeengt, der Rückstand mit Wasser versetzt und mit 1 N Salzsäure angesäuert. Der ausgefallene Feststoff wird abfiltriert und mittels präparativer HPLC gereinigt (Laufmittel-Gradient: Acetonitril/Wasser 10:90 → 95:5). Man erhält 23 mg (23% d. Th.) der Zielverbindung.

¹H-NMR (400 MHz, DMSO-d₆): δ = 8.03 (dd, 2H), 7.98-7.95 (m, 2H), 7.63-7.56 (m, 3H), 7.50 (d, 2H), 7.11 (d, 2H), 4.80 (s, 2H), 4.08 (t, 2H), 3.74 (t, 2H).

LC-MS (Methode 3): Rₜ = 2.38 min; MS (ESIpos): m/z = 514 [M+H]⁺.

Die in Tabelle 17 aufgeführten Beispiele werden aus den entsprechenden Ausgangsverbindungen analog zu Beispiel 116 hergestellt:

**Tabelle 17**

| **Beispiel Nr.** | **Struktur (Ausbeute)** | **LC-MS: Rₜ [min] (Methode); MS (ESI): m/z [M+H]⁺** | **¹H-NMR (DMSO-d₆):** δ **=** |
|---|---|---|---|
| **124** | 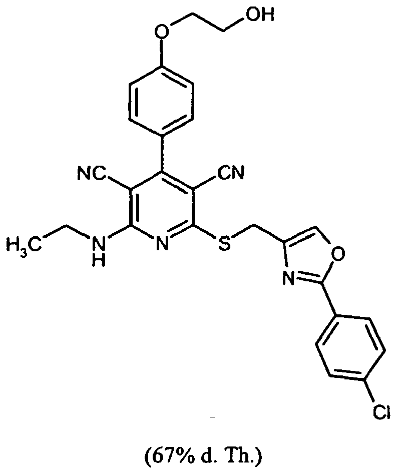 | 2.81 min (3); m/z = 532 | 8.21 (t, 1H), 8.18 (s, 1H), 7.97 (d, 2H), 7.61 (d, 2H), 7.48 (d, 2H), 7.11 (d, 2H), 4.50 (s, 2H), 4.09 (t, 2H), 3.73 (t, 2H), 3.53 (q, 2H), 1.11 (t, 3H). |
| **125** | 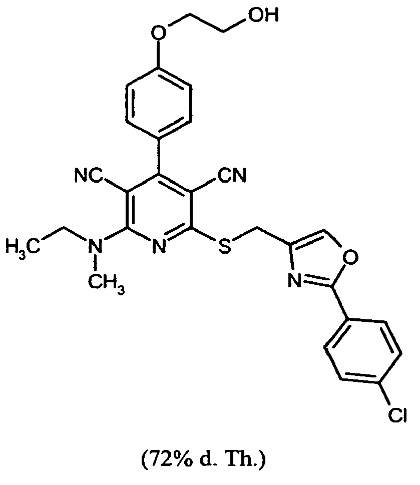 | 2.87 min (3); m/z = 546 | 8.19 (s, 1H), 7.98 (d, 2H), 7.62 (d, 2H), 7.51 (d, 2H), 7.10 (d, 2H), 4.90 (br. s, 1H), 4.50 (s, 2H), 4.08 (t, 2H), 3.80 (q, 2H), 3.73 (t, 2H), 3.32 (s, 3H), 1.20 (t, 3H). |
| **126** | 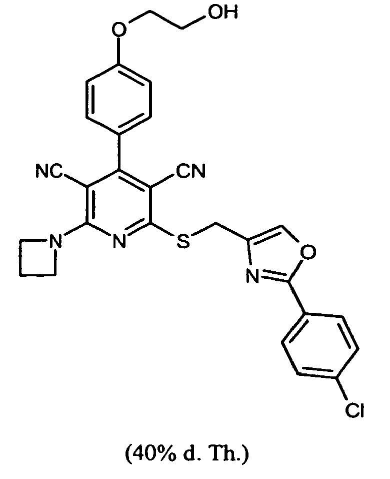 | 2.39 min (7); m/z = 544 | 8.18 (s, 1H), 7.98 (d, 2H), 7.62 (d, 2H), 7.44 (d, 2H), 7.10 (d, 2H), 4.89 (br. s, 1H), 4.58-4.34 (m, 6H), 4.09 (t, 2H), 3.73 (t, 2H), 2.39 (quin, 2H). |
| **127** | 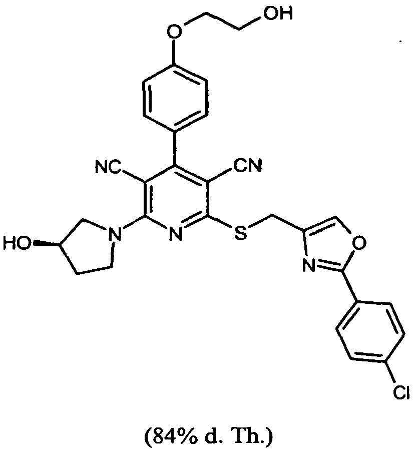 | 2.07 min (7); m/z = 574 | 8.19 (s, 1H), 7.97 (d, 2H), 7.61 (d, 2H), 7.48 (d, 2H), 7.10 (d, 2H), 5.14 (d, 1H), 4.90 (t, 1H), 4.52 (s, 2H), 4.41 (br. s, 1H), 4.08 (t, 2H), 4.01-3.82 (m, 3H), 3.80-3.71 (m, 3H), 2.09-1.88 (m, 2H). |
| **128** | 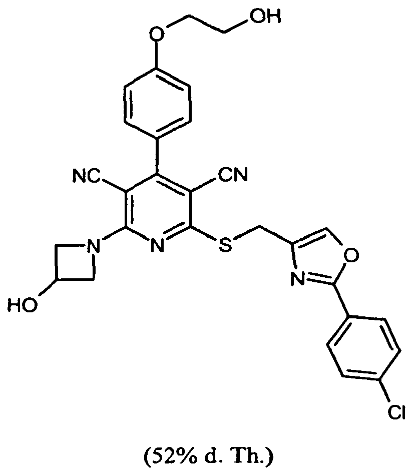 | 2.06 min (7); m/z = 560 | 8.18 (s, 1H), 7.98 (d, 2H), 7.62 (d, 2H), 7.45 (d, 2H), 7.10 (d, 2H), 5.88 (d, 1H), 4.90 (t, 1H), 4.73-4.55 (m, 3H), 4.48 (s, 2H), 4.18 (d, 2H), 4.08 (t, 2H), 3.73 (q, 2H). |
| **129** | 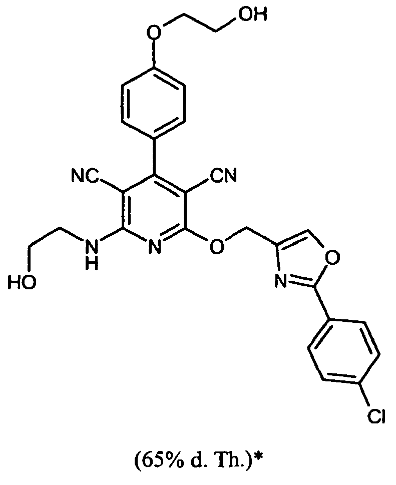 | 2.37 min (3); m/z = 532 | 8.38 (s, 1H), 8.09-7.97 (m, 3H), 7.62 (d, 2H), 7.46 (d, 2H), 7.10 (d, 2H), 5.50 (s, 2H), 4.91 (t, 1H), 4.86-4.80 (m, 1H), 4.08 (t, 2H), 3.73 (q, 2H), 3.64-3.57 (m, 4H). |
| **130** | 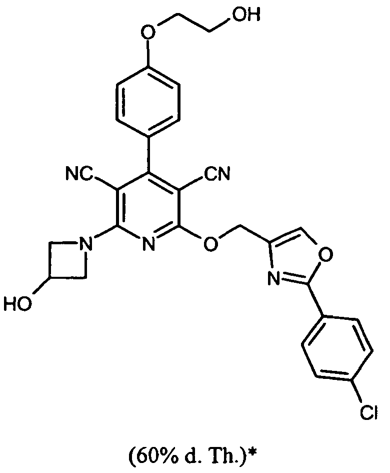 | 2.43 min (3); m/z = 544 | 8.36 (s, 1H), 8.00 (d, 2H), 7.62 (d, 2H), 7.45 (d, 2H), 7.10 (d, 2H), 5.89 (d, 1H), 5.46 (s, 2H), 4.90 (t, 1H), 4.75-4.56 (m, 3H), 4.25-4.07 (m, 4H), 3.73 (q, 2H). |
| * Reaktionsführung in DMF als Lösungsmittel (anstelle von THF) | | | |

### B. Bewertung der pharmakologischen und physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promotor wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:

Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2b- und A2a-Zellen 50% der maximalen Stimulierbarkeit mit der entsprechenden Substanz erreicht sind. Als Referenzverbindung dient in diesen Experimenten die Adenosin-analoge Verbindung NECA (5-*N*-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 1**

| **Beispiel Nr.** | **EC₅₀ A1 [nM] (1** µ**M Forskolin)** | **EC₅₀ A2a [nM]** | **EC₅₀ A2b [nM]** |
|---|---|---|---|
| **10** | 0.5 | 1130 | 922 |
| **11** | 0.3 | 703 | 845 |
| **31** | 0.9 | 467 | 315 |
| **48** | 0.3 | 138 | 4.4 |
| **49** | 0.4 | 300 | 100 |
| **50** | 0.4 | 3000 | 118 |
| **57** | 0.2 | 525 | 44 |
| **60** | 0.3 | 3000 | 236 |
| **61** | 0.7 | 439 | 221 |
| **66** | 0.9 | 575 | 370 |
| **80** | 0.8 | 461 | 89 |
| **81** | 0.3 | 64 | 20 |
| **93** | 8.9 | 522 | 336 |
| **95** | 0.5 | 3000 | 3000 |
| **101** | 0.4 | 72 | 226 |
| **106** | 0.3 | 318 | 48 |
| **110** | 0.3 | 497 | 95 |
| **114** | 0.2 | 1970 | 969 |
| **116** | 0.4 | 3000 | 698 |
| **117** | 0.2 | 1440 | 1090 |
| **119** | 0.3 | 1950 | 3000 |
| **122** | 4.1 | 3000 | 2250 |
| **126** | 0.5 | 684 | 78 |
| **127** | 0.4 | 984 | 283 |
| **128** | 0.1 | 1050 | 237 |
| **129** | 0.4 | 3000 | 3000 |
| **130** | 0.6 | 3000 | 245 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Bestimmung pharmakokinetischer Kenngrößen nach intravenöser und oraler Gabe

Die zu untersuchende Substanz wird Tieren (z.B. Maus, Ratte, Hund) intravenös als Lösung appliziert, die orale Applikation erfolgt als Lösung oder Suspension über eine Schlundsonde. Nach Substanzgabe wird den Tieren zu festgelegten Zeitpunkten Blut entnommen. Dieses wird heparinisiert, anschließend wird daraus durch Zentrifugation Plasma gewonnen. Die Substanz wird im Plasma über LC/MS-MS analytisch quantifiziert. Aus den so ermittelten Plasmakonzentration-Zeit-Verläufen werden die pharmakokinetischen Kenngrößen wie AUC (Fläche unter der Konzentration-Zeit-Kurve), Cₘₐₓ (maximale Plasmakonzentration), T_{1/2} (Halbwertszeit) und CL (Clearance) mittels eines validierten pharmakokinetischen Rechenprogramms berechnet.

### B-6. Bestimmung der Löslichkeit

### Benötigte Reagenzien:

- PBS-Puffer pH 6.5: 90.00 g NaCl p.a. (z.B. Fa. Merck, Art.-Nr. 1.06404.1000), 13.61 g KH₂PO₄ p.a. (z.B. Fa. Merck, Art.-Nr. 1.04873.1000) und 83.35 g 1N Natronlauge (z.B. Fa. Bernd Kraft GmbH, Art.-Nr. 01030.4000) werden in einen 1 Liter-Messkolben eingewogen, mit destilliertem Wasser auf 1 Liter aufgefüllt und für 1 Stunde gerührt. Danach wird mit 1 N Salzsäure (z.B. Fa. Merck, Art.-Nr. 1.09057.1000) der pH-Wert auf 6.5 eingestellt.
- PEG/Wasser-Lösung (70:30 v/v): 70 ml Polyethylenglykol 400 (z.B. Fa. Merck, Art.-Nr. 8.17003.1000) und 30 ml destilliertes Wasser werden in einem 100 ml-Messkolben homogenisiert.
- PEG/PBS-Puffer pH 6.5 (20:80 v/v): 20 ml Polyethylenglykol 400 (z.B. Fa. Merck, Art.-Nr. 8.17003.1000) und 80 ml PBS-Puffer pH 6.5 werden in einem 100 ml-Messkolben homogenisiert.
- Dimethylsulfoxid (z.B. Fa. Baker, Art.-Nr. 7157.2500)
- destilliertes Wasser.

### Herstellung der Ausgangslösung (Urlösung):

Mindestens 4 mg der Testsubstanz werden in ein Weithals-10 mm Screw V-Vial (Fa. Glastechnik Gräfenroda GmbH, Art.-Nr. 8004-WM-H/V15µ) mit passender Schraubkappe und Septum genau eingewogen, in einem Pipettierroboter mit DMSO bis zu einer Konzentration von 50 mg/ml versetzt und 10 Minuten geschüttelt.

### Herstellung der Kalibrierlösungen:

*Herstellung der Ausgangslösung für Kalibrierlösungen (Stammlösung):* In eine Mikrotiterplatte werden 10 µl der Urlösung mit Hilfe eines Pipettierroboters überführt und mit DMSO bis zu einer Konzentration von 600 µg/ml ausgefüllt. Die Probe wird bis zu ihrer vollständigen Lösung geschüttelt.
*Kalibrierlösung 1 (20* µ*g*/*ml):* 34.4 µl der Stammlösung werden mit 1000 µl DMSO versetzt und homogenisiert.
*Kalibrierlösung 2 (2.5* µ*g*/*ml):* 100 µl der Kalibrierlösung 1 werden mit 700 µl DMSO versetzt und homogenisiert.

### Herstellung der Probenlösungen:

*Probenlösung für Löslichkeit bis 5 g*/*Liter in PBS-Puffer pH 6.5*: In eine Mikrotiterplatte werden 10 µl Urlösung transferiert und mit 1000 µl PBS-Puffer pH 6.5 versetzt.
*Probenlösung für Löslichkeit bis 5 g*/*Liter in PEG*/*Wasser (70:30)*: In eine Mikrotiterplatte werden 10 µl Urlösung transferiert und mit 1000 µl PEG/Wasser (70:30) versetzt.

*Probenlösung für Löslichkeit bis 5 g*/*Liter in PEG*/*PBS-Puffer pH 6.5 (20:80)*: In eine Mikrotiterplatte werden 10 µl Urlösung transferiert und mit 1000 µl PEG/PBS-Puffer pH 6.5 (20:80) versetzt.

### Durchführung:

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011 mit Wechselblock Art.-Nr. 5362.000.019) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und mit DMSO zu 1:5 und 1:100 verdünnt. Es wird von jeder Verdünnung eine Abfüllung in ein geeignetes Gefäß für die HPLC-Analytik vorgenommen.

### Analytik:

Die Proben werden mittels RP-HPLC analysiert. Quantifiziert wird über eine Zwei-Punkt-Kalibrationskurve der Testverbindung in DMSO. Die Löslichkeit wird in mg/Liter ausgedrückt. Analysensequenz: 1) Kalibrierlösung 2.5 mg/ml; 2) Kalibrierlösung 20 µg/ml; 3) Probenlösung 1:5; 4) Probenlösung 1:100.

### HPLC-Methode für Säuren:

Agilent 1100 mit DAD (G1315A), quat. Pumpe (G1311A), Autosampler CTC HTS PAL, Degaser (G1322A) und Säulenthermostat (G1316A); Säule: Phenomenex Gemini C18, 50 mm x 2 mm, 5 µ;
Temperatur: 40°C; Eluent A: Wasser/Phosphorsäure pH 2; Eluent B: Acetonitril; Flussrate: 0.7 ml/min; Gradient: 0-0.5 min 85% A, 15% B; Rampe: 0.5-3 min 10% A, 90% B; 3-3.5 min 10% A, 90% B; Rampe: 3.5-4 min 85% A, 15% B; 4-5 min 85% A, 15% B.

### HPLC-Methode für Basen:

Agilent 1100 mit DAD (G1315A), quat. Pumpe (G1311A), Autosampler CTC HTS PAL, Degaser (G1322A) und Säulenthermostat (G1316A); Säule: VDSoptilab Kromasil 100 C18, 60 mm x 2.1 mm, 3.5 µ; Temperatur: 30°C; Eluent A: Wasser + 5 ml Perchlorsäure/Liter; Eluent B: Acetonitril; Flussrate: 0.75 ml/min; Gradient: 0-0.5 min 98% A, 2% B; Rampe: 0.5-4.5 min 10% A, 90% B; 4.5-6 min 10% A, 90% B; Rampe: 6.5-6.7 min 98% A, 2% B; 6.7-7.5 min 98% A, 2% B.

### B-7. Bestimmung der metabolischen Stabilität

Zur Bestimmung der metabolischen Stabilität von Testverbindungen werden diese *in vitro* mit Lebermikrosomen oder bevorzugt mit primären frischen Hepatozyten verschiedener Tierspezies (z.B. von Ratte und Hund) als auch humanen Ursprungs inkubiert, um Metabolitenprofile eines möglichst kompletten hepatischen Phase I- und Phase II-Metabolismus zu erhalten und zu vergleichen.

Die Testverbindungen werden mit einer Konzentration von 10-20 µM inkubiert. Dazu werden Stammlösungen der Substanzen mit einer Konzentration von 1-2 mM in Acetonitril hergestellt und dann mit einer 1:100-Verdünnung in den Inkubationsansatz pipettiert. Die Lebermikrosomen werden in 50 mM Kaliumphosphat-Puffer (pH 7.4) mit und ohne NADPH-generierendem System, bestehend aus 1 mM NADP⁺, 10 mM Glucose-6-phosphat und 1 Einheit Glucose-6-phosphat-Dehydrogenase, bei 37°C inkubiert. Primäre Hepatozyten werden in Suspension in Williams E-Medium ebenfalls bei 37°C inkubiert. Nach einer Inkubationszeit von 0-4 Stunden werden die Inkubationsansätze mit Acetonitril abgestoppt (Endkonzentration ca. 30%) und das Protein bei ca. 15000 x g abzentrifugiert. Die so abgestoppten Proben werden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung chromatographiert. Die UV-Chromatogramme in Verbindung mit massenspektrometrischen MS/MS-Daten dienen zur Identifizierung und Strukturaufklärung der Metabolite.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 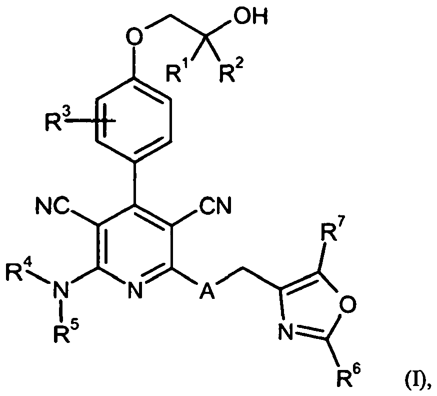 in welcher
A für O oder S steht,
R¹ für Wasserstoff oder (C₁-C₄)-Alkyl steht,
R² für Wasserstoff oder (C₁-C₄)-Alkyl, das mit Hydroxy, (C₁-C₄)-Alkoxy oder bis zu dreifach mit Fluor substituiert sein kann, steht
oder
R¹ und R² miteinander verknüpft sind und gemeinsam mit dem Kohlenstoffatom, an das sie gebunden sind, einen Cyclopropan- oder Cyclobutan-Ring bilden,
R³ für Wasserstoff, Halogen oder (C₁-C₄)-Alkyl steht,
R⁴ und R⁵ gleich oder verschieden sind und unabhängig voneinander für Wasserstoff oder (C₁-C₆)-Alkyl, das ein- oder zweifach, gleich oder verschieden, mit Hydroxy, (C₁-C₄)-Alkoxy, Amino, Mono-(C₁-C₄)-alkylamino, Di-(C₁-C₄)-alkylamino, Carboxyl, (C₁-C₄)-Alkoxycarbonyl und/oder einem 4- bis 7-gliedrigen Heterocyclus substituiert sein kann, stehen,
wobei der genannte Heterocyclus ein oder zwei Ring-Heteroatome aus der Reihe N, O und/oder S enthält und seinerseits ein- oder zweifach, gleich oder verschieden, mit (C₁-C₄)-Alkyl, Hydroxy, Oxo und/oder (C₁-C₄)-Alkoxy substituiert sein kann,
oder
R⁴ und R⁵ zusammen mit dem Stickstoffatom, an das sie gebunden sind, einen 4- bis 7-gliedrigen Heterocyclus bilden, der ein weiteres Ring-Heteroatom aus der Reihe N, O oder S enthalten und ein- oder zweifach, gleich oder verschieden, mit (C₁-C₄)-Alkyl, Hydroxy, Oxo und/oder (C₁-C₄)-Alkoxy substituiert sein kann,
und entweder (i)
R⁶ für (C₆-C₁₀)-Aryl oder 5- bis 10-gliedriges Heteroaryl mit bis zu drei Ring-Heteroatomen aus der Reihe N, O und/oder S steht, welche jeweils ein- bis dreifach, gleich oder verschieden, mit Halogen, Nitro, Cyano, (C₁-C₄)-Alkyl, Trifluormethyl, Hydroxy, (C₁-C₄)-Alkoxy, Difluormethoxy, Trifluormethoxy, Mono-(C₁-C₄)-alkylaminocarbonyl, (C₁-C₄)-Alkoxycarbonyl und/oder Carboxyl substituiert sein können,
und
R⁷ für Wasserstoff, Fluor, Chlor, (C₁-C₄)-Alkyl, Trifluormethyl, (C₁-C₄)-Alkoxycarbonyl, Carboxyl oder Phenyl steht, wobei
(C₁-C₄)-Alkyl mit Hydroxy oder (C₁-C₄)-Alkoxy
und
Phenyl mit Halogen, Cyano, (C₁-C₄)-Alkyl oder Trifluormethyl
substituiert sein können,
oder (ii)
R⁶ für Wasserstoff oder (C₁-C₄)-Alkyl steht
und
R⁷ für Phenyl oder 5- oder 6-gliedriges Heteroaryl mit bis zu zwei Ring-Heteroatomen
aus der Reihe N, O und/oder S steht, welche jeweils ein- oder zweifach, gleich oder verschieden, mit Halogen, Cyano, (C₁-C₄)-Alkyl und/oder Trifluormethyl substituiert sein können,
sowie ihre Salze, Solvate und Solvate der Salze.

2. Verbindung der Formel (I) nach Anspruch 1, in welcher
A für O oder S steht,
R¹ für Wasserstoff oder Methyl steht,
R² für Wasserstoff, Methyl, Hydroxymethyl, Methoxymethyl oder Trifluormethyl
steht,
R³ für Wasserstoff, Fluor oder Methyl steht,
R⁴ für Wasserstoff oder (C₁-C₄)-Alkyl, das ein- oder zweifach, gleich oder verschieden, mit Hydroxy, (C₁-C₄)-Alkoxy, Amino, Mono-(C₁-C₄)-alkylamino, Di-(C₁-C₄)-alkylamino, Carboxyl und/oder einem 4- bis 6-gliedrigen Heterocyclus substituiert sein kann, steht,
wobei der genannte Heterocyclus ein oder zwei Ring-Heteroatome aus der Reihe N und/oder O enthält und seinerseits ein- oder zweifach, gleich oder verschieden, mit Methyl, Hydroxy und/oder Methoxy substituiert sein kann,
R⁵ für Wasserstoff oder Methyl steht
oder
R⁴ und R⁵ zusammen mit dem Stickstoffatom, an das sie gebunden sind, einen 4- bis 6-gliedrigen Heterocyclus bilden, der ein weiteres Ring-Heteroatom aus der Reihe N oder O enthalten und ein- oder zweifach, gleich oder verschieden, mit Methyl, Hydroxy und/oder Methoxy substituiert sein kann,
und entweder (i)
R⁶ für Phenyl, Pyridyl oder Thienyl steht, welche jeweils ein- bis dreifach, gleich oder
verschieden, mit Fluor, Chlor, Cyano, (C₁-C₄)-Alkyl, Trifluormethyl, (C₁-C₄)-Alkoxy, Trifluormethoxy, Mono-(C₁-C₄)-alkylaminocarbonyl, (C₁-C₄)-Alkoxycarbonyl und/oder Carboxyl substituiert sein können,
und
R⁷ für Wasserstoff, (C₁-C₄)-Alkyl, Trifluormethyl, (C₁-C₄)-Alkoxycarbonyl, Carboxyl
oder Phenyl, das mit Fluor oder Chlor substituiert sein kann, steht,
oder (ii)
R⁶ für Wasserstoff steht
und
R⁷ für Phenyl, das ein- oder zweifach, gleich oder verschieden, mit Fluor, Chlor,
Cyano, Methyl und/oder Trifluormethyl substituiert sein kann, steht,
sowie ihre Salze, Solvate und Solvate der Salze.

3. Verbindung der Formel (I) nach Anspruch 1 oder 2, in welcher A für O oder S steht,
R¹ für Wasserstoff oder Methyl steht,
R² für Wasserstoff, Methyl, Hydroxymethyl oder Trifluormethyl steht,
R³ für Wasserstoff oder Fluor steht,
R⁴ für Wasserstoff oder (C₁-C₄)-Alkyl, das ein- oder zweifach, gleich oder verschieden, mit Hydroxy, Amino, Methylamino, Ethylamino, Dimethylamino und/oder Diethylamino substituiert sein kann, steht,
R⁵ für Wasserstoff oder Methyl steht
oder
R⁴ und R⁵ zusammen mit dem Stickstoffatom, an das sie gebunden sind, einen Azetidino-,
Pyrrolidino- oder Piperidino-Ring, welcher jeweils mit Hydroxy substituiert sein kann, oder einen Morpholino-Ring bilden,
R⁶ für Phenyl oder Thienyl steht, welche jeweils ein- oder zweifach, gleich oder verschieden, mit Fluor, Chlor, Methyl, Trifluormethyl, Methoxy und/oder Carboxyl substituiert sein können,
und
R⁷ für Wasserstoff, Methyl, Trifluormethyl, Methoxycarbonyl oder Carboxyl steht, sowie ihre Salze, Solvate und Solvate der Salze.

4. Verfahren zur Herstellung von Verbindungen der Formel (I), wie in den Ansprüchen 1 bis 3 definiert, **dadurch gekennzeichnet, dass** man eine Verbindung der Formel (II) 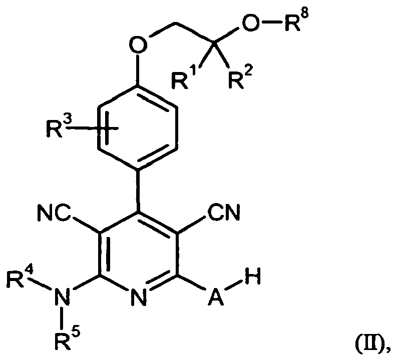 in welcher A, R¹, R², R³, R⁴ und R⁵ jeweils die in den Ansprüchen 1 bis 3 angegebenen Bedeutungen haben
und
R⁸ für Wasserstoff oder eine temporäre Hydroxy-Schutzgruppe steht,
in einem inerten Lösungsmittel in Gegenwart einer Base mit einer Verbindung der Formel (III) 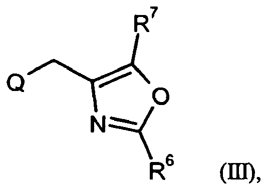 in welcher R⁶ und R⁷ die in den Ansprüchen 1 bis 3 angegebenen Bedeutungen haben und Q für eine geeignete Abgangsgruppe, vorzugsweise für Halogen, insbesondere Chlor,
Brom oder Iod, oder für Mesylat, Tosylat oder Triflat steht,
umsetzt
oder alternativ im Fall, dass A für O steht, eine Verbindung der Formel (IV) 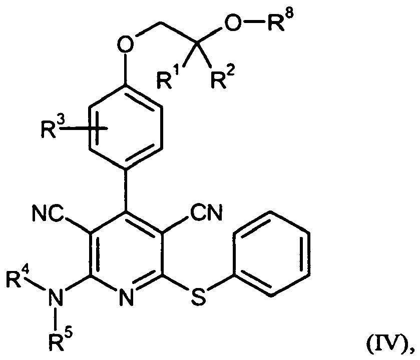 in welcher R¹, R², R³, R⁴, R⁵ und R⁸ jeweils die zuvor angegebenen Bedeutungen haben,
in einem inerten Lösungsmittel in Gegenwart einer Base mit einer Verbindung der Formel (V) 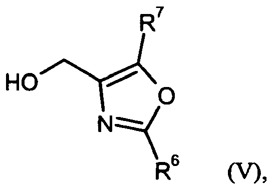 in welcher R⁶ und R⁷ die in den Ansprüchen 1 bis 3 angegebenen Bedeutungen haben, umsetzt,
anschließend gegebenenfalls vorhandene Schutzgruppen abspaltet und die resultierenden Verbindungen der Formel (I) gegebenenfalls mit den entsprechenden (i) Lösungsmitteln und/oder (ii) Basen oder Säuren in ihre Solvate, Salze und/oder Solvate der Salze überführt.

5. Verbindung, wie in einem der Ansprüche 1 bis 3 definiert, zur Behandlung und/oder Prophylaxe von Krankheiten.

6. Verwendung einer Verbindung, wie in einem der Ansprüche 1 bis 3 definiert, zur Herstellung eines Arzneimittel zur Behandlung und/oder Prophylaxe von Hypertonie, koronarer Herzerkrankung, akutem Koronarsyndrom, Angina pectoris, Herzinsuffizienz, Myokardinfarkt und Vorhofflimmern.

7. Verwendung einer Verbindung, wie in einem der Ansprüche 1 bis 3 definiert, zur Herstellung eines Arzneimittels zur Behandlung und/oder Prophylaxe von Diabetes, Metabolischem Syndrom und Dyslipidämien.

8. Arzneimittel enthaltend eine Verbindung, wie in einem der Ansprüche 1 bis 3 definiert, in Kombination mit einem oder mehreren inerten, nicht-toxischen, pharmazeutisch geeigneten Hilfsstoffen.

9. Arzneimittel enthaltend eine Verbindung, wie in einem der Ansprüche 1 bis 3 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

10. Arzneimittel nach Anspruch 8 oder 9 zur Behandlung und/oder Prophylaxe von Hypertonie, koronarer Herzerkrankung, akutem Koronarsyndrom, Angina pectoris, Herzinsuffizienz, Myokardinfarkt und Vorhofflimmern.

11. Arzneimittel nach Anspruch 8 oder 9 zur Behandlung und/oder Prophylaxe von Diabetes, Metabolischem Syndrom und Dyslipidämien.

12. Verbindung, wie in einen der Ansprüche 1 bis 3 definiert, oder eines Arzneimittels, wie in einem der Ansprüche 8, 9 und 11 definiert zur Verwendung in einem Verfahren zur Behandlung und/oder Prophylaxe von Hypertonie, koronarer Herzerkrankung, akutem Koronarsyndrom, Angina pectoris, Herzinsuffizienz, Myokardinfarkt und Vorhofflimmem in Menschen und Tieren unter Verwendung einer wirksamen Menge mindestens einer Verbindung, wie in einem der Ansprüche 1 bis 3 definiert, oder eines Arzneimittels, wie in einem der Ansprüche 8 bis 10 definiert.

13. Verbindung, wie in einen der Ansprüche 1 bis 3 definiert, oder eines Arzneimittels, wie in einem der Ansprüche 8, 9 und 11 definiert zur Verwendung in einem Verfahren zur Behandlung und/oder Prophylaxe von Diabetes, Metabolischem Syndrom und Dyslipidämien in Menschen und Tieren unter Verwendung einer wirksamen Menge mindestens einer Verbindung, wie in einem der Ansprüche 1 bis 3 definiert, oder eines Arzneimittels, wie in einem der Ansprüche 8, 9 und 11 definiert.

## Claims

1. Compound of the formula (I) 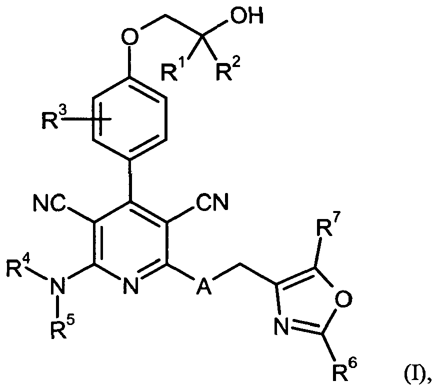 in which
A represents O or S,
R¹ represents hydrogen or (C₁-C₄)-alkyl,
R² represents hydrogen or (C₁-C₄)-alkyl which may be substituted by hydroxyl, (C₁-C₄)-alkoxy or up to three times by fluorine
or
R¹ and R² are attached to one another and together with the carbon atom to which they are
attached form a cyclopropane or cyclobutane ring,
R³ represents hydrogen, halogen or (C₁-C₄)-alkyl,
R⁴ and R⁵ are identical or different and independently of one another represent hydrogen or
(C₁-C₆)-alkyl which may be mono- or disubstituted by identical or different substituents from the group consisting of hydroxyl, (C₁-C₄)-alkoxy, amino, mono-(C₁-C₄)-alkylamino, di-(C₁-C₄)-alkylamino, carboxyl, (C₁-C₄)-alkoxycarbonyl and a 4- to 7-membered heterocycle,
where the heterocycle mentioned contains one or two ring heteroatoms from the group consisting of N, O and S and for its part may be mono- or disubstituted by identical or different substituents from the group consisting of (C₁-C₄)-alkyl, hydroxyl, oxo and (C₁-C₄)-alkoxy,
or
R⁴ and R⁵ together with the nitrogen atom to which they are attached form a 4- to 7-membered heterocycle which may contain a further ring heteroatom from the group consisting of N, O and S and which may be mono- or disubstituted by identical or different substituents from the group consisting of (C₁-C₄)-alkyl, hydroxyl, oxo and (C₁-C₄)-alkoxy,
and either (*i*)
R⁶ represents (C₆-C₁₀)-aryl or 5- to 10-membered heteroaryl having up to three ring
heteroatoms from the group consisting of N, O and S, which radicals may in each case be mono- to trisubstituted by identical or different substituents from the group consisting of halogen, nitro, cyano, (C₁-C₄)-alkyl, trifluoromethyl, hydroxyl, (C₁-C₄)-alkoxy, difluoromethoxy, trifluoromethoxy, mono-(C₁-C₄)-alkylaminocarbonyl, (C₁-C₄)-alkoxycarbonyl and carboxyl,
and
R⁷ represents hydrogen, fluorine, chlorine, (C₁-C₄)-alkyl, trifluoromethyl, (C₁-C₄)-alkoxycarbonyl, carboxyl or phenyl, where
(C₁-C₄)-alkyl may be substituted by hydroxyl or (C₁-C₄)-alkoxy
and
phenyl may be substituted by halogen, cyano, (C₁-C₄)-alkyl or trifluoromethyl,
or (*ii*)
R⁶ represents hydrogen or (C₁-C₄)-alkyl
and
R⁷ represents phenyl or 5- or 6-membered heteroaryl having up to two ring
heteroatoms from the group consisting of N, O and S, which radicals may in each case be mono- or disubstituted by identical or different substituents from the group consisting of halogen, cyano, (C₁-C₄)-alkyl and trifluoromethyl,
and also salts, solvates and solvates of the salts thereof.

2. Compound of the formula (I) according to Claim 1 in which
A represents O or S,
R¹ represents hydrogen or methyl,
R² represents hydrogen, methyl, hydroxymethyl, methoxymethyl or trifluoromethyl, R³ represents hydrogen, fluorine or methyl,
R⁴ represents hydrogen or (C₁-C₄)-alkyl which may be mono- or disubstituted by
identical or different substituents from the group consisting of hydroxyl, (C₁-C₄)-alkoxy, amino, mono-(C₁-C₄)-alkylamino, di-(C₁-C₄)-alkylamino, carboxyl and a 4-to 6-membered heterocycle,
where the heterocycle mentioned contains one or two ring heteroatoms from the group consisting of N and O and for its part may be mono- or disubstituted by identical or different substituents from the group consisting of methyl, hydroxy and methoxy,
R⁵ represents hydrogen or methyl
or
R⁴ and R⁵ together with the nitrogen atom to which they are attached form a 4- to 6-membered heterocycle which may contain a further ring heteroatom from the group consisting of N and O and which may be mono- or disubstituted by identical or different substituents from the group consisting of methyl, hydroxyl and methoxy,
and either (*i*)
R⁶ represents phenyl, pyridyl or thienyl which may in each case be mono- to
trisubstituted by identical or different substituents from the group consisting of fluorine, chlorine, cyano, (C₁-C₄)-alkyl, trifluoromethyl, (C₁-C₄)-alkoxy, trifluoromethoxy, mono-(C₁-C₄)-alkylaminocarbonyl, (C₁-C₄)-alkoxycarbonyl and carboxyl,
and
R⁷ represents hydrogen, (C₁-C₄)-alkyl, trifluoromethyl, (C₁-C₄)-alkoxycarbonyl,
carboxyl or phenyl which may be substituted by fluorine or chlorine,
or (*ii*)
R⁶ represents hydrogen
and
R⁷ represents phenyl which may be mono- or disubstituted by identical or different
substituents from the group consisting of fluorine, chlorine, cyano, methyl and trifluoromethyl,
and also salts, solvates and solvates of the salts thereof.

3. Compound of the formula (I) according to Claim 1 or 2 in which
A represents O or S,
R¹ represents hydrogen or methyl,
R² represents hydrogen, methyl, hydroxymethyl or trifluoromethyl,
R³ represents hydrogen or fluorine,
R⁴ represents hydrogen or (C₁-C₄)-alkyl which may be mono- or disubstituted by
identical or different substituents from the group consisting of hydroxyl, amino, methylamino, ethylamino, dimethylamino and diethylamino,
R⁵ represents hydrogen or methyl
or
R⁴ and R⁵ together with the nitrogen atom to which they are attached form an azetidino,
pyrrolidino or piperidino ring, each of which may be substituted by hydroxyl, or a morpholino ring,
R⁶ represents phenyl or thienyl which may in each case be mono- or disubstituted by
identical or different substituents from the group consisting of fluorine, chlorine, methyl, trifluoromethyl, methoxy and carboxyl,
and
R⁷ represents hydrogen, methyl, trifluoromethyl, methoxycarbonyl or carboxyl,
and also salts, solvates and solvates of the salts thereof.

4. Process for preparing compounds of the formula (I) as defined in any of Claims 1 to 3,
**characterized in that** a compound of the formula (II) 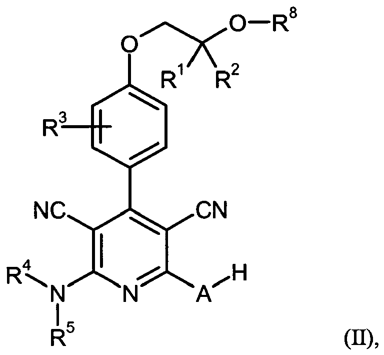 in which A, R¹, R², R³, R⁴ and R⁵ each have the meanings given in Claims 1 to 3
and
R⁸ represents hydrogen or a temporary hydroxyl protective group
is reacted in an inert solvent in the presence of a base with a compound of the formula (III) 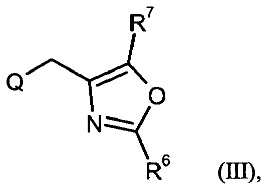 in which R⁶ and R⁷ have the meanings given in Claims 1 to 3 and Q represents a suitable leaving group, preferably halogen, in particular chlorine,
bromine or iodine, or represents mesylate, tosylate or triflate,
or alternatively, if A represents O, a compound of the formula (IV) 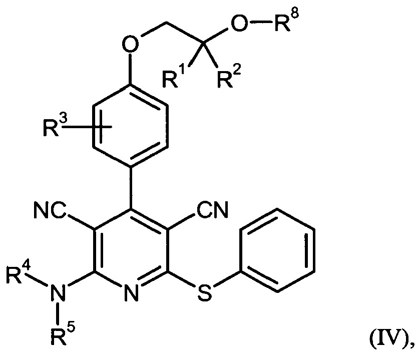 in which R¹, R², R³, R⁴, R⁵ and R⁸ each have the meanings given above
is reacted in an inert solvent in the presence of a base with a compound of the formula (V) 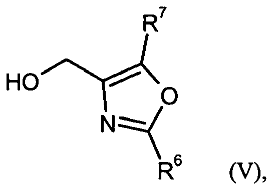 in which R⁶ and R⁷ have the meanings given in Claims 1 to 3,
any protective groups present are then removed and the resulting compounds of the formula (I) are, if appropriate, converted with the appropriate (i) solvents and/or (ii) bases or acids into their solvates, salts and/or solvates of the salts.

5. Compound as defined in any of Claims 1 to 3 for the treatment and/or prophylaxis of diseases.

6. Use of a compound as defined in any of Claims 1 to 3 for preparing a medicament for the treatment and/or prophylaxis of hypertension, coronary heart disease, acute coronary syndrome, angina pectoris, heart failure, myocardial infarction and atrial fibrillation.

7. Use of a compound as defined in any of Claims 1 to 3 for preparing a medicament for the treatment and/or prophylaxis of diabetes, metabolic syndrome and dyslipidemias.

8. Medicament comprising a compound as defined in any of Claims 1 to 3 in combination with one or more inert nontoxic pharmaceutically suitable auxiliaries.

9. Medicament comprising a compound as defined in any of Claims 1 to 3 in combination with one or more further active compounds selected from the group consisting of lipid metabolism-modifying active compounds, antidiabetics, antihypertensive drugs and antithrombotic drugs.

10. Medicament according to Claim 8 or 9 for the treatment and/or prophylaxis of hypertension, coronary heart disease, acute coronary syndrome, angina pectoris, heart failure, myocardial infarction and atrial fibrillation.

11. Medicament according to Claim 8 or 9 for the treatment and/or prophylaxis of diabetes, metabolic syndrome and dyslipidemias.

12. Compound as defined in any of Claims 1 to 3 or a medicament as defined in any of Claims 8, 9 and 11 for use in a method for the treatment and/or prophylaxis of hypertension, coronary heart disease, acute coronary syndrome, angina pectoris, heart failure, myocardial infarction and atrial fibrillation in humans and animals using an effective amount of at least one compound as defined in any of Claims 1 to 3 or a medicament as defmed in any of Claims 8 to 10.

13. Compound as defined in any of Claims 1 to 3 or a medicament as defined in any of Claims 8, 9 and 11 for use in a method for the treatment and/or prophylaxis of diabetes, metabolic syndrome and dyslipidemias in humans and animals using an effective amount of at least one compound as defined in any of Claims 1 to 3 or a medicament as defined in any of Claims 8, 9 and 11.

## Revendications

1. Composé de formule (I) 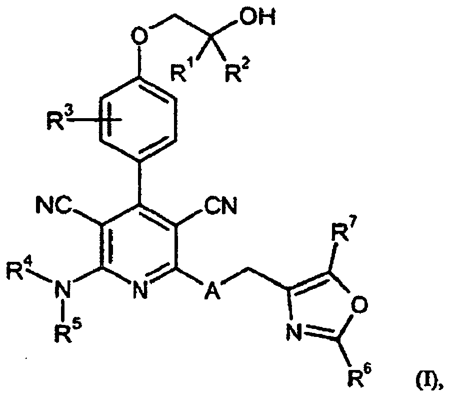 dans laquelle
A est O ou S,
R¹ est un atome d'hydrogène ou un groupe alkyle en C₁-C₄,
R² est un atome d'hydrogène ou un groupe alkyle en C₁-C₄, qui peut être substitué par un substituant hydroxy, alcoxy en C₁-C₄, ou jusqu'à trois fois par un ou des substituants fluoro,
ou
R¹ et R² sont reliés l'un à l'autre et forment avec l'atome de carbone auquel ils sont liés un noyau cyclopropane ou cyclobutane,
R³ est un atome d'hydrogène, un groupe halogéno ou alkyle en C₁-C₄,
R⁴ et R⁵ sont identiques ou différents et représentent chacun indépendamment de l'autre un atome d'hydrogène ou un groupe alkyle en C₁-C₆, qui peut être substitué une ou deux fois par un ou deux substituants identiques ou différents hydroxy, alcoxy en C₁-C₄, amino, mono(alkyle en C₁-C₄)amino, di (alkyle en C₁-C₄)amino, carboxyle, (alcoxy en C₁-C₄)carbonyle et/ou hétérocyclyle à 4 à 7 chaînons, le substituant hétérocyclyle mentionné contenant un ou deux hétéroatomes nucléaires de la série consistant en N, O et/ou S, et pour sa part pouvant être substitué une ou deux fois par un ou des substituants identiques ou différents alkyle en C₁-C₄, hydroxy, oxo et/ou alcoxy en C₁-C₄,
ou
R⁴ et R⁵, avec l'atome d'azote auquel ils sont liés, forment un radical hétérocyclique à 4 à 7 chaînons, qui peut contenir un hétéroatome nucléaire supplémentaire de la série consistant en N, O ou S, et qui peut être une ou deux fois substitué par un ou deux substituants identiques ou différents alkyle en C₁-C₄, hydroxy, oxo et/ou alcoxy en C₁-C₄,
ou bien (i)
R⁶ est un groupe aryle en C₆-C₁₀ ou hétéroaryle à 5 à 10 chaînons, ayant jusqu'à trois hétéroatomes nucléaires de la série consistant en N, O et/ou S, dont chacun peut être une à trois fois substituépar un ou des substituants identiques ou différents halogéno, nitro, cyano, alkyle en C₁-C₄, trifluorométhyle, hydroxy, alcoxy en C₁-C₄, difluorométhoxy, trifluorométhoxy, mono (alkyle en C₁-C₄)aminocarbonyle, (alcoxy en C₁-C₄)carbonyle et/ou carboxyle,
et
R⁷ est un atome d'hydrogène, un groupe fluoro, chloro, alkyle en C₁-C₄, trifluorométhyle, (alcoxy en C₁-C₄)carbonyle, carboxyle ou phényle, le radical alkyle en C₁-C₄ pouvant être substitué par un ou des substituants hydroxy ou alcoxy en C₁-C₄, et le groupe phényle pouvant être substitué par un ou des substituants halogéno, cyano, alkyle en C₁-C₄ ou trifluorométhyle,
ou bien (ii)
R⁶ est un atome d'hydrogène ou un groupe alkyle en C₁-C₄, et
R⁷ est un groupe phényle ou un groupe hétéroaryle à 5 ou 6 chaînons, ayant jusqu'à deux hétéroatomes nucléaires de la série consistant en N, O et/ou S, dont chacun peut être une ou deux fois substitué par un ou deux substituants identiques ou différents halogéno, cyano, alkyle en C₁-C₄ et/ou trifluorométhyle,
ainsi que ses sels, ses produits de solvatation et les produits de solvatation de ses sels.

2. Composé de formule (I) selon la revendication 1, dans lequel :
A est O ou S,
R¹ est un atome d'hydrogène ou le groupe méthyle,
R² est un atome d'hydrogène, le groupe méthyle, hydroxyméthyle, méthoxyméthyle ou trifluorométhyle, R³ est un atome d'hydrogène, le groupe fluoro ou méthyle,
R⁴ est un atome d'hydrogène ou un groupe alkyle en C₁-C₄, qui peut être substitué une ou deux fois par un ou deux substituants identiques ou différents hydroxy, alcoxy en C₁-C₄, amino, mono(alkyle en C₁-C₄)amino, di(alkyle en C₁-C₄)amino, carboxyle et/ou hétérocyclyle ayant 4 à 6 chaînons, le groupe hétérocyclyle mentionné contenant un ou deux hétéroatomes nucléaires de la série consistant en N et/ou O, et pouvant pour sa part être substitué une ou deux fois par un ou deux substituants identiques ou différents méthyle, hydroxy et/ou méthoxy,
R⁵ est un atome d'hydrogène ou le groupe méthyle,
ou
R⁴ et R⁵, avec l'atome d'azote auquel ils sont liés, forment un groupe hétérocyclyle à 4 à 6 chaînons, qui peut contenir un hétéroatome nucléaire supplémentaire de la série consistant en N ou O, et qui peut être substitué une ou deux fois par un ou deux substituants identiques ou différents méthyle, hydroxy et/ou méthoxy,
et ou bien (*i*)
R⁶ est le groupe phényle, pyridyle ou thiényle, dont chacun peut être substitué une à trois fois par un à trois substituants identiques ou différents fluoro, chloro, cyano, alkyle en C₁-C₄, trifluorométhyle, alcoxy en C₁-C₄, trifluorométhoxy, mono (alkyle en C₁-C₄)aminocarbonyle, (alcoxy en C₁-C₄)carbonyle et/ou carboxyle,
et
R⁷ est un atome d'hydrogène, un groupe alkyle en C₁-C₄, trifluorométhyle, (alcoxy en C₁-C₄)carbonyle, carboxyle ou phényle, qui peut être substitué par un ou des substituants fluoro ou chloro,
ou bien (*ii*)
R⁶ est un atome d'hydrogène,
et
R⁷ est le groupe phényle, qui peut être substitué une ou deux fois par un ou des substituants identiques ou différents fluoro, chloro, cyano, méthyle et/ou trifluorométhyle,
ainsi que ses sels, ses produits de solvatation et les produits de solvatation de ses sels.

3. Composé de formule (I) selon la revendication 1 ou 2, dans laquelle
A est O ou S,
R¹ est un atome d'hydrogène ou le groupe méthyle,
R² est un atome d'hydrogène, le groupe méthyle, hydroxyméthyle ou trifluorométhyle,
R³ est un atome d'hydrogène ou le groupe fluoro,
R⁴ est un atome d'hydrogène ou un groupe alkyle en C₁-C₄, qui peut être substitué une ou deux fois par un ou deux substituants identiques ou différents hydroxy, amino, méthylamino, éthylamino, diméthylamino et/ou diéthylamino,
R⁵ est un atome d'hydrogène ou le groupe méthyle,
ou
R⁴ et R⁵, avec l'atome d'azote auquel ils sont liés, forment un noyau azétidino, pyrrolidino ou pipéridino, dont chacun peut être substitué par un ou des substituants hydroxy, ou forment un noyau morpholino,
R⁶ est le groupe phényle ou thiényle, dont chacun peut être une ou deux fois substitué par un ou deux substituants identiques ou différents fluoro, chloro, méthyle, trifluorométhyle, méthoxy et/ou carboxyle,
et
R⁷ est un atome d'hydrogène, le groupe méthyle, trifluorométhyle, méthoxycarbonyle ou carboxyle,
ainsi que ses sels, ses produits de solvatation et les produits de solvatation de ses sels.

4. Procédé de préparation de composés de formule (I) tels que définis dans les revendications 1 à 3, **caractérisé en ce qu'**on fait réagir un composé de formule (II) 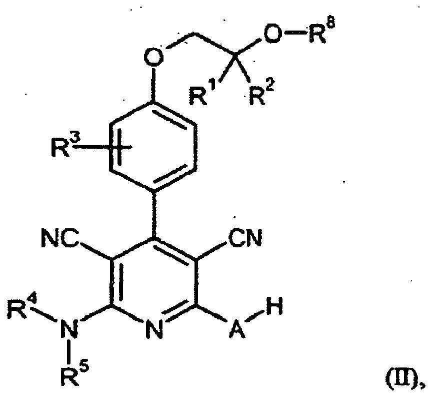 dans laquelle A, R¹, R², R³, R⁴ et R⁵ ont chacun les significations données dans les revendications 1 à 3, et R⁸ est un atome d'hydrogène ou un groupe hydroxy-protecteur provisoire,
dans un solvant inerte en présence d'une base, avec un composé de formule (III) 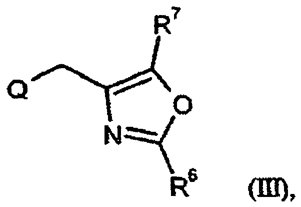 dans laquelle R⁶ et R⁷ ont les significations données dans les revendications 1 à 3, et Q est un groupe partant approprié, de préférence halogéno, en particulier chloro, bromo ou iodo, ou un groupe mésylate, tosylate ou triflate,
ou encore, dans le cas dans lequel A est O, on fait réagir un composé de formule (IV) 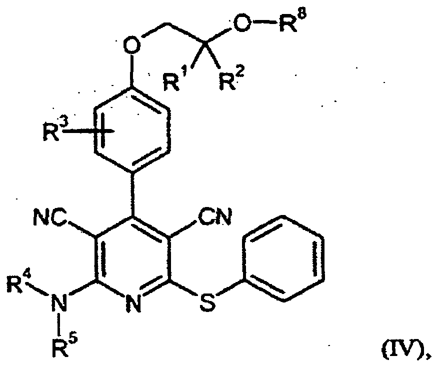 dans laquelle R¹, R², R³, R⁴, R⁵ et R⁸ ont chacun les significations données ci-dessus,
dans un solvant inerte en présence d'une base, avec un composé de formule (V) 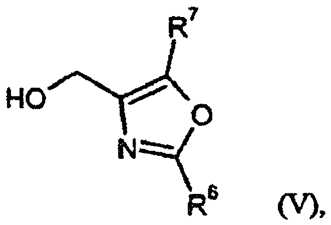 dans laquelle R⁶ et R⁷ ont les significations données dans les revendications 1 à 3, puis on dissocie les groupes protecteurs éventuellement présents, et éventuellement on convertit les composés obtenus de formule (I), avec (*i*) les solvants et/ou (*ii*) les bases ou acides correspondants, en leurs produits de solvatation, leurs sels et/ou les produits de solvatation de leurs sels.

5. Composé tel que défini dans l'une des revendications 1 à 3, pour le traitement et/ou la prophylaxie de maladies.

6. Utilisation d'un composé tel que défini dans l'une des revendications 1 à 3, pour la préparation d'un médicament destiné au traitement et/ou à la prophylaxie de l'hypertension, de la maladie coronarienne, du syndrome coronarien aigu, de l'angine de poitrine, de l'insuffisance cardiaque, de l'infarctus du myocarde et de la fibrillation auriculaire.

7. Utilisation d'un composé tel que défini dans l'une des revendications 1 à 3 pour la préparation d'un médicament destiné au traitement et/ou à la prophylaxie du diabète, du syndrome métabolique et des dyslipidémies.

8. Médicament contenant un composé tel que défini dans l'une des revendications 1 à 3, en combinaison avec un ou plusieurs adjuvants inertes, non toxiques, pharmaceutiquement appropriés.

9. Médicament contenant un composé tel que défini dans l'une des revendications 1 à 3, en combinaison avec un ou plusieurs autres principes actifs choisis dans le groupe consistant en les principes actifs modifiant le métabolisme des lipides, les antidiabétiques, les principes actifs hypotenseurs et les agents à effet antithrombotique.

10. Médicament selon la revendication 8 ou 9 pour le traitement et/ou la prophylaxie de l'hypertension, de la maladie coronarienne, du syndrome coronarien aigu, de l'angine de poitrine, de l'insuffisance cardiaque, de l'infarctus du myocarde et de la fibrillation auriculaire.

11. Médicament selon la revendication 8 ou 9 pour le traitement et/ou la prophylaxie du diabète, du syndrome métabolique et des dyslipidémies.

12. Composé tel que défini dans l'une des revendications 1 à 3 ou médicament tel que défini dans l'une des revendications 8, 9 et 11, pour utilisation dans un procédé pour le traitement et/ou la prophylaxie de l'hypertension, de la maladie coronarienne, du syndrome coronarien aigu, de l'angine de poitrine, de l'insuffisance cardiaque, de l'infarctus du myocarde et de la fibrillation auriculaire chez l'homme et l'animal, par utilisation d'une quantité efficace d'au moins un composé tel que défini dans l'une des revendications 1 à 3 ou d'un médicament tel que défini dans l'une des revendications 8 à 10.

13. Composé tel que défini dans l'une des revendications 1 à 3 ou médicament tel que défini dans l'une des revendications 8, 9 et 11, pour utilisation dans un procédé pour le traitement et/ou la prophylaxie du diabète, du syndrome métabolique et des dyslipidémies chez l'homme et l'animal, par utilisation d'une quantité efficace d'au moins un composé tel que défini dans l'une des revendications 1 à 3 ou d'un médicament tel que défini dans l'une des revendications 8, 9 et 11.
